(19) Europäisches Patentamt
European Patent Office
Office européen des brevets

(11) Publication number: **0 034 894**
**B1**

# (12) EUROPEAN PATENT SPECIFICATION

(45) Date of publication of patent specification: **12.09.84**

(21) Application number: **81300569.1**

(22) Date of filing: **12.02.81**

(51) Int. Cl.$^3$: **C 07 D 295/10,**
C 07 D 295/12,
C 07 D 295/14,
C 07 D 295/18,
C 07 D 241/04,
C 07 D 233/76, A 61 K 31/495

(54) **2-(1-Piperazinyl)-2,4,6-cycloheptatrien-1-one derivatives, processes for their preparation, intermediates used in the processes and the preparation thereof and pharmaceutical compositions containing the derivatives.**

(30) Priority: **25.02.80 US 124163**
**25.02.80 US 124164**
**25.02.80 US 124165**

(43) Date of publication of application:
**02.09.81 Bulletin 81/35**

(45) Publication of the grant of the patent:
**12.09.84 Bulletin 84/37**

(84) Designated Contracting States:
**AT BE CH DE FR GB IT LI LU NL SE**

(56) References cited:
**FR-A-2 218 891**
**US-A-3 651 143**

**CHEMICAL ABSTRACTS, vol. 69, no. 17, 21st October 1968, page 6288, abstract no. 67325c Columbus, Ohio, U.S.A. J.M. KHANNA et al.: "Agents acting on the central nervous system. IX. 5,6-disubstituted 6,7,8,9-tetrahydro-5H-benzocycloheptenes and 8,9;dihydro-7H-benzocycloheptadienes"**

(73) Proprietor: **AYERST, MCKENNA AND HARRISON INC.**
**1025 Laurentian Boulevard**
**St. Laurent Quebec H4R 1J6 (CA)**

(72) Inventor: **Bagli, Jehan Framroz**
**12, Allancroft Road**
**Kirkland Quebec H9J 2G9 (CA)**
Inventor: **Bogri, Tibor**
**740, Montpellier Apt. 1403**
**Montreal Quebec H4L 4B1 (CA)**
Inventor: **Voith, Katherine**
**45, Ballantyne Terr.**
**Dorval Quebec H9S 3E4 (CA)**

(74) Representative: **Brown, Keith John Symons et al**
**c/o John Wyeth & Brother Limited Patent and Trademark Department Huntercombe Lane South**
**Taplow Maidenhead Berkshire SL6 0PH. (GB)**

(56) References cited:
**JOURNAL OF THE AMERICAN CHEMICAL SOCIETY, vol. 95, no. 21, 17th October 1973, pages 7101-7107 G. BIGGI et al.: "Normal vs. Abnormal nucleophilic substitutions on cycloheptatrienones carrying a mobile alpha-substituent. A rationalization"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 894 B1

## Description

This invention relates to novel 2-(1-piperazinyl)-2,4,6-cycloheptatrien-1-one derivatives, to therapeutically acceptable acid addition salts thereof, to a process for their preparation, and to pharmaceutical compositions of the derivatives. These derivatives exhibit dopamine-receptor stimulating activity in a mammal. Thus, they can be useful for treating hyperprolactinemia, galactorrhea, amenorrhea, impotence, Parkinsonism, diabetes, acromegaly, hypertension and other central nervous system disorders which respond to dopamine-receptor stimulation.

Of the known 2,4,6-cycloheptatrien-1-one-derivatives, the 2-piperidinyl-2,4,6-cycloheptatrien-1-one described by G. Biggi *et al.*, J. Amer. Chem. Soc., *94*, 4700 (1972), can be considered the most closely related to the compounds of this invention. However, the latter 2-piperidinyl derivative is treated as a chemical curiosity without any indicated useful pharmacological activity. Furthermore, the compounds of this invention differ from the compounds of Biggi *et al* by having a 1-piperazinyl group at position 2 of the 2,4,6-cycloheptatrien-1-one ring.

The compounds of this invention are represented by formula 1

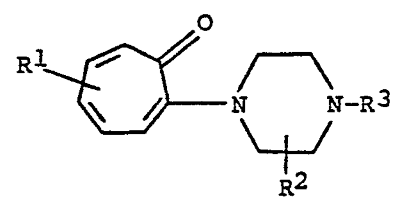

(I)

in which $R^1$ represents a substituent at positions 3, 4, 5, 6 or 7 of the 2,4,6-cycloheptatrien-1-one ring and is selected from hydrogen, halo, lower alkyl, lower alkoxy, trifluoromethyl or 1-oxo(lower)alkylamino; $R^2$ is hydrogen or lower alkyl having one to three carbon atoms; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alkyl; lower alkenyl substituted with phenyl; phenyl mono-, di- or trisubstituted with lower alkyl, halo, lower alkoxy, hydroxy or trifluoromethyl; 2-(4,5-dihydro-2-oxazolyl)benzoyl, 2,3-dihydro-8-oxo-cyclohepta[b]furan-2-ylmethyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to six carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di- or trisubstituted with lower alkyl, halo, lower alkoxy, hydroxy or trifluoromethyl, or phenoxy mono-, di- or trisubstituted with lower alkyl, halo, lower alkoxy, acetylamino, hydroxy or trifluoromethyl, or a group of formula

$$CR^5R^6\text{—}\underset{\underset{OH}{|}}{Alk^1}\text{—}R^7$$

wherein $Alk^1$ is a trivalent alkylene having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkyl, halo, lower alkoxy, hydroxy or trifluoromethyl, or phenoxy mono-, di- or trisubstituted with lower alkyl, halo, lower alkoxy, hydroxy or trifluoromethyl; with the proviso that when $R^7$ is hydroxy, the hydroxy groups are joined to different carbon atoms; or a therapeutically acceptable acid addition salt thereof.

A preferred group of compounds of this invention is represented by formula I in which $R^1$ represents a substituent at position 7 of the 2,4,6-cycloheptatrien-1-one ring and is selected from hydrogen, halo, lower alkyl, lower alkoxy, trifluoromethyl, or 1-oxo(lower)alkylamino; $R^2$ is hydrogen or lower alkyl having one to three carbon atoms; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alky; lower alkenyl substituted with phenyl; phenyl mono-, di- or trisubstituted with lower alkyl, halo, lower alkoxy, hydroxy or trifluoromethyl; 2-(4,5-dihydro-2-oxazolyl)benzoyl, 2,3-dihydro-8-oxo-cyclohepta[b]furan-2-ylmethyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to six carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di- or trisubstituted with lower alkoxy or hydroxy, or

phenoxy mono-, di- or trisubstituted with lower alkyl, halo or acetylamino, or a group of formula

$$\begin{array}{l} CR^5R^6—Alk^1—R^7 \\ \quad\quad\quad\;\; | \\ \quad\quad\quad\; OH \end{array}$$

wherein $Alk^1$ is a trivalent alkylene having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkoxy or hydroxy, or phenoxy mono- di- or trisubstituted with lower alkyl or halo; with the proviso that when $R^7$ is hydroxy, the hydroxy groups are joined to different carbon atoms; or a therapeutically acceptable acid addition salt thereof.

A more preferred group of compounds of this invention is represented by formula I in which $R^1$ represents a substituent at position 7 of the 2,4,6-cycloheptatrien-1-one ring and is selected from hydrogen or bromo; $R^2$ is hydrogen or methyl; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alkyl, phenyl monosubstituted with lower alkyl, halo or trifluoromethyl, 2-(4,5-dihydro-2-oxazolyl)benzoyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to three carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono- or disubstituted with lower alkoxy or hydroxy, or phenoxy monosubstituted with lower alkyl, halo or acetylamino; or a group of formula

$$\begin{array}{l} CR^5R^6—Alk^1—R^7 \\ \quad\quad\quad\;\; | \\ \quad\quad\quad\; OH \end{array}$$

wherein $Alk^1$ is a trivalent alkylene having one to three carbon atoms; $R^5$ and $R^6$ each is hydrogen or lower alkyl having one to three carbon atoms; $R^7$ is hydrogen, phenyl, hydroxy, phenoxy, phenyl mono-, di- or trisubstituted with lower alkoxy or hydroxy or phenoxy monosubstituted with lower alkyl or halo; with the proviso that when $R^7$ is hydroxy, the hydroxy groups are joined to different carbon atoms; or a therapeutically acceptable acid addition salt thereof.

A most preferred group of compounds of this invention is represented by formula I in which $R^1$ and $R^2$ are hydrogen; and $R^3$ is hydrogen, lower alkyl, lower alkenyl, lower alkoxycarbonyl, a group of formula Alk-$R^4$ wherein Alk is a divalent alkyl having one or two carbon atoms and $R^4$ is lower alkoxy, cyano, 1-oxo(lower)alkoxy, or phenyl mono- or disubstituted with lower alkoxy or hydroxy, or a group of formula

$$\begin{array}{l} CR^5R^6—Alk^1—R^7 \\ \quad\quad\quad\;\; | \\ \quad\quad\quad\; OH \end{array}$$

wherein $Alk^1$ is CH; $R^5$ is hydrogen; $R^6$ is hydrogen or lower alkyl having one to three carbon atoms; and $R^7$ is hydrogen, phenyl or phenyl mono-, di- or trisubstituted at positions 3, 4 or 5 with lower alkoxy or hydroxy; or a therapeutically acceptable acid addition salt thereof.

The compounds of formula I may be prepared by a process, which comprises selecting a process from the group of:

a) when a compound of formula I in which $R^1$, $R^2$ and $R^3$ are as defined herein is required, condensing a compound of formula II

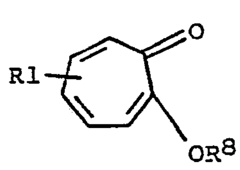

(II)

in which $R^1$ is as defined herein and $R^8$ is lower alkyl with a compound of formula III

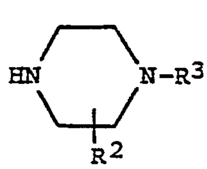

(III)

in which $R^2$ is as defined herein and $R^3$ is as defined above or is amino(lower)alkyl;

b) when a compound of formula I in which $R^1$ and $R^2$ are as defined herein and $R^3$ is lower alkyl,

lower alkenyl, lower alkynyl, cyclo(lower)alkyl, lower alkoxycarbonyl, 1-oxo(lower)alkyl, cyclo(lower)-alkyl substituted with a lower alkyl, lower alkenyl substituted with phenyl, 2-(4,5-dihydro-2-oxazolyl)benzoyl, 2,3-dihydro-8-oxo-cyclohepta[b]furan-2-ylmethyl, 2,3-diacetyloxypropyl, 2-(1,3-dihydro-1,3-dioxo-2H-isoindol-2-yl)ethyl, a group of formula Alk-$R^4$ wherein Alk and $R^4$ are as defined herein, or a group of formula

$$CR^5R^6—\underset{\displaystyle OH}{\underset{|}{Alk^1}}—R^7$$

wherein $R^5$, $R^6$, $R^7$ and $Alk^1$ are as defined herein is required, reacting the compound of formula I in which $R^1$ and $R^2$ are as defined herein and $R^3$ is hydrogen with a halide of formula X—$R^3$ wherein X is bromo, chloro or iodo and $R^3$ is lower alkyl, lower alkenyl, lower alkynyl, cyclo(lower)alkyl, lower alkoxycarbonyl, 1-oxo(lower)alkyl, cyclo(lower)alkyl substituted with a lower alkyl, lower alkenyl substituted with phenyl, 2,3-dihydro-8-oxo-cyclohepta[b]furan-2-ylmethyl, 2,3-diacetyloxypropyl, 2-(1,3-dihydro-1,3-dioxo-2H-isoindol-2-yl)ethyl; a halide of formula X-Alk-$R^4$ wherein $R^4$, X and Alk are as defined herein; or a halide of formula

$$X—CR^5R^6—\underset{\displaystyle OH}{\underset{|}{Alk^1}}—R^7$$

wherein $R^5$, $R^6$, $R^7$, X and $Alk^1$ are as defined herein in the presence of a proton acceptor;

(c) reacting the compound of formula I in which $R^1$ and $R^2$ are as defined therein and $R^3$ is hydrogen, with epichlorohydrin to obtain the corresponding dihydroxy intermediate and acetylating this intermediate to give the corresponding compound of formula I in which $R^1$ and $R^2$ are as defined herein and $R^3$ is 2,3-diacetyloxypropyl;

(d) acylating the compound of formula I in which $R^1$ and $R^2$ are as defined therein and $R^3$ is hydrogen, to give the corresponding compound of formula I in which $R^1$ and $R^2$ are as defined therein and $R^3$ is 1-oxo(lower)alkyl;

(e) condensing the compound of formula I in which $R^1$ and $R^2$ are as defined therein and $R^3$ is hydrogen, with 1-chloro-2-propanone to obtain the corresponding intermediate having a 2-oxopropyl group and reacting the latter intermediate with potassium cyanide and ammonium carbonate to obtain the corresponding compound of formula I in which $R^1$ and $R^2$ are as defined therein and $R^3$ is 5-methyl-2,4-imidazolidinedione-5-yl-methyl;

(f) reacting the compound of formula I in which $R^1$ and $R^2$ are as defined therein and $R^3$ is 2-(1,3-dihydro-1,3-dioxo-2H-isoindol-2-yl)ethyl, with hydrazine hydrate to give the corresponding intermediate having an 2-aminoethyl group and acetylating said latter intermediate to give the corresponding compound of formula I in which $R^1$ and $R^2$ are as defined therein and $R^3$ is 2-(acetylamino)ethyl;

(g) reacting the compound of formula I in which $R^1$ and $R^2$ are as defined herein and $R^3$ is hydrogen in the presence of a proton acceptor with a tosylate of the formula TsO-Alk-$R^4$ wherein Alk is as defined herein and $R^4$ is phenyl, phenoxy, phenyl, mono-, di- or trisubstituted with lower alkyl, halo, lower alkoxy or trifluoromethyl, or phenoxy mono-, di- or trisubstituted with lower alkyl, halo, lower alkoxy, acetylamino or trifluoromethyl to give the corresponding compound of formula I in which $R^1$ and $R^2$ are as defined herein and $R^3$ is a group of formula Alk-$R^4$ wherein Alk is as defined herein and $R^4$ is as defined immediately above;

(h) reacting the compound of formula I in which $R^1$ and $R^2$ are as defined herein and $R^3$ is a group of formula Alk-$R^4$ wherein Alk is as defined herein and $R^4$ is phenyl mono-, di- or trisubstituted with lower alkoxy with boron tribromide to obtain the corresponding compound of formula I in which $R^1$ and $R^2$ are as defined herein and $R^3$ is a group of formula Alk-$R^4$ wherein Alk is as defined herein and $R^4$ is phenyl mono-, di or trisubstituted with hydroxy;

(i) reacting the compound of formula I in which $R^1$ and $R^2$ are as defined herein and $R^3$ is a group of formula Alk-$R^4$ wherein Alk is as defined herein and $R^4$ is cyano with concentrated sulfuric acid, followed by concentrated ammonium hydroxide, to give the corresponding compound of formula I in which $R^1$ and $R^2$ are as defined herein and $R^3$ is a group of formula Alk-$R^4$ wherein Alk is as defined herein and $R^4$ is aminocarbonyl;

(j) acylating a compound of formula IV in which

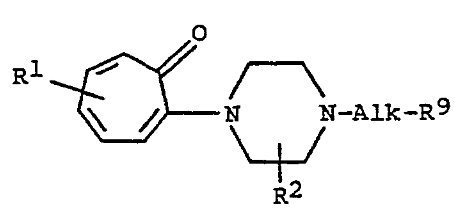

(IV)

Alk, $R^1$ and $R^2$ are as defined herein and $R^9$ is hydroxy to obtain the corresponding compound of formula I in which $R^1$ and $R^2$ are as defined herein and $R^3$ is a group of formula Alk-$R^4$ wherein Alk is as defined herein and $R^4$ is 1-oxo(lower)alkoxy;

(k) reacting the compound of formula IV in which Alk, $R^1$ and $R^2$ are as defined herein and $R^9$ is chloro with thiourea to give the corresponding intermediate having an [(aminoiminomethyl)amino]thio group and acetyling said latter intermediate with acetic anhydride to give the corresponding compound of formula I in which $R^1$ and $R^2$ are as defined herein and $R^3$ is a group of formula Alk-$R^4$ wherein Alk is as defined herein and $R^4$ is acetylthio;

(l) reacting the compound of formula I in which $R^1$ and $R^2$ are as defined herein and $R^3$ is hydrogen with an epoxide of formula

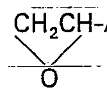

wherein $Alk^2$ is a divalent lower alkyl having one to five carbon atoms and $R^7$ is as defined herein to give the corresponding compound of formula I in which $R^1$ and $R^2$ are as defined herein; and $R^3$ is a group of formula

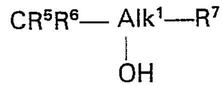

wherein $R^5$ and $R^6$ are hydrogen;

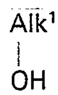

is CH(OH)-$Alk^2$ wherein $Alk^2$ is as defined herein; and $R^7$ is as defined herein;

(m) reacting the compound of formula I in which $R^1$ and $R^2$ are as defined herein and $R^3$ is hydrogen in the presence of a proton acceptor with a chloro-epoxide of the formula

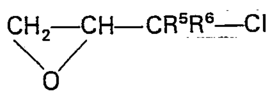 or 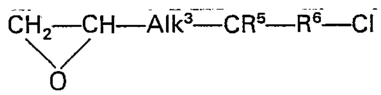

wherein $Alk^3$ is a divalent lower alkyl having one to four carbon atoms and $R^5$ and $R^6$ are as defined herein to give the corresponding compound of formula I in which $R^1$ and $R^2$ are as defined herein and $R^3$ is a group of formula

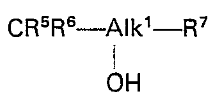

wherein $R^5$ and $R^6$ are as defined herein; $R^7$ is a hydroxy; and

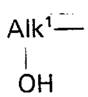 is 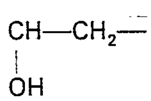 or 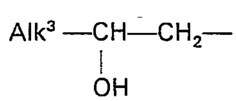

wherein $Alk^3$ is as defined herein;

(n) reacting the compound of formula I in which $R^1$ and $R^2$ are as defined herein and $R^3$ is hydrogen in the presence of a proton acceptor with an alkanone of the formula

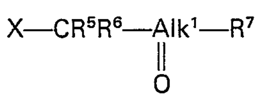

wherein X is bromo, chloro or iodo; $R^5$, $R^6$ and $R^7$ are as defined herein; and

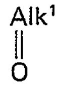

is a divalent alkanone having one to six carbon atoms, with the proviso that when $R^7$ is hydroxy, the hydroxy and ketone groups are joined to different carbon atoms to give the corresponding intermediate ketone of formula V in which

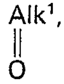,

$R^1$, $R^2$, $R^5$, $R^6$ and $R^7$ are

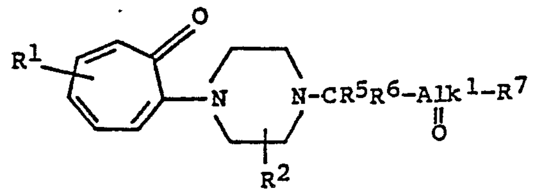

(V)

as defined herein, and reducing said intermediate ketone to obtain the corresponding compound of formula I in which $R^1$ and $R^2$ are as defined herein and $R^3$ is a group of formula

$$\begin{array}{c} CR^5R^6—Alk^1—R^7 \\ | \\ OH \end{array}$$

wherein $R^5$, $R^6$, $R^7$ and $Alk^1$ are as defined herein;

(o) reacting a compound of formula (I) in which $R^1$ and $R^2$ are as defined herein and $R^3$ is hydrogen with ethylene oxide to give a compound of formula (I) in which $R^1$ and $R^2$ are as defined herein and $R^3$ is $—CH_2CH_2OH$; and

(p) reacting the compound of formula I in which $R^1$, $R^2$ and $R^3$ are as defined herein with a therapeutically acceptable acid to obtain the corresponding compound of formula I in which $R^1$, $R^2$ and $R^3$ are as defined herein as a therapeutically acceptable acid addition salt.

A pharmaceutical composition is provided by admixing the compound of formula I, or a therapeutically acceptable acid addition salt thereof, with a pharmaceutically acceptable carrier.

The compounds of this invention may be used to stimulate dopamine receptors in a mammal in need thereof by administering to the mammal an effective dopamine receptor stimulating amount of a compound of formula I or a therapeutically acceptable acid addition salt thereof.

The term "lower alkyl" as used herein means straight chain alkyl radicals containing from one to six carbon atoms and branched chain alkyl radicals containing three or four carbon atoms and includes methyl, ethyl, propyl, 1-methylethyl, butyl, 1-methylpropyl, pentyl, hexyl and the like, unless stated otherwise. 1-Methylethyl and 1-methylpropyl also are known as isopropyl and sec-butyl.

The term "lower alkoxy" as used herein means straight chain alkoxy radicals containing from one to six carbon atoms and branched chain alkoxy radicals containing three or four carbon atoms and includes methoxy, ethoxy, 1-methylethoxy, butoxy, hexoxy and the like.

The term "1-oxo(lower)alkyl" or "lower alkanoyl" as used herein means straight chain 1-oxoalkyl radicals containing from two to six carbon atoms and branched chain 1-oxoalkyl radicals containing four to six carbon atoms and includes acetyl, 1-oxopropyl, 2-methyl-1-oxopropyl, 1-oxohexyl and the like.

The term "1-oxo(lower)alkoxy" as used herein means straight chain 1-oxoalkoxy radicals containing from two to six carbon atoms and branched chain 1-oxoalkoxy radicals containing four to six carbon atoms and includes acetyloxy, 1-oxopropoxy, 1-oxobutoxy, 2,2-dimethyl-1-oxopropoxy, 1-oxohexoxy and the like.

The term "halo" as used herein means halogens and includes fluorine, chlorine, bromine and iodine, unless stated otherwise.

The term "lower alkynyl" as used herein means straight chain alkynyl radicals containing from two to six carbon atoms and a branched chain alkynyl radical containing four carbon atoms and includes ethynyl, 2-propynyl, 1-methyl-2-propynyl, 3-hexynyl and the like.

The term "lower alkenyl" as used herein means straight chain alkenyl radicals containing from two to six carbon atoms and branched chain alkenyl radicals containing three or four carbon atoms and includes ethenyl, 2-methyl-2-propenyl, 4-hexenyl and the like.

The term "cyclo(lower)alkyl" as used herein means saturated cyclic hydrocarbon radicals containing from three to six carbon atoms and includes cyclopropyl, cyclobutyl, cyclopentyl and cyclohexyl.

The term "cyclo(lower)alkyl substituted with a lower alkyl" as used herein means saturated cyclic hydrocarbon radicals containing from three to six carbon atoms, as defined above, which is substituted with a lower alkyl group and includes 1-methylcyclopropyl, 2-ethylcyclobutyl, 4-propylcyclohexyl and the like.

The term "—Alk—" as used herein means a divalent alkyl radical derived from a straight and branched chain aliphatic hydrocarbons containing from one to six carbon atoms by removal of two hydrogen atoms, unless stated otherwise, and includes, for example $—CH_2—$, $—CH_2—CH_2—$, $—CH(CH_3)—CH_2—CH_2—$, $—(CH_2)_6—$, $—CH_2—\underset{\displaystyle CH_2—CH_3}{\underset{|}{CH}}—CH_2—$

and the like.

The term "—$Alk^1$—" as used herein means a trivalent alkyl radical derived from a straight and branched chain aliphatic hydrocarbons containing from one to six carbon atoms by removal of three hydrogen atoms, unless stated otherwise, and includes, for example

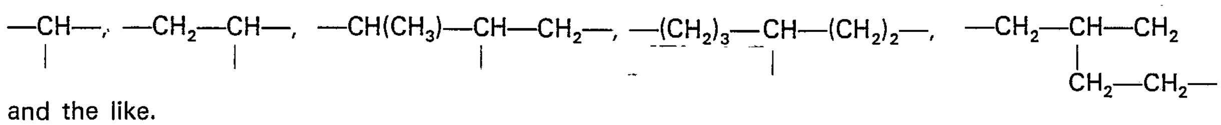

and the like.

The term "$\underset{\underset{O}{\|}}{-Alk^1-}$" as used herein means a divalent alkanone radical derived from straight and branched chain alkanones having one to six carbon atoms by removal of two hydrogen atoms, unless stated otherwise, and includes, for example,

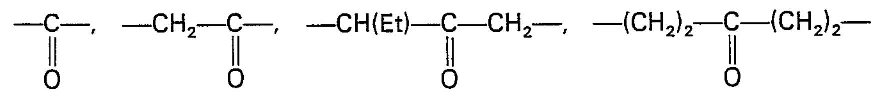

and the like.

The term "lower alkanol" as used herein means both straight and branched chain alkanols containing from one to four carbon atoms and includes methanol, ethanol, isopropanol, butanol and the like.

The term "organic proton acceptor" as used herein means the organic bases, or amines for instance, triethylamine, pyridine, N-ethyl-morpholine, 1,5-diazabicyclo[4.3.0]non-5-ene and the like.

The term "inorganic proton acceptor" as used herein means the inorganic bases, preferably the alkali metal hydroxides, carbonates and bicarbonates, for example, sodium hydroxide, potassium hydroxide, sodium carbonate, sodium bicarbonate, potassium carbonate and the like.

The term "proton acceptor" as used herein means a proton acceptor selected from an organic proton acceptor and inorganic proton acceptor, as defined herein.

The compounds of formula I are capable of forming acid addition salts with therapeutically acceptable acids. The acid addition salts may be prepared by reacting the base form of the appropriate compound of formula I with one or more equivalents, preferably with an excess, of the appropriate acid in an organic solvent, for example, diethyl ether or an ethanol-diethyl ether mixture. These salts, when administered to a mammal, possess the same pharmacologic activities as the corresponding bases. For many purposes it is preferable to administer the salts rather than the base compounds. Examples of suitable acids to form these salts include: the common mineral acids, e.g., hydrohalic, sulfuric or phosphoric acids; the organic acids, e.g. formic, acetic, maleic, malic, citric, or tartaric acid; and acids which are sparingly soluble in body fluids and which impart slow-release properties to their respective salts, e.g., pamoic acid, tannic acid or carboxymethyl cellulose. The addition salts thus obtained are the functional equivalent of the parent base compound in respect to their therapeutic use. Hence, these additional salts are included within the scope of this invention and are limited only by the requirement that the acids employed in forming the salts be therapeutically acceptable.

The discovery in the mid-1960's of two major dopamine (DA) systems indicated that this neurotransmitter exerted control over a number of physiological functions. Against this background an interest arose to develop DA receptor agonists to study the function of the dopaminergic systems and to evaluate these agonists as possible therapeutic agents in Parkinson's disease and certain neuroendocrine disorders, for example, hyperprolactinemia, galactorrhea, amenorrhea, impotence, hypertension and other central nervous system disorders.

The DA receptor agonists exert a variety of pharmacological effects, some of the most characteristic being the ones that occur in animals in which DA deficiency is brought about to mimic the Parkinsonian syndrome. An important model was developed by U. Ungerstedt, Acta. Physiol. Scand., Suppl. 367, 69—93 (1971) who, by means of unilateral injections of 6-hydroxydopamine (6-OHDA) into the DA pathway, could produce selective lesions of the ascending DA pathways on one side of the brain. Ungerstedt (1971) demonstrated in these lesioned rats that DA receptor agonists induced rotational behaviour towards the innervated side. The response is due to the development of receptor supersensitivity in the denervated striatum resulting in a higher degree of DA receptor activity on the denervated-as compared to the innervated-side after treatment with DA receptor agonists. Due to this imbalance between the two sides, a rotational behavior is elicited, the direction being always towards the less activated side. It is of interest that in the discovery of the DA receptor stimulating properties of bromocriptine, the 6-OHDA rotational model was utilized [H. Corrodi *et al.*, J. Pharm. Pharmacol., *25*, 409—412 (1973)].

In the test for rotational behavior in rats following the unilateral 6-OHDA-induced destruction of one nigrostriatal pathway, the method described by C. J. Pycock and C. D. Marsden, Europ. J. Pharmacol., *47*, 167 (1978) was followed. The rats (230-250 g) were anesthetized with sodium pentobarbital (40 mg/kg i.p.) and intracerebral injections were made using a Stoelting stereotaxic

instrument, (C. H. Stoelting Co., Chicago, Ill., U.S.A.). Unilateral injections of 6-OHDA hydrobromide (8 μg/3 μl delivered at a rate of 1 μl per min) were made into the ascending median forebrain bundle (MFB) in the lateral hypothalamus according to the coordinates of the De Groot brain atlas, J. De Groot, Verhandel, Koninkl. Ned. Akad. Wetenschap. Natuurk. *52*: 1—40 (1959), (A:+4.6, L:±1.9, V:—2.7). 6-OHDA was made up in ice-cold distilled water containing 0.2 mg/ml ascorbic acid.

Three weeks after operation, the rats were tested for rotational behavior in response to apomorphine hydrochloride (0.25 mg/kg, s.c.). Rats which consistently showed more than 5 turns/min after apomorphine were selected and the compound of formula I was then administered. The rat was immediately placed in the rotometer, described by K. Voith and J. R. Cummings, Can. J. Pharmacol., *54*, 551 (1976), and the rotation was continuously recorded until drug effect subsided. By using this test, the following compound of formula I is an effective dopamine receptor agonist (the amount of the compound, route of administration and total turns ± S.E. (standard error) during the time observed are indicated in the parenthesis): 2-[4-[2-hydroxy-2-(3,4-dimethoxyphenyl)ethyl]-1-piperazinyl]-2,4,6-cycloheptatrien-1-one (described in Example 19, at a subcutaneous dose of 5 mg/kg exhibited 2233 ± 607 turns in a four hour duration) and 2-[4-[2-hydroxy-2-(3,4-dimethoxyphenyl)ethyl]-1-piperazinyl]-2,4,6-cycloheptatrien-1-one hydrochloride (described in Example 19, at an oral dose of 10 mg/kg exhibited 1649±298 turns in a six hour duration and at an oral dose of 20 mg/kg exhibited 5167±1065 turns in an eight hour duration).

A recently developed animal model, described by G. P. Smith and R. C. Young in "Advances in Neurology", Vol. 5, F. H. McDowell and A. Barbeau, Eds., Raven Press, New York, pp. 427—432 (1974), shows that rats exhibit almost complete akinesia in an open field following the bilateral injection of 6-OHDA into the anterolateral hypothalamus. The troponylpiperazines of formula I are able to reverse this 6-OHDA- induced hypokinesia and as a result, function as dopamine receptor agonists. In this test for dopamine receptor agonists, the compounds of formula I exhibit a pharmacological response that is quantitatively comparable to that of apomophine and bromocriptine.

Experiments were performed on male Sprague-Dawley rats housed in air-conditioned quarters. The room was lighted between 0700 and 1900 hr. daily and was maintained at a temperature of 24°C ± 2°C.

The method of Smith and Young, cited above, was followed. Rats (approximately 280 g) were operated on under sodium pentobarbital anesthesia. Using a Stoelting stereotaxic instrument, the tip of a 26 gauge cannula was positioned in the anterolateral hypothalamus (7 mm anterior to the interaural line, 2 mm lateral to the midline and 8 mm below the dura) according to the De Groot brain atlas, noted above. Via a polyethylene tubing (PE 20) the cannula was connected to a 10 μl syringe which was mounted in a Starrett micrometer head drive, C. H. Stoelting Co., Chicago, Illinois, U.S.A. All injections were bilateral. Each injection consisted of 4 μl of distilled water containing 6-OHDA (6.5 μg base/μl) and ascorbic acid (0.4 μg/μl).

The animals had free access to Purina Laboratory Chow pellets and tap water. However since anterolateral hypothalamic 6-OHDA injections produce aphagia and adipsia, intragastric feeding was necessary in order to prevent drastic weight loss. The rats received a daily gastric intubation of 2 g of the "modified rat tube feeding diet" (ICN Pharmaceuticals, Inc., Cleveland, Ohio, U.S.A.) mixed with approximately 2 ml tap water.

Ambulation in the open field was evaluated in an apparatus consisting of a wooden box (69 cm × 69 cm × 42 cm) with an arborite floor. The floor was divided into 36 squares (11.5 cm × 11.5 cm). The placement of all four limbs in one square was taken as one ambulation score.

In the present experiments all compounds were evaluated four days after the intracerebral injection of 6-OHDA. The rat was placed into the center of the open field and observed for a 2-min period. Only rats with almost total akinesia were used. Apomorphine, bromocriptine or the compounds of formula I were injected s.c. to groups of 4—12 rats. Subsequently, the number of squares were counted which the animal entered during several 2-min observation periods. Apomorphine was evaluated at 5, 10, 15, 20 and 30 min; bromocriptine at 2, 3, 4, 5, 6 and 7 hr; and the compounds of formula I at 15, 30, 45, 60, 90 and 120 min after injection. Each animal was used only once. The results are expressed as cumulative number of ambulation scores which are the sums of the scores obtained during the 2-min observation periods.

The following substances were used; apomorphine hydrochloride (Macfarlan Smith Ltd., Edinburgh, Scotland), bromocriptine (CB-154) (Sandoz Pharmaceuticals, East Hanover, N.J., U.S.A.). and 6-OHDA hydrobromide (Aldrich Chemical Co., Inc., Milwaukee, Wisconsin, U.S.A.). The compounds were dissolved in distilled water or suspended in distilled water with a few drops of polysorbate 80 (Tween 80; "Tween" is a registered trade mark). If the compound was an oil, 0.4 ml of dimethyl sulfoxide was added. Solutions were prepared fresh on the day of the experiment. The 6-OHDA solution was kept in ice throughout the injection procedure. All doses refer to the base.

Using the above described method, apomorphine at a dose of 0.5 mg/kg exhibited a score of 135±41 and bromocriptine at a dose of 10 mg/kg exhibited a score of 112±23. Similarly, the following representative compounds of formula I are effective dopamine receptor agonists (the amount of the compound and its cumulative ambulation score are indicated in the parentheses): 2-(4-methyl-1-piperazinyl)-2,4,6-cycloheptatrien-1-one (described in Example 1, at a dose of 50 mg/kg exhibited a score of 278±57), 4-(2-oxo-3,5,7-

cycloheptatrien-1-yl)-1-piperazine carboxylic acid, ethyl ester (described in Example 1, at a dose of 50 mg/kg exhibited a score of 79±31), 2-[4-(2-propenyl)-1-piperazinyl]-2,4,6-cycloheptatrien-1-one (described in Example 4, at a dose of 50 mg/kg exhibited a score of 155±43), 2-[4-(2-propynyl)-1-piperazinyl]-2,4,6-cycloheptatrien-1-one (described in Example 4, at a dose of 50 mg/kg exhibited a score of 28±6), 2-[4-(1-methylethyl)-1-piperazinyl]-2,4,6-cycloheptatrien-1-one (described in Example 4, at a dose of 50 mg/kg exhibited a score of 431±113), 2-(4-ethyl-1-piperazinyl)-2,4,6-cycloheptatrien-1-one (described in Example 4, at a dose of 50 mg/kg exhibited a score of 70±15), 2-(4-propyl-1-piperazinyl)-2,4,6-cycloheptatrien-1-one (described in Example 4, at a dose of 50 mg/kg exhibited a score of 159±34), N-[2-[4-(2-oxo-3,5,7-cycloheptatrien-1-yl)-1-piperazinyl]ethyl]-acetamide (described in Example 7 at a dose of 50 mg/kg exhibited a score of 36±8), 4-(2-oxo-3,5,7-cycloheptatrien-1-yl)-1-piperazine-acetonitrile (described in Example 10, at a dose of 50 mg/kg exhibited a score of 126±51), 2-[4-(2-ethoxyethyl)-1-piperazinyl]-2,4,6-cycloheptatrien-1-one (described in Example 10, at a dose of 50 mg/kg exhibited a score of 67±16), 2-[4-[2-(3,4-dimethoxyphenyl)ethyl]-1-piperazinyl]-2,4,6-cycloheptatrien-1-one (described in Example 11, at a dose of 10 mg/kg exhibited a score of 101±28), 2-[4-[2-(3,4-dihydroxyphenyl)ethyl]-1-piperazinyl]-2,4,6-cycloheptatrien-1-one (described in Example 12, at a dose of 25 mg/kg exhibited a score of 98±57), 4-(2-oxo-3,5,7-cycloheptatrien-1-yl)-1-piperazine-acetamide (described in Example 13, at a dose of 50 mg/kg exhibited a score of 42±14), 2-[4-[2-(acetyloxy)ethyl]-1-piperazinyl]-2,4,6-cycloheptatrien-1-one (described in Example 14, at a dose of 50 mg/kg exhibited a score of 232±54), 2,2-dimethylpropanoic acid, 2-[4-(2-oxo-3,5,7-cycloheptatrien-1-yl)-1-piperazinyl]ethyl ester (described in Example 15, at a dose of 50 mg/kg exhibited a score of 137±29), 4-(2-oxo-3,5,7-cycloheptatrien-1-yl)-1-piperazineethanol (described in Example 17, at a dose of 50 mg/kg exhibited a score of 187±35), 2-[4-(3-hydroxypropyl)-1-piperazinyl]-2,4,6-cycloheptatrien-1-one (described in Example 18, at a dose of 50 mg/kg exhibited a score of 71±10), and 2-[4-[2-hydroxy-2-(3,4-dimethoxyphenyl)ethyl]-1-piperazinyl]-2,4,6-cycloheptatrien-1-one (described in Example 19, at a dose of 10 mg/kg exhibited a score of 191±47).

Furthermore, the intermediate ketones of formula V, described hereinafter, also exhibit dopamine-receptor stimulating activity in a mammal in a manner similar to the compounds of formula I. For example, in the latter test for testing dopamine receptor agonists, the following compound of formula VII is an effective dopamine receptor agonist; 2-[4-(2-oxopropyl)-1-piperazinyl]-2,4,6-cycloheptatrien-1-one (described in Example 21, at a dose of 50 mg/kg exhibited a score of 65±13).

The above described test method for dopamine receptor agonists shows that the compounds of formula I are active as dopamine receptor agonists. The compounds, thus, can be used clinically in the treatment of hyperprolactinemia, galactorrhoea, amenorrhoea, impotence, diabetes, Parkinsonism, acromegaly, hypertension and other central nervous system disorders which respond to dopamine-receptor stimulation.

The compounds of formula I of this invention are used alone or in combination with pharmacologically acceptable carriers, the proportion of which is determined by the solubility and chemical nature of the compound, chosen route of administration and standard biological practice. For example, they are administered orally in solid form i.e. capsule or tablet. They can also be administered orally in the form of suspensions or solutions or they may be injected parenterally. For parenteral administration they can be used in the form of a sterile solution containing other solutes, for example, enough saline or glucose to make the solution isotonic.

The tablet compositions contain the active ingredient in admixture with non-toxic pharmaceutical excipients known to be suitable in the manufacture of tablets. Suitable pharmaceutical excipients are, for example, starch, milk sugar, certain types of clay and so forth. The tablets can be uncoated or they can be coated by known techniques so as to delay disintegration and absorption in the gastrointestinal tract and thereby provide a sustained action over a longer period.

The aqueous suspensions of the compounds of formula I contain the active ingredient in admixture with one or more non-toxic pharmaceutical excipients known to be suitable in the manufacture of aqueous suspensions. Suitable excipients are, for example, methylcellulose, sodium alginate, gum acacia, lecithin and so forth. The aqueous suspensions can also contain one or more preservatives, one or more coloring agents, one or more flavoring agents and one or more sweetening agents.

Non-aqueous suspensions can be formulated by suspending the active ingredient in a vegetable oil, for example, arachis oil, olive oil, sesame oil, or coconut oil, or in a mineral oil, for example liquid paraffin, and the suspension may contain a thickening agent, for example beeswax, hard paraffin or cetyl alcohol. These compositions can also contain a sweetening agent, flavoring agent and antioxidant.

The dosage of the compounds of formula I as dopamine receptor agonists will vary with the form of administration and the particular compound chosen. Furthermore, it will vary with the particular host as well as the age, weight and condition of the host under treatment as well as with the nature and extent of the symptoms. Generally, treatment is initiated with small dosages substantially less than the optimum dose of the compound. Thereafter, the dosage is increased by small increments until the optimum effect under circumstances is reached. In general, the compounds of this invention are most desirably administered at a concentration level that will generally afford effective results without

causing any harmful or deleterious side effects. For example, the effective dopamine receptor stimulating amount of the compounds for i.p. administration usually ranges from about 0.1 mg to about 250 mg per kilogram body weight per day in single or divided doses although as aforementioned variations will occur. However a dosage level that is in the range of from about 1.0 to about 100 mg per kilogram body weight per day in single or divided doses is employed most desirably for i.p. administration in order to achieve effective results. For oral administration, effective amounts can range from about 1.0 to about 250 mg per kilogram body weight per day in single or divided doses preferably about 5.0 to 100 mg per kilogram body weight per day.

The compound of formula I, or a therapeutically acceptable salt thereof, also can be used to produce beneficial effects in the treatment of Parkinsonism, hyperprolactinemia and related disorders when combined with a therapeutically effective amount of an agent commonly used in the treatment of Parkinsonism, hyperprolactinemia and related disorders. Such agents include, for example, apomorphine and its derivatives, piribedil and its derivatives, dopaminergic ergot derivatives, especially bromocriptine and lergotrile, 2-amino-6,7-dihydroxy-(1,2,3,4)-tetrahydronaphthalene (ADTN), levodihydroxyphenylalanine (levodopa), combination of levodopa with carbidopa, L-prolyl-L-leucylglycinamide (MIF) and its derivatives, especially L-prolyl-N-methyl-D-leucylglycinamide (pareptide), biperiden, cycrimine hydrochloride, procyclidine, trihexyphenidyl hydrochloride, benztropine mesylate, chlorphenoxamine hydrochloride, diphenhydramine hydrochloride, orphenadrine hydrochloride, ethopropazine hydrochloride and the enzymes, monoamine oxidase B and catechol-O-methyl transferase. A combination of the foregoing agents can be substituted for a single agent. Suitable methods of administration, compositions and dosages of the agents are well known in the art; for instance, "Physican Desk Reference", 32 ed., Medical Economics Co., Oradell, N. J. U.S.A., 1978. When used in combination, the compound of formula I, or its therapeutically acceptable salt, is administered as described previously.

Reaction scheme I illustrates a method for preparing compounds of formula I.

1.

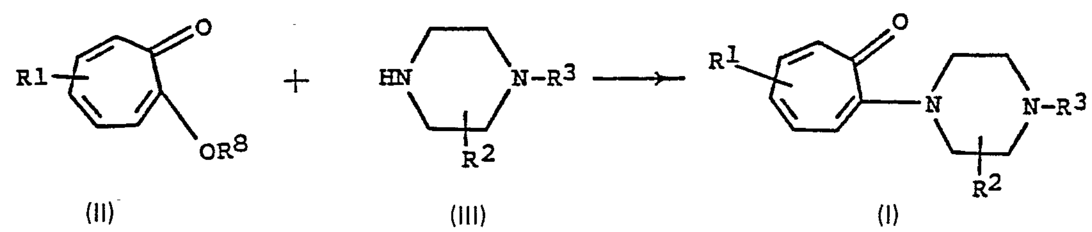

(II) (III) (I)

The 2-alkoxy-tropones, of formula II in which $R^1$ represents a substituent at positions 3, 4, 5, 6 or 7 of the 2,4,6-cycloheptatrien-1-one ring and is selected from hydrogen, halo, lower alkyl, lower alkoxy, trifluoromethyl, or 1-oxo(lower)alkylamino; and $R^8$ is lower alkyl, suitable as starting materials, are described in a number of reports; for example, see the review on tropone derivatives, their preparation and their interconversions by F. Pietra, Chem. Rev., *73*, 293 (1973). Thus, the 2-alkoxy-tropones are either known or they can be prepared by conventional means.

Also, the piperazine and piperazine derivatives of formula III are either known, commercially available or can be prepared by conventional means. For example, one useful method of preparing a compound of formula III, the appropriate nitrogen of the piperazine of formula III wherein $R^2$ is as defined herein and $R^3$ is hydrogen is first protected with an amino protecting group, for instance benzyl, formyl, tert-butoxycarbonyl and the like. The desired $R^3$ group is then introduced on to the other nitrogen of this protected piperazine; various methods of introducing this group are described hereinafter. Subsequent removal of the protecting group, e.g. hydrogenation in the case of benzyl, gives the corresponding piperazine derivative of formula III wherein $R^3$ is other than hydrogen.

With reference to reaction scheme 1, the 2-alkoxy-tropone of formula II in which $R^1$ is as defined herein and $R^8$ is lower alkyl, preferably methyl or ethyl, is condensed with the piperazine derivative of formula III in which $R^2$ and $R^3$ are as defined herein to obtain the corresponding compound of formula I in which $R^1$, $R^2$ and $R^3$ are as defined immediately above. The condensation is readily effected by heating a solution of the compound of formula II with one to five, preferably 1.3 to 2.0, molar equivalents of the piperazine of formula III in an inert organic solvent, for example, a lower alkanol, benzene, chloroform, acetonitrile, toluene and the like, preferably methanol or ethanol, at 50 to 100°C for 10 to 60 hours and isolating the corresponding compound of formula I.

By using the above condensation conditions, condensation of the compound of formula II in which $R^1$ is hydrogen with about one-half molar equivalent of the compound of formula III in which $R^2$ is as defined herein and $R^3$ is hydrogen gives the corresponding compound of formula I in which $R^1$ is hydrogen, $R^2$ is as defined herein and $R^3$ is 1-oxo-2,4,6-cycloheptatrien-2-yl.

The above described condensation of the compounds of formula II and formula III is especially useful for preparing the compounds of formula I in which $R^1$ and $R^2$ are as defined herein and $R^3$ is hydrogen, phenyl, lower alkyl, lower alkoxycarbonyl, 1-oxo-2,4,6-cycloheptatrien-2-yl or phenyl mono-, di- or trisubstituted with lower alkyl, halo, trifluoromethyl, a group of formula Alk-$R^4$ wherein Alk is as

defined herein and $R^4$ is phenyl or 1-oxo-2,4,6-cycloheptatrien-2-ylamino-, or a group of formula

$$CR^5R^6—Alk^1—R^7 \quad (\text{with } OH \text{ on } CR^5R^6)$$

wherein $Alk^1$, $R^5$ and $R^6$ are as defined herein and $R^7$ is hydrogen.

By using the above condensation conditions, condensation of the compound of formula II with about one-half molar equivalent an 1-[amino(lower)alkyl]piperazine gives the corresponding compound of formula I in which $R^1$ and $R^2$ are as defined herein and $R^3$ is a group of formula Alk-$R^4$ wherein Alk is as defined herein and $R^4$ is 1-oxo-2,4,6-cycloheptatrien-2-ylamino.

The above described compounds of formula I in which $R^1$ and $R^2$ are as defined herein and $R^3$ is hydrogen are especially useful for conversion to other compounds of formula I. In one such conversion, the latter compound of formula I in which $R^3$ is hydrogen is condensed in the presence of a proton acceptor with a halide of formula X—$R^3$ wherein X is bromo, chloro or iodo and $R^3$ is lower alkyl, lower alkenyl, lower alkynyl, cyclo(lower)alkyl, lower alkoxycarbonyl, 1-oxo(lower)alkyl, cyclo(lower)alkyl substituted with a lower alkyl; lower alkenyl substituted with phenyl; 2,3-dihydro-8-oxo-cyclohepta[b]furan-2-ylmethyl, 2,3-diacetyloxypropyl, or 2-(1,3-dihydro-1,3-dioxo-2H-isoindol-2-yl)ethyl to obtain the compound of formula I in which $R^1$ and $R^2$ are as defined herein and $R^3$ is as defined immediately above or 2-(4,5-dihydro-2-oxazolyl)benzoyl. About one to ten, preferably 1.0 to 1.5 molar equivalents of the proton acceptor and about one to five, preferably 1.0 to 1.5, molar equivalents of the halide of formula X—$R^3$ are used. In this condensation, it should be noted that the condensation of the compound of formula I in which $R^3$ is hydrogen with 2-(bromoethyl)-1,3-dihydro-2H-isoindol-1,3-dione gives a separable mixture of the compound of formula I in which $R^1$ and $R^2$ are defined herein and $R^3$ is 2-(4,5-dihydro-2-oxazolyl)benzoyl or 2-(1,3-dihydro-1,3-dioxo-2H-isoindol-2-yl)ethyl. For this condensation, suitable proton acceptors can be selected from organic and inorganic proton acceptors, for example triethylamine, pyridine, N-ethylmorpholine, sodium bicarbonate, sodium or potassium carbonate, sodium or potassium lower alkoxide and the like. Sodium or potassium carbonate is the preferred proton acceptor. Usually the condensation is conducted in an inert organic solvent, for example, benzene, toluene, dichloromethane, chloroform, lower alkanol, acetonitrile, dimethylformide, acetone and the like. Acetonitrile and/or methanol is the preferred solvent for the condensation. To achieve the condensation, the reaction mixture is preferably maintained at 20 to 85°C for three hours to three days and the compound of formula I is isolated.

In a similar manner, the compound of formula I in which $R^1$ and $R^2$ are as defined herein and $R^3$ is hydrogen may be condensed in the presence of a proton acceptor with a halide of formula X—Alk—$R^4$ wherein X is bromo, chloro or iodo; Alk is a divalent alkyl having one to six carbon atoms; and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di- or trisubstituted with lower alkyl, halo, lower alkoxy, hydroxy or trifluoromethyl, or phenoxy, mono-, di- or trisubstituted with lower alkyl, halo, lower alkoxy, acetylamino, hydroxy or trifluoromethyl or with a halide of formula

$$X—CR^5R^6—Alk^1—R^7 \quad (\text{with } OH \text{ on } Alk^1)$$

wherein $Alk^1$, $R^5$, $R^6$ and X are as defined herein and $R^7$ is hydrogen, phenyl, hydroxy, phenoxy, phenyl mono-, di- or trisubstituted with methylsulfonylamino, lower alkyl, halo, lower alkoxy, hydroxy or trifluoromethyl, or phenoxy mono-, di- or trisubstituted with lower alkyl, halo, lower alkoxy, hydroxy or trifluoromethyl; with the proviso that when $R^7$ is hydroxy, the hydroxy groups are joined to different carbon atoms to obtain the corresponding compound of formula I in which $R^1$ and $R^2$ are as defined herein and $R^3$ is a group of formula Alk—$R^4$ wherein Alk and $R^4$ are as defined immediately above or a group of formula

$$CR^5R^6—Alk^1—R^7 \quad (\text{with } OH \text{ on } Alk^1)$$

wherein $Alk^1$, $R^5$, $R^6$ and $R^7$ are as defined immediately above.

The latter conditions are also useful for the condensation of the compound of formula I in which $R^1$ and $R^2$ are as defined herein and $R^3$ is hydrogen with epichlorohydrin to give the corresponding intermediate having the 2,3-dihydroxypropyl group at position 4 of the piperazino ring. Acetylation of this intermediate, in the same manner as described hereinafter, gives the corresponding compound of formula I in which $R^1$ and $R^2$ are as defined herein and $R^3$ is 2,3-diacetyloxypropyl.

The latter condensation conditions are also useful for the condensation of the compound of

formula I in which $R^1$ and $R^2$ are as defined herein and $R^3$ is hydrogen with an epoxide of formula

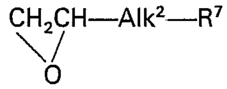

wherein $Alk^2$ is a divalent lower alkyl having one to five carbon atoms and $R^7$ is hydrogen, phenyl, hydroxy, phenoxy, phenyl mono-, di- or trisubstituted with methylsulfonylamino, lower alkyl, halo, lower alkoxy, hydroxy or trifluoromethyl, or phenoxy mono-, di- or trisubstituted with lower alkyl, halo, lower alkoxy, hydroxy or trifluoromethyl to give the corresponding compound of formula I in which $R^1$ and $R^2$ are as defined herein; and $R^3$ is a group of formula

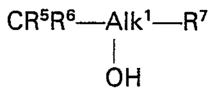 wherein $R^5$ and $R^6$ are hydrogen, 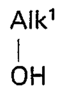 is CH(OH)-$Alk^2$

wherein $Alk^2$ is a divalent lower alkyl having one to five carbon atoms and $R^7$ is as defined immediately above.

Similarly, condensation of the compound of formula I in which $R^1$ and $R^2$ are as defined herein and $R^3$ is hydrogen with for example about one to two molar equivalents of a chloro-epoxide of the formula

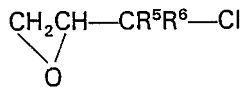 or 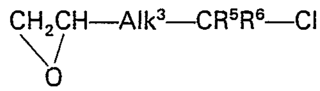

wherein $Alk^3$ is a divalent lower alkyl having one to four carbon atoms and $R^5$ and $R^6$ are as defined herein in the presence of for example about 1.4 to 1.9 molar equivalents of a proton acceptor, preferably potassium carbonate, gives the corresponding compound of formula I in which $R^1$ and $R^2$ are as defined herein; and $R^3$ is a group of formula

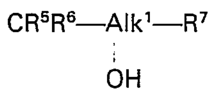

wherein $R^7$ is hydroxy and 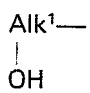 is 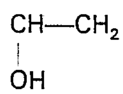 or 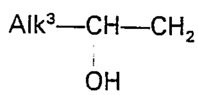

wherein $Alk^3$ is as defined herein respectively. The condensation may, for example, be carried out in an inert organic solvent, preferably acetonitrile, at 60 to 85°C for 30 to 60 hours.

Reaction of the compound of formula I in which $R^1$ and $R^2$ are as defined herein and $R^3$ is hydrogen with for example 1.5 to 3.0 molar equivalents of ethylene oxide for example in methanol at about 0°C for about 10 to 30 hours gives the corresponding compound of formula I in which $R^1$ and $R^2$ are as defined herein; and $R^3$ is a group of formula

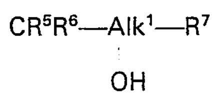

wherein $Alk^1$ is CH and $R^5$, $R^6$ and $R^7$ are hydrogen.

Acylation of the compound of formula I in which $R^1$ and $R^2$ are as defined herein and $R^3$ is hydrogen gives the corresponding compound of formula I in which $R^1$ and $R^2$ are as defined herein and $R^3$ is 1-oxo (lower)alkyl. The acylation is preferably carried out with about an equivalent of a lower alkanoyl bromide or chloride, or an excess of a lower alkanoic anhydride, in the presence of an organic proton acceptor, preferably triethylamine or pyridine, in an inert organic solvent, preferably benzene or dichloromethane, at for example 0 to 20°C for one to ten hours. When this acylation involves acetylation, a preferred method of acetylation is the reaction of the compound of formula I in which $R^3$ is hydrogen with about 5 to 20 molar equivalents of acetic anhydride at 10 to 30°C for about 10 to 60 minutes to obtain the corresponding compound of formula I in which $R^1$ and $R^2$ are defined herein and $R^3$ is acetyl.

In another series of reactions, the compound of formula I in which $R^1$ and $R^2$ are as defined herein and $R^3$ is hydrogen is condensed with 1-chloro-2-propanone to obtain the corresponding intermediate having a 2-oxopropyl group at position 4 of the piperazine ring. Reaction of this intermediate with, for example, about one molar equivalent of potassium cyanide and, for example, about two molar equivalents of ammonium carbonate in, for example, a solution of aqueous methanol at, for example, about

50 to 70°C for about 10 to 30 hours gives the corresponding compound of formula I in which $R^1$ and $R^2$ are as defined herein and $R^3$ is 5-methyl-2,4-imidazolidinedione-5-yl-methyl.

In another transformation, the compound of formula I in which $R^1$ and $R^2$ are as defined herein and $R^3$ is 2-(1,3-dihydro-1,3-dioxo-2H-isoindol-2-yl)ethyl may be reacted with for example about one molar equivalent of hydrazine hydrate e.g. in ethanol or methanol at about 20 to 30°C for about 10 to 40 hours to obtain the corresponding intermediate having an 2-aminoethyl group. Acetylation of this intermediate with, for example, acetic anhydride, in the same manner as described above, affords the corresponding compound of formula I in which $R^1$ and $R^2$ are as defined herein and $R^3$ is 2-(acetylamino)ethyl.

Another useful condensation involves the reaction of the compound of formula I in which $R^1$ and $R^2$ are as defined herein and $R^3$ is hydrogen with about one molar equivalent of a tosylate of formula TsO-Alk-$R^4$ wherein Alk is as defined herein $R^4$ is phenyl, phenoxy, phenyl mono-, di- or trisubstituted with lower alkyl, halo, lower alkoxy or trifluoromethyl, or phenoxy mono-, di- or trisubstituted with lower alkyl, halo, lower alkoxy, acetylamino or trifluoromethyl to obtain the corresponding compound of formula I in which $R^1$ and $R^2$ are as defined herein and $R^3$ is a group of formula Alk-$R^4$ wherein Alk is as defined herein and $R^4$ is as defined immediately above. This condensation may be carried out in the presence of about two molar equivalents of a proton acceptor, preferably sodium or potassium carbonate. An especially useful solvent for this condensation is acetone. The reaction mixture may be maintained at about 50 to 60°C for about 15 to 40 hours to obtain the corresponding compound of formula I.

Treatment of the above described compound of formula I in which $R^1$ and $R^2$ are as defined herein and $R^3$ is a group of formula Alk-$R^4$ wherein Alk is as defined herein and $R^4$ is phenyl mono-, di- or trisubstituted with lower alkoxy with boron tribromide gives the corresponding compound of formula I in which $R^1$ and $R^2$ are as defined herein and $R^3$ is a group of formula Alk-$R^4$ wherein Alk is as defined herein and $R^4$ is phenyl mono-, di- or trisubstituted with hydroxy. About four to ten molar equivalents of boron tribromide may be used in an inert organic solvent, preferably dichloromethane, at 10 to 30°C for about two to ten hours.

Conveniently, the reaction of the above described compound of formula I in which $R^1$ and $R^2$ are as described herein and $R^3$ is a group of formula Alk-$R^4$ wherein Alk is as defined herein and $R^4$ is cyano with five to twenty molar equivalents of concentrated sulfuric acid at 10 to 30°C for about two to five days, followed by treatment with a mixture of ice and concentrated ammonium hydroxide until the reaction is alkaline, gives the corresponding compound of formula I in which $R^1$ and $R^2$ are as defined herein and $R^3$ is a group of formula Alk-$R^4$ wherein Alk is as defined herein and $R^4$ is aminocarbonyl.

Intermediates of formula IV in which Alk, $R^1$ and $R^2$ are as defined herein

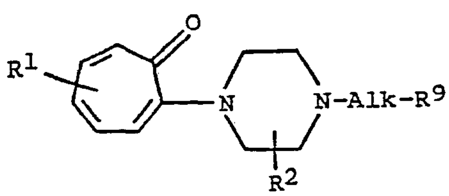

(IV)

and $R^9$ is hydroxy or chloro are also useful for preparing some compounds of formula I. A compound of formula IV in which Alk, $R^1$ and $R^2$ are as defined herein and $R^9$ is hydroxy may be prepared by condensing a compound of formula II in which $R^1$ and $R^8$ are as defined herein with a 1-[hydroxy-(lower)alkyl]-piperazine, in the same manner as described above for the condensation of the compounds of formulae II and III.

Acylation of the compound of formula IV in which Alk, $R^2$ and $R^3$ are as defined herein and $R^9$ is hydroxy gives the corresponding compound of formula I in which $R^1$ and $R^2$ are as defined herein and $R^3$ is a group of formula Alk-$R^4$ wherein Alk is as defined herein and $R^4$ is 1-oxo(lower)alkoxy. The acylation may be achieved by reacting the latter compound of formula IV with about an equimolar amount of a lower alkanoyl bromide or chloride and an organic proton acceptor, preferably triethylamine or pyridine, in an inert organic solvent, preferably benzene or dichloromethane, at 0 to 20°C for one to three days, or with an excess of a lower alkanoic anhydride in the presence of an organic proton acceptor. When this acylation involves acetylation, a preferred method of acetylation is the reaction of the compound of formula IV with about 5 to 20 molar equivalents each of acetic anhydride and pyridine at 20 to 50°C for 10 to 30 hours to obtain the corresponding compound of formula I in which $R^1$ and $R^2$ are as defined herein and $R^3$ is a group of formula Alk-$R^4$ wherein Alk is as defined herein and $R^4$ is acetyloxy.

Reaction of the compound of formula IV in which Alk, $R^1$ and $R^2$ are as defined herein and $R^9$ is hydroxy with, for example, about two to three molar equivalents of thionyl chloride in an inert organic solvent, preferably chloroform, at about 10 to 30°C for about 10 to 40 hours gives the corresponding intermediate of formula IV in which Alk, $R^1$ and $R^2$ are as defined herein and $R^9$ is chloro.

Conveniently, reaction of the compound of formula IV in which Alk, $R^1$ and $R^2$ are as defined herein and $R^9$ is chloro with about an equimolar amount of thiourea in a solution of ethanol at about 70 to 80°C for about one to five hours gives the corresponding intermediate having an [(aminoimino-methyl)amino]thio group. Acetylation of this intermediate with, for example, an excess of acetic

anhydride, about 50 to 20 molar equivalents, at about 40 to 80°C for about 30 minutes to five hours gives the corresponding compound of formula I in which $R^1$ and $R^2$ are as defined herein and $R^3$ is a group of formula Alk-$R^4$, wherein Alk is as defined herein and $R^4$ is acetylthio.

Another method of preparing the compounds of formula I in which $R^1$ and $R^2$ are as defined herein and $R^3$ is a group of formula

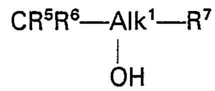

wherein $Alk^1$, $R^5$ and $R^6$ are as defined herein and $R^7$ is hydrogen, hydroxy, phenyl, phenoxy, phenyl mono-, di- or trisubstituted with methylsulfonylamino, lower alkyl, halo, lower alkoxy, hydroxy or trifluoromethyl, or phenoxy mono-, di- or trisubstituted with lower alkyl, halo, lower alkoxy, hydroxy or trifluoromethyl involves for example, the reaction of the compound of formula I in which $R^1$ and $R^2$ are as defined herein and $R^3$ is hydrogen with about 1.1 to 1.5 molar equivalents of a proton acceptor, preferably potassium carbonate, and about one molar equivalent of an alkanone of the formula

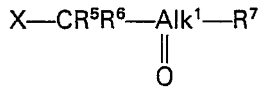

wherein X is bromo, chloro or iodo; $Alk^1$, $R^5$ and $R^6$ are as defined herein, and $R^7$ is hydrogen, hydroxy, phenyl, phenoxy, phenyl mono-, di or tri-substituted with methylsulfonylamino, lower alkyl, halo, lower alkoxy, hydroxy or trifluoromethyl, or phenoxy mono-, di or trisubstituted with lower alkyl, halo, lower alkoxy, hydroxy or trifluoromethyl, with the proviso that when $R^7$ is hydroxy the hydroxy and ketone groups are joined to different carbon atoms, in an inert organic solvent, preferably acetonitrile, at 50 to 90°C for one to ten hours to obtain the corresponding intermediate ketone of formula V in which $R^1$, $R^2$, $R^5$, $R^6$ and $Alk^1$ are as defined herein and $R^7$ is as defined immediately above. Reduction of this intermediate with, for example, five to ten molar equivalents of a reducing agent, preferably sodium borohydride, in an inert organic solvent preferably

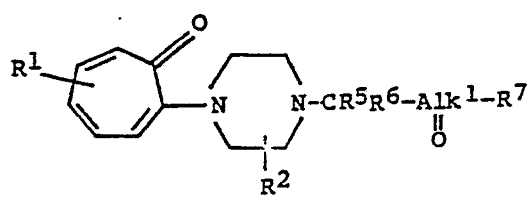

(V)

a mixture of methanol and chloroform, at 10 to 30°C for one-half to ten hours gives the corresponding compound of formula I in which $R^1$ and $R^2$ are as defined herein and $R^3$ is a group of formula

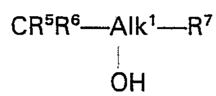

wherein $Alk^1$, $R^5$ and $R^6$ are as defined herein and $R^7$ is hydrogen, hydroxy, phenyl, phenoxy, phenyl mono-, di- or trisubstituted with methylsulfonylamino, lower alkyl, halo, lower alkoxy, hydroxy or trifluoromethyl, or phenoxy mono-, di or trisubstituted with lower alkyl, halo, lower alkoxy, hydroxy or trifluoromethyl, with the proviso that when $R^7$ is hydroxy, the hydroxy groups are joined to different carbon atoms.

The following examples illustrate further this invention.

Example 1

2-[4-(3-Trifluoromethylphenyl)-1-piperazinyl]-2,4-6-cycloheptatrien-1-one (I: $R^1$ and $R^2$ = H and $R^3$ = 3-trifluoromethylphenyl)

A mixture of 1-(3-trifluoromethylphenyl)-piperazine (2.3 g) and 2-methoxy-2,4,6-cycloheptatrien-1-one (2.0 g) in methanol (50 ml) was refluxed for 24 hr and evaporated. The residue was chromatographed on silica gel (100 g) using ethyl acetate. The eluates were evaporated and crystallized from ethyl acetate-benzene to give the title compound (4.08 g): mp 112—114°C; ir($CHCl_3$) 1570 $cm^{-1}$; uv max(MeOH) 350 ($\varepsilon$=10575) and 256 nm ($\varepsilon$=31730); nmr($CDCl_3$) $\delta$ 3.5 (m, 8H) and 7.0 (m, 9H); and *Anal.* Calcd. for $C_{18}H_{17}F_3N_2O$: C, 64.66% H, 5.12% N, 8.38% and Found: C, 64.84% H, 5.17% N, 8.54%.

In the same manner, but replacing 1-(3-trifluoromethylphenyl)-piperazine with an equivalent amount of 1-methylpiperazine, 1-(2-methylphenyl)piperazine, 1-(4-chlorophenyl)-3-methylpiperazine, 1-piperazine carboxylic acid ethyl ester, 1-(1,1-dimethylethyl)piperazine, 2-methylpiperazine or 1-phenylpiperazine and when desired, preparing the acid addition salt of the compound of formula I by using the appropriate acid; the following compounds of formula I were obtained respectively: 2-(4-methylpiperazinyl)-2,4,6-cycloheptatrien-1-one (Z)-2-butenedioate (I: $R^1$ and $R^2$=H and $R^3$=Me): mp 118—120°C (crystallized from acetone-diethyl ether); ir($CHCl_3$) 2400, 1705, 1620, 1350 and 1570 $cm^{-1}$; uv max(MeOH) 343 ($\varepsilon$=9440) and 250 nm ($\varepsilon$=14975); and nmr($CDCl_3$) $\delta$ 2.9 (s, 3H), 3.5 (m, 8H), 6.27 (s, 2H), 7.0 (m, 5H) and 14.5 (broad, 2H); 2-[4-(2-methylphenyl)-1-piperazinyl]-2,4,6-cycloheptatrien-1-one (I: $R^1$ and $R^2$=H and $R^3$=2-methylphenyl): mp 115—116°C (crystallized from diethyl ether-hexane); ir($CHCl_3$) 1565 $cm^{-1}$; uv max(MeOH) 353 ($\varepsilon$=10000) and 253 nm ($\varepsilon$=20095); nmr($CDCl_3$) $\delta$ 2.33 (s, 3H), 3.05 (m, 4H), 3.45 (m, 4H) and 6.9 (m, 9H); and *Anal.* Calcd. for $C_{18}H_{20}N_2O$: C, 77.20% H, 7.14% N, 10.0% and Found: C, 76.97% H, 7.21% N, 9.86%; 2-[4-(4-chlorophenyl)-2-methyl-1-piperazinyl]-2,4,6-cycloheptatrien-1-one (I: $R^1$=H, $R^2$=2-Me and $R^3$=4-chlorophenyl): mp 120—121°C (crystallized from diethyl ether-hexane); ir($CHCl_3$) 1565 $cm^{-1}$; uv max(MeOH) 352 ($\varepsilon$=7250) and 259 nm ($\varepsilon$=22285); nmr($CDCl_3$) $\delta$ 1.13 (d, 3H), 3.5 (m, 7H) and 6.9 (m, 9H); and *Anal. Calcd. for* $C_{18}H_{19}ClN_2$: C, 68.60% H, 6.08% N, 8.89% and Found: C, 68.61% H, 6.15% N, 8.86%; 4-(2-oxo-3,5,7-cycloheptatrien-1-yl)-1-piperazine carboxylic acid ethyl ester (I: $R^1$ and $R^2$=H and $R^3$=ethoxycarbonyl): mp 95—98°C (crystallized from diethyl ether); ir($CHCl_3$) 1690 and 1570 $cm^{-1}$; uv max(MeOH) 350 ($\varepsilon$=9629), 254 ($\varepsilon$=13089) and 225 nm ($\varepsilon$=12171); nmr($CDCl_3$) $\delta$ 1.3 (t, 3H), 3.35 (m, 4H), 3.67 (m, 4H), 4.2 (q, 2H) and 6.9 (m, 5H); and *Anal.* Calcd. for $C_{14}H_{18}N_2O_3$: C, 64.1% H, 6.92% N, 10.68% and Found: C, 64.19% H, 7.03% N, 10.53%; 2-[4-(1,1-dimethylethyl)-1-piperazinyl]-2,4,6-cycloheptatrien-1-one (Z)-2-butenedioate (I: $R^1$ and $R^2$=H and $R^3$=1,1-dimethylethyl): mp 160—162°C (crystallized from diethyl ether-methanol); ir(mull) 2400, 1700 and 1570 $cm^{-1}$; uv max(MeOH) 343 ($\varepsilon$=9180) and 250 nm ($\varepsilon$=14380); nmr($CDCl_3$) $\delta$ 1.45 (s, 9H), 3.50 (m, 8H), 6.20 (s, 2H) and 6.90 (m, 5H); and *Anal. Calcd for* $C_{15}H_{22}N_2O.C_4H_4O_4$: C, 62.97% H, 7.23% N, 7.73% and Found: C, 62.56% H, 7.33% N, 7.41%; 2-(3-methyl-1-piperazinyl)-2,4,6-cycloheptatrien-1-one (I: $R^1$ and $R^3$=H and $R^2$=3-Me); and 2-(4-phenyl-1-piperazinyl)-2,4,6-cycloheptatrien-1-one: (I: $R^1$ and $R^2$=H and $R^3$=Ph): mp 110—112°C (crystallized from ethyl acetate-hexane); ir($CHCl_3$) 1570 $cm^{-1}$; uv max(MeOH) 350 ($\varepsilon$=10280), 254 ($\varepsilon$=26020) and 230 nm ($\varepsilon$=15445); nmr($CDCl_3$) $\delta$ 3.45 (8H) and 7.05 (10H); and *Anal.* Calcd for $C_{17}H_{18}N_2O$: C, 76.66% H, 6.81% N, 10.52% and Found: C, 76.54% H, 6.78% N, 10.45%.

Similarly, by condensing 1-methylpiperazine with 7-bromo-2-methoxy-2,4,6-cycloheptatrien-1-one or 5-acetylamino-2-methoxy- 2,4,6-cycloheptatrien-1-one and when desired, preparing the acid addition salt of the compound of formula 1 by using the appropriate acid, the following compounds of formula I were obtained, respectively: 7-bromo-2(4-methyl-1-piperazinyl)-2,4,6-cycloheptatrien-1-one methane sulfonate salt (I: $R^1$=7-Br, $R^2$=H and $R^3$=Me): mp 196—197°C (crystallized from diethyl ether-methanol); ir(mull) 2600, 1570, 1155 and 1030 $cm^{-1}$; uv max(MeOH) 352 ($\varepsilon$=9225) and 270 nm ($\varepsilon$=13800): nmr(DMSO-$d_6$) $\delta$ 2.35 (s, 3H), 2.85 (s, 3H), 3.35 (m, 8H), 7.00 (m, 3H) and 8.15 (m, 1H); and *Anal.* Calcd. for $C_{12}H_{15}BrN_2O.CH_3SO_3H$: C, 41.16% H, 5.05% N, 7.39% and Found: C, 41.32% H, 5.01% N, 7.35% and 5-acetylamino-2-(4-methyl-1-piperazinyl)-2,4,6-cycloheptatrien-1-one (I: $R^1$=5-acetylamino, $R^2$=H and $R^3$=Me): mp 122—123°C (crystallized from methanol); ir($CHCl_3$) 3420, 3280, 1680 and 1540 $cm^{-1}$; uv max(MeOH) 252 nm ($\varepsilon$=15230); nmr($CDCl_3$) $\delta$ 2.15 (s, 3H), 2.30 (s, 3H), 2.55 (t, 4H), 3.30 (t, 4H), 7.10 (m, 4H) and 8.0 (s, 1H); and *Anal.* Calcd for $C_{14}H_{19}N_3O_2$: C, 64.34% H, 7.33% N, 16.08% and Found: C, 63.87% H, 7.28% N, 15.96%.

### Example 2

2,2′-(1,4-Piperazindiyl)-bis-2,4,6-cycloheptatrien-1-one (I: $R^1$ and $R^2$=H and $R^3$=1-oxo-2,4,6-cycloheptatrien-2-yl)

To a solution of piperazine (7.74 g) in methanol (10 ml) at 70°C, a solution of 2-methoxy-2,4,6-cycloheptatrien-1-one (8.16 g) in methanol (50 ml) was added drop by drop. The reaction mixture was heated at 95°C for 5 hr, cooled and evaporated. The residue was dissolved in chloroform and water. The organic extract was separated, dried and evaporated. The residue was chromatographed on silica gel (250 g) using chloroform then 5% methanol in chloroform, increasing gradually the concentration of methanol up to 20%. The product was recrystallized from methanol to yield 2.9 g of the title compound: mp 184—186°C; ir($CHCl_3$) 1565 $cm^{-1}$; uv max(MeOH) 355 ($\varepsilon$=19075), 256 ($\varepsilon$=29080) and 223 nm ($\varepsilon$=22460); nmr($CDCl_3$) $\delta$ 3.55 (m, 8H) and 7.0 (m, 10H); and *Anal.* Calcd. for $C_{18}H_{18}N_2O_2$: C, 73.45% H, 6.16% N, 9.52% and Found: C, 73.76% H, 6.17% N, 9.45%.

### Example 3

2-(1-Piperazinyl)-2,4,6-cycloheptatrien-1-one (I: $R^1$, $R^2$ and $R^3$=H)

A solution of 2-methoxy-2,4,6-cycloheptatrien-1-one (136 g) and piperazine (136 g) in methanol (250 ml) was refluxed for 4 hr and the reaction vessel was placed in an ice bath. Water (150 ml), then acetic acid was slowly added until the solution was acidic. The mixture was filtered and the filtrate was evaporated and chromatographed on silica gel using chloroform-acetone (1:1) and then with acetic acid-methanol (1:4). The eluates from the latter solvent were evaporated to give an oil of the acetate salt (153 g) of the title compound.

Alternatively, the reaction solution was cooled to induce crystallization of the dimer and filtered. The filtrate was diluted with acetone to 1000 ml and a solution of methane sulfonic acid (106 g) in

acetone (250 ml) was added to the filtrate in an ice bath. The precipitate was collected and washed with acetone and diethyl ether to give 146 g of the methane sulfonate salt of the title compound: mp 174—176°C; ir(mull) 2900, 1563 and 1180 $cm^{-1}$; uv max(MeOH) 343 ($\varepsilon$=8910), 252 ($\varepsilon$=13110) and 223 nm ($\varepsilon$=11250); nmr(DMSO-$d_6$) $\delta$ 2.35 (s, 3H), 3.35 (m, 8H), 6.95 (m, 5H) and 8.8 (broad, 2H); and *Anal.* Calcd. for $C_{11}H_{14}N_2$—O.$CH_3SO_3H$: C, 50.32% H, 6.33% N, 9.80% and Found: C, 50.06% H, 6.39% N, 9.44%.

Example 4

2-[4-(2-Propenyl)-1-piperazinyl]-2,4,6-cycloheptatrien-1-one (I: $R^1$ and $R^2$=H, and $R^3$=2-propenyl)

A mixture of 2-(1-piperazinyl)-2,4,6-cycloheptatrien-1-one acetate (15.5 g, described in Example 3), 2-propenyl bromide (7.0 g) and potassium carbonate (13.8 g) in acetonitrile (100 ml) was stirred at room temperature for 16 hr, diluted with water and extracted with chloroform. The chloroform extract was dried and evaporated. The residue (11.0 g) was chromatographed on silica gel (100 g) using chloroform-acetone (1:1) to give 4.7 g of the title compound. The latter material was dissolved in acetone and a solution of maleic acid (2.5 g) in acetone was added. The crystals were collected to obtain 6.4 g of the (Z)-2-butenedioate salt of the title compound: mp 136—138°C; ir($CHCl_3$) 2400, 1900, 1705 and 1570 $cm^{-1}$; uv max(MeOH) 344 ($\varepsilon$=11370) and 250 nm ($\varepsilon$=17900); nmr($CDCl_3$) $\delta$ 3.5 (m, 10H), 5.6 (m, 3H), 6.3 (s, 2H), 7.0 (m, 5H) and 12.4 (s, 2H); and *Anal.* Calcd. for $C_{14}H_{18}N_2O.C_4H_4O_4$: C, 62.42% H, 6.40% N, 8.09% and Found: C, 62.16% H, 6.57% N, 8.31%.

In the same manner but replacing 2-propenyl bromide with an equivalent amount of 2-propynyl bromide, 2-bromopropane, ethyl iodide, 1-bromopropane, 2-bromobutane, bromocyclopentane, bromocyclopropane, 3-chloro-2-methyl-1-propene or 1-bromobutane and when desired, omitting the salt preparation step, the following compounds of formula I were obtained respectively: 2-[4-2-propynyl)-1-piperazinyl]-2,4,6-cycloheptatrien-1-one-(Z)-butenedioate (I: $R^1$ and $R^2$=H and $R^3$=2-propynyl): mp 145—146°C (crystallized from acetone); ir(mull) 3220, 2500 and 1565 $cm^{-1}$; uv max(MeOH) 345 ($\varepsilon$=9125) and 252 ($\varepsilon$=14150); nmr($CDCl_3$) $\delta$ 2.65 (t, 1H), 3.5 (m, 8H), 3.95 (d, 2H), 6.3 (s, 2H), 6.95 (m, 5H) and 13.2 (s, 2H); and *Anal.* Calcd. for $C_{14}H_{16}N_2O.C_4H_4O_4$: C, 62.78% H, 5.80% N, 8.14% and Found: C, 62.31% H, 6.20% N, 8.37%; 2-[4-(1-methylethyl)-1-piperazinyl]-2,4,6-cycloheptatrien-1-one (I: $R^1$ and $R^2$=H and $R^3$=1-methylethyl): mp 51.5—52.5°C (crystallized from hexane): ir($CHCl_3$) 1560 $cm^{-1}$; uv max(MeOH) 351 ($\varepsilon$=9815), 254 ($\varepsilon$=14730) and 222 nm ($\varepsilon$=11720); nmr($CDCl_3$) $\delta$ 1.05 (d, 6H), 2.65 (m, 5H), 3.35 (t, 4H) and 6.75 (m, 5H); and *Anal.* Calcd. for $C_{14}H_{20}N_2O$: C, 72.37% H, 8.67% N, 12.05% and Found: C, 72.05% H, 8.78% N, 11.74%; 2-(4-ethyl-1-piperazinyl)-2,4,6-cycloheptatrien-1-one (I: $R^1$ and $R^2$=H and $R^3$=Et): ir($CHCl_3$) 1560 $cm^{-1}$; uv max(MeOH) 350 ($\varepsilon$=8760), 255 ($\varepsilon$=13135) and 223 nm ($\varepsilon$=10545); nmr($CDCl_3$) $\delta$ 1.10 (t, 3H), 2.45 (q, 2H), 2.60 (t, 4H), 3.35 (t, 4H) and 6.80 (m, 5H); and *Anal.* Calcd. for $C_{13}H_{18}N_2O$: C, 71.52% H, 8.31% N, 12.83% and Found: C, 71.86% H, 8.05% N, 12.48%; 2-(4-propyl-1-piperazinyl)-2,4,6-cycloheptatrien-1-one (I: $R^1$ and $R^2$=H and $R^3$=propyl); ir($CHCl_3$) 1565 $cm^{-1}$; uv max(MeOH) 351 ($\varepsilon$=8780), 255 ($\varepsilon$=13180) and 222 nm($\varepsilon$=10810); nmr($CDCl_3$) $\delta$ 0.9 (t, 3H), 1.55 (m, 2H), 2.40 (m, 2H), 2.65 (t, 4H), 3.35 (t, 4H) and 6.80 (m, 5H); and *Anal.* Calcd. for $C_{14}H_{20}N_2O$: C, 72.37% H, 8.68% N, 12.06% and Found: C, 71.75% H, 8.76% N, 11.95%; 2-[4-(1-methylpropyl)-1-piperazinyl]-2,4,6-cycloheptatrien-1-one (Z)-2-butenedioate (I: $R^1$ and $R^2$=H and $R^3$=1-methylpropyl): mp 120—121°C (crystallized from diethyl ether-methanol); ir(mull) 2500, 1695, 1585, 1564 and 1375 $cm^{-1}$; uv max(MeOH) 343 ($\varepsilon$=9240) and 251 nm ($\varepsilon$=14425); nmr(DMSO-$d_6$) $\delta$ 0.9 (t, 3H), 1.23 (d, 3H), 1.6 (m, 2H), 3.35 (m, 9H), 6.0 (s, 2H) and 7.0 (m, 5H); and *Anal.* Calcd. for $C_{15}H_{22}N_2O.C_4H_4O_4$: C, 62.97% H, 7.23% N, 7.73% and Found: C, 62.76% H, 7.18% N, 7.46%; 2-(4-cyclopentyl-1-piperazinyl)-2,4,6-cycloheptatrien-1-one (I: $R^1$ and $R^2$=H and $R^3$=cyclopentyl): mp 102—103°C (crystallized from hexane); ir($CHCl_3$) 1611 and 1555 $cm^{-1}$; uv max(MeOH) 350 ($\varepsilon$=9720), 254 ($\varepsilon$=14370) and 221 nm($\varepsilon$=12200); nmr($CDCl_3$) $\delta$ 1.56 (m, 8H), 2.50 (m, 1H), 2.65 (t, 4H), 3.35 (t, 4H) and 6.30—7.30 (m, 5H); and *Anal.* Calcd. for $C_{16}H_{22}N_2O$: C, 74.38% H, 8.58% N, 10.84% and Found: C, 74.66% H, 8.75% N, 10.79%; 2-(4-cyclopropyl-1-piperazinyl)-2,4,6-cycloheptatrien-1-one (I: $R^1$ and $R^2$=H and $R^3$=cyclopropyl); 2-[4-(2-methyl-2-propenyl)-1-piperazinyl]-2,4,6-cycloheptatrien-1-one (I: $R^1$ and $R^2$=H and $R^3$=2-methyl-2-propenyl): ir($CHCl_3$) 1560 $cm^{-1}$; uv max(MeOH) 351 ($\varepsilon$=10425), 255 ($\varepsilon$=16015) and 220 nm($\varepsilon$=13640); nmr($CDCl_3$) $\delta$ 1.75 (s, 3H), 2.53 (t, 4H), 2.9 (s, 2H), 3.35 (t, 4H), 4.85 (s, 2H) and 6.85 (m, 5H); and *Anal.* Calcd. for $C_{15}H_{20}N_2O$: C, 73.73% H, 8.25% N, 11.47% and Found: C, 73.04% H, 8.26% N, 11.35%; and 2-(4-butyl-1-piperazinyl)-2,4,6-cycloheptatrien-1-one hydrochloride (I: $R^1$ and $R^2$=H and $R^3$=butyl): mp 192—194°C (crystallized from diethyl ether-ethyl acetate); ir (nujol) 3450, 2500 and 1553 $cm^{-1}$; uv max(MeOH) 342, 250 and 223 nm: nmr(DMSO-$d_6$) $\delta$ 0.9 (t, 3H), 1.3 (m, 2H), 1.75 (m, 2H), 3.15 (m, 4H), 3.35 (m, 4H), 3.85 (m, 2H), 6.95 (m, 5H) and 11.5 (b, 1H); and *Anal.* Calcd. for $C_{15}H_{23}ClN_2O$: C, 59.83% H, 8.31% N, 9.30% and Found: C, 59.95% H, 8.49% N, 9.23%.

In the same manner but replacing 2-propenyl bromide with an equivalent amount of 2-(2-bromoethyl)-1,3-dihydro-2H-isoindol-1,3-dione and omitting the salt preparation step, the following two compounds of formula I were obtained, namely; 1,3 - dihydro - 2 - [[4 - (2 - oxo - 3,5,7 - cycloheptatrien - 1 - yl) - 1 - piperazinyl]ethyl] - 2H - isoindol - 1,3 - dione (I: $R^1$ and $R^2$=H and $R^3$=2-(1,3-dihydro-1,3-dioxo-2H-isoindol-2-yl)ethyl): mp 124—126°C; ir($CHCl_3$) 1770, 1705 and 1560 $cm^{-1}$; uv max(MeOH) 352 ($\varepsilon$=8540), 255 ($\varepsilon$=13450), 241 ($\varepsilon$=18610) and 219 nm ($\varepsilon$=52770);

nmr($CDCl_3$) δ 2.7 (m, 6H), 3.3 (t, 4H), 3.85 (t, 2H), 6.8 (m, 5H) and 7.75 (m, 4H); and *Anal.* Calcd. for $C_{21}H_{21}N_3O_3$: C, 69.41% H, 5.82% N, 11.56% and Found: C, 68.79% H, 5.77% N, 11.30%; and a second product, 2 - [4 - [2 - (4,5 - dihydro - 2 - oxazolyl)benzoyl] - 1 - piperazinyl] - 2,4,6 - cycloheptatrien - 1 - one (I: $R^1$ and $R^2$=H and $R^3$=2-(4,5-dihydro-2-oxazolyl)benzoyl: mp 195—195°C; ir($CHCl_3$) 1630 and 1565 $cm^{-1}$; uv max(MeOH) 350 (ε=10140) and 251 nm (ε=20715); nmr($CDCl_3$) δ 3.3 (m, 6H), 4.15 (m, 6H) and 7.2 (m, 9H); and *Anal.* Calcd. for $C_{21}H_{21}N_3O_3$: C, 69.41% H, 5.82% N, 11.56% and Found: C, 68.97% H, 5.77% N, 11.22%.

## Example 5

2-(4-Acetyl-1-piperazinyl)-2,4,6-cycloheptatrien-1-one (I: $R^1$ and $R^2$=H and $R^3$=acetyl)

A solution of 2-(1-piperazinyl)-2,4,6-cycloheptatrien-1-one acetate (8.0 g, described in Example 3) in acetic anhydride (50 ml) was allowed to react at room temperature for 15 min. Methanol (20 ml) was then added, and the solution was evaporated. The residue (7.3 g) was chromatographed on 75 g silica gel with methanol-ethyl acetate (15:85) to yield 5.3 g of purified product. Recrystallization of 4 g from ethyl acetate-hexane afforded 2.9 g of the title compound: mp 64—66°C; ir($CHCl_3$) 3660, 3400, 1635 and 1570 $cm^{-1}$; uv max(MeOH) 350 (ε=9920) and 254 nm (ε=13140); nmr($CDCl_3$) δ 2.10 (s, 3H), 3.30 (m, 4H), 3.70 (m, 4H) and 6.80 (m, 5H); and *Anal.* Calcd. for $C_{13}H_{16}N_2O_2$.7.01% $H_2O$: C, 62.50% H, 7.23% N, 11.21% and Found: C, 62.21% H, 7.16% N, 11.21%.

## Example 6

5-Methyl-5-[[4-(2-oxo-3,5,7-cycloheptatrien-1-yl)-1-piperazinyl]methyl]-2,4-imidazolidinedione (I: $R^1$ and $R^2$=H and $R^3$=5-methyl-2,4-imidazolidinedione-5-ylmethyl)

A mixture of 2-(1-piperazinyl)-2,4,6-cycloheptatrien-1-one (12.4 g, described in Example 3), 1-chloro-2-propanone (5.55 g), potassium carbonate (11.04 g) and acetonitrile (120 ml) was refluxed for 4 hr and filtered. Chloroform and water were added to the filtrate. The organic phase was separated, washed with water, dried and evaporated. The residue was chromatographed on silica gel using methanol-ethyl acetate and the eluates were evaporated to give an oil (6.21 g) of 2-[4-(2-oxopropyl)-1-piperazinyl]-2,4-6-cycloheptatrien-1-one: ir($CHCl_3$) 3660, 3360, 1720, 1710, 1660 and 1560 $cm^{-1}$; uv max(MeOH) 351 (ε=9310), 255 (ε=13915) and 221 nm(ε=11750); nmr($CDCl_3$) δ 2.12 (s, 3H), 2.62 (t, 4H), 3.21 (s, 2H), 3.35 (s, 4H) and 6.40—7.10 (m, 5H); and *Anal.* Calcd. for $C_{14}H_{18}N_2O)_2$: C, 68.26% H, 7.37% N, 11.37% and Found: C, 67.83% H, 7.59% N, 11.44%.

To a solution of the latter compound (7.5 g) in methanol (10 ml) heated to 55°C was added a solution of ammonium carbonate (5.78 g) in 15 ml of water, followed by the addition of potassium cyanide (1.98 g) in 4.5 ml of water. The reaction mixture was maintained at 55°C for 16 hr, cooled to room temperature and filtered to yield 6.0 g of crude product. Recrystallization from methanol gave 5.4 g of the title compound: mp 231—232°C; ir(mull) 3160, 1770, 1710 and 1535 $cm^{-1}$; uv max(MeOH) 353 (ε=9600), 255 (ε=14520) and 218 nm(ε=12800); nmr(DMSO-$d_6$) δ 1.20 (s, 3H), 2.60 (m, 6H), 3.20 (t, 4H), 6.90 (m, 5H), 7.75 (s, 1H) and 10.40 (s, 1H); and *Anal.* Calcd. for $C_{16}H_{20}N_4O_3$: C, 60.74% H, 6.37% N, 17.7% and Found: C, 60.84% H, 6.40% N, 17.59%.

## Example 7

N-[2-[4-(2-Oxo-3,5,7-cycloheptatrien-1-yl)-1-piperazinyl]ethyl]acetamide (I: $R^1$ and $R^2$ = H and $R^3$ = 2-(acetylamino)ethyl)

To a solution of 1,3-dihydro-2-[4-(2-oxo-3,5,7-cycloheptatrien-1-yl)ethyl]-2H-isoindol-1,3-dione (8.7 g, described in Example 4) in ethanol, hydrazine hydrate (1.305 g) was added and the solution was stirred at room temperature for 24 hr. It was then acidified with 1N hydrochloric acid, left at room temperature for 2 hr and the precipitate was removed by filtration. The filtrate was dissolved in water, made alkaline with diluted sodium hydroxide and extracted with chloroform. The chloroform was evaporated and the residue (6 g) was chromatographed on 100 g silica gel with acetic acid-ethyl acetate (2:8) to yield 4 g of 2-[4-[(2-aminoethyl)-1-piperazinyl]-2,4,6-cycloheptatrien-1-one. A mixture of this compound (4.0 g) and acetic anhydride (40 ml) was allowed to react at room temperature for 48 hr. The acetic anhydride was evaporated at reduced pressure and the residue was chromatographed on 200 g silica gel with methanol to yield 1.6 g of the title compound: mp 117—118°C; ir($CHCl_3$) 3410, 3330, 1665 and 1560 $cm^{-1}$; uv max(MeOH) 356 (ε=9780) and 255 nm (ε=14600); nmr($CDCl_3$)δ 2.0 (s, 3H), 2.6 (m, 6H), 3.35 (m, 6H), 6.0 (broad, 1H) and 6.85 (m, 5H); and *Anal.* Calcd for $C_{15}H_{21}N_3O_2$: C, 65.43% H, 7.69% N, 15.26% and Found: C, 65.09% H, 7.67% N, 15.11%.

## Example 8

2-[4-(2,3-Diacetyloxypropyl)-1-piperazinyl]-2,4,6-cycloheptatrien-1-one (I: $R^1$ and $R^2$ = H and $R^3$ = 2,3-diacetyloxypropyl)

A mixture of 2-(1-piperazinyl)-2,4,6-cycloheptatrien-1-one acetate (12.4 g, described in Example 3), epichlorohydrin (5.55 g), potassium carbonate (11.04 g) and acetonitrile (120 ml) was refluxed for 48 hr and filtered. The filtrate was evaporated and the residue (9 g) was chromatographed twice on silica gel with methanol-ethyl acetate (1:9) to yield 2.92 g of a mixture of 2-[4-(2,3-dihydroxypropyl)-1-piperazinyl]-2,4,6-cycloheptatrien-1-one and 1,3-bis[4-(2-oxo-3,5,7-cycloheptatrien-1-yl)-piperazinyl]-2-propanol.

A solution of this mixture (2.8 g) in pyridine (20 ml) and acetic anhydride (20 ml) was stirred at room temperature for 24 hr and evaporated. The residue was dissolved in ethyl acetate, and the solution was washed with 1N sodium hydroxide, then with water, dried over magnesium sulfate and evaporated. The residue (1.9 g) was chromatographed on 100 g silica gel first with ethyl acetate to give the title compound (0.66 g): ir($CHCl_3$) 1730, 1612, 1560 and 1250 $cm^{-1}$; uv max(MeOH) 357 ($\varepsilon$=4520) and 255 nm ($\varepsilon$=6750); nmr($CDCl_3$)$\delta$ 2.08 (s, 6H), 2.55 (d, 2H), 2.65 (m, 4H), 3.25 (m, 4H), 4.09 and 4.36 (2d, 2H), 5.20 (m, 1H) and 6.68—6.99 (m, 5H); and *Anal.* Calcd for $C_{18}H_{24}N_2O_5$: C, 62.05% H, 6.94% N, 8.04% and Found: C, 61.99% H, 7.10% N, 7.70%. The above silica gel column was then eluted with acetone. The eluates were evaporated to give a residue (1.2 g) which was crystallized from diethyl ether-ethyl acetate to give 0.35 g of 1,3-bis[4-(2-oxo-3,5,7-cycloheptatrien-1-yl)-1-piperazinyl]-2-propanol acetate: mp 104—106°C; ir($CHCl_3$) 1724, 1612 and 1555 $cm^{-1}$; uv max(MeOH) 358 ($\varepsilon$=19380), 255 ($\varepsilon$=29100) and 220 nm ($\varepsilon$=23020); nmr($CDCl_3$)$\delta$ 2.06 (s, 3H), 2.55 (d, 4H), 2.65 (t, 8H), 3.33 (t, 8H), 5.20 (m, 1H) and 6.50—7.30 (m, 10H); and *Anal.*Calcd for $C_{27}H_{34}N_4O_4$: C, 67.76% H, 7.16% N, 11.71% and Found: C, 67.45% H, 7.10% N, 11.44%.

## Example 9

2-[4-(Phenylmethyl)-1-piperazinyl]-2,4,6-cycloheptatrien-1-one (I: $R^1$ and $R^2$ = H and $R^3$ = phenylmethyl)

A mixture of 1-(phenylmethyl)-piperazine (3.2 g) and 2-methoxy-2,4,6-cycloheptatrien-1-one (1.6 g) in methanol (6 ml) was refluxed for 10 hr and evaporated. The residue was dissolved in a mixture of chloroform and water. The organic phase was separated, dried and evaporated. The residue was chromatographed on silica gel (200 g) using methanol-chloroform (5:95). The eluates were evaporated to give the title compound (2.7 g): mp 78—80°C; ir($CHCl_3$) 2400, 1705, 1620, 1350 and 1570 $cm^{-1}$; uv max(MeOH) 343 ($\varepsilon$=9440) and 250 nm ($\varepsilon$=14975); nmr($CDCl_3$)$\delta$ 2.9 (s, 3H), 3.5 (m, 8H), 6.27 (s, 2H), 7.0 (m, 5H) and 14.5 (broad, 2H); and *Anal.* Calcd for $C_{18}H_{20}N_2O_2$: C, 77.11% H, 7.19% N, 9.99% and Found: C, 77.08% H, 7.17% N, 9.74%.

In the same manner, but replacing 1-(phenylmethyl)-piperazine with an equivalent amount of N-(2-oxo-2,4,6-cycloheptatrien-1-yl)-1-piperazinyl-ethanamine, the following compound of formula I was obtained: N,4-bis-(2-oxo-3,5,7-cycloheptatrien-1-yl)-1-piperazine-ethanamine (I: $R^1$ and $R^2$ = H and $R^3$ = 2-(1-oxo-2,4,6-cycloheptatrien-2-ylamino)ethyl): mp 130—132°C (crystallized from ethyl acetate); ir($CHCl_3$) 3300 and 1555 $cm^{-1}$; uv max(MeOH) 340 ($\varepsilon$=22740) and 248 nm ($\varepsilon$=40,285); nmr($CDCl_3$) $\delta$ 2.8 (m, 6H), 3.4 (m, 6H), 6.9 (m, 10H) and 7.6 (broad, 1H); and *Anal.* Calcd for $C_{20}H_{23}N_3O_2$: C, 71.19% H, 6.87% N, 12.45% and Found: C, 71.60% H, 7.09% N, 12.40%.

## Example 10

4-(2-Oxo-3,5,7-cycloheptatrien-1-yl)-1-piperazine-acetonitrile (I: $R^1$ and $R^2$ = H and $R^3$ = cyanomethyl)

A mixture of 2-(1-piperazinyl)-2,4,6-cycloheptatrien-1-one acetate (10.0 g, described in Example 3), chloroacetonitrile (13.2 g) and potassium carbonate (14.5 g) in acetonitrile (110 ml) was stirred at room temperature for 16 hr, and filtered. The filtrate was evaporated. The residue was chromatographed on silica gel (300 g) using ethyl acetate-acetone (4:1) to give 1.9 g of the title compound, which was then crystallized from acetone-diethyl ether: mp 133—135°C; ir($CHCl_3$) 2230 and 1565 $cm^{-1}$; uv max(MeOH) 350 ($\varepsilon$=9810), 254 ($\varepsilon$=14575) and 222 nm ($\varepsilon$=11960); nmr($CDCl_3$)$\delta$ 2.75 (t, 4H), 3.4 (t, 4H), 3.55 (s, 2H) and 7.0 (m, 5H); and *Anal.* Calcd for $C_{13}H_{15}N_3O$: C, 68.4% H, 6.56% N, 18.38% and Found: C, 68.15% H, 6.68% N, 18.25%.

In the same manner but replacing chloroacetonitrile with an equivalent amount of ethyl bromoacetate, 2-phenoxyethyl bromide, 2-ethoxyethyl bromide, 2-(2-chloroethoxy)-ethanol, phenethyl bromide, 2-[4-(1,1-dimethylethyl)phenoxy]ethyl bromide, 2-(4-chlorophenoxy)ethyl bromide, 2-[4-(acetylamino)phenoxy]ethyl bromide, 2-(3-indolyl)ethyl bromide, 2-(1H-imidazol-4-yl)ethyl bromide, 1-(p-toluenesulfonyl)-2-(4-fluorophenyl)ethane or 3,4-dimethoxyphenylmethyl bromide, the following compounds of formula I were obtained respectively: 4-(2-oxo-3,5,7-cycloheptatrien-1-yl)-1-piperazine acetic acid, ethyl ester (Z)-2-butenedioate (I: $R^1$ and $R^2$ = H and $R^3$ = ethoxycarbonylmethyl): mp 106—116°C (crystallized from acetone-diethyl ether); ir(mull) 2340, 1738, 1695 and 1553 $cm^{-1}$; uv max(MeOH) 344 ($\varepsilon$=7630) and 251 nm ($\varepsilon$=12080); nmr(DMSO-$d_6$)$\delta$ 1.25 (t, 3H), 3.0 (m, 4H), 3.4 (m, 4H), 3.7 (s, 2H), 4.15 (q, 2H), 6.15 (s, 2H) and 6.9 (m, 5H); and *Anal.* Calcd for $C_{15}H_{20}N_2O_3 \cdot C_4H_4O_4 1/2H_2O$: C, 56.85% H, 6.28% N, 6.98% and Found: C, 56.52% H, 6.27% N, 6.74%; 2-[4-(2-phenoxyethyl)-1-piperazinyl]-2,4,6-cycloheptatrien-1-one (I: $R^1$ and $R^2$ = H and $R^3$ = 2-phenoxyethyl): mp 94—95°C (crystallized from ethyl acetate-diethyl ether); ir($CHCl_3$) 1565 $cm^{-1}$; uv max(MeOH) 351 ($\varepsilon$=9840), 256 ($\varepsilon$=15580) and 219 nm ($\varepsilon$=21075); nmr($CDCl_3$) $\delta$ 2.75 (m, 6H), 3.35 (m, 4H), 4.10 (t, 2H) and 7.0 (m, 9H); and *Anal.* Calcd for $C_{19}H_{22}N_2O_2$: C, 73.52% H, 7.08% N, 9.02% and Found: C, 73.43% H, 7.16% N, 8.97%; 2-[4-(2-ethoxyethyl)-1-piperazinyl]-2,4,6-cycloheptatrien-1-one (I: $R^1$ and $R^2$ = H and $R^3$ = 2-ethoxyethyl): mp 137—138°C (crystallized from acetone-diethyl ether); ir($CHCl_3$) 2450, 1900 and 1705 $cm^{-1}$; uv max(MeOH) 344 ($\varepsilon$=9470) and 251 nm ($\varepsilon$=14940); nmr($CDCl_3$) $\delta$ 1.2 (t, 3H), 3.55 (m, 14H), 6.2 (s, 2H), 6.9 (m, 5H) and 13.3 (broad, 2H); and *Anal.* Calcd

for $C_{15}H_{22}N_2O_2 \cdot C_4H_4O_4$: C, 60.30% H, 6.87% N, 7.40% and Found: C, 60.27% H, 6.89% N, 7.17%; 2-[4-[2-(2-hydroxyethoxy)ethyl]-1-piperazinyl]-2,4,6-cycloheptatrien-1-one (I: $R^1$ and $R^2$ = H and $R^3$ = 2-(2-hydroxyethoxy)ethyl): ir($CHCl_3$) 3660, 3400, 1555 and 1612 $cm^{-1}$; uv max(MeOH) 351 ($\varepsilon$=8825), 255 ($\varepsilon$=12810) and 222 nm ($\varepsilon$=10330); and nmr($CDCl_3$) $\delta$ 2.65 (m, 6H), 3.34 (t, 4H), 3.60 (m, 6H), and 6.60—6.95 (m, 5H); 2-[4-(2-phenylethyl)-1-piperazinyl]-2,4,6-cycloheptatrien-1-one (I: $R^1$ and $R^2$ = H and $R^3$=2-phenylethyl): ir($CHCl_3$) 1365 $cm^{-1}$; uv max(MeOH) 255 nm ($\varepsilon$=12290) and nmr($CDCl_3$) $\delta$ 2.75 (m, 8H), 3.35 (m, 4H) and 7.0 (m, 10H); 2-[4-[2-[4-(1,1-dimethylethyl)phenoxy]ethyl]-1-piperazinyl]-2,4,6-cycloheptatrien-1-one (I: $R^1$ and $R^2$ = H and $R^3$ = 2-[4-(1,1-dimethylethyl)phenoxy]ethyl): ir($CHCl_3$) 1565 $cm^{-1}$; uv max(MeOH) 351 ($\varepsilon$=9830), 256 ($\varepsilon$=15290) and 223 nm ($\varepsilon$=22515); nmr($CDCl_3$) $\delta$ 1.30 (s, 9H), 2.80 (m, 6H), 3.40 (m, 4H), 4.15 (t, 2H) and 7.00 (m, 9H); and *Anal.* Calcd for $C_{23}H_{30}N_2O_2$: C, 75.37% H, 8.25% N, 7.65% and Found: C, 75.07% H, 8.15% N, 7.67%; 2-[4-[2-(4-chlorophenoxy)ethyl]-1-piperazinyl]-2,4,6-cycloheptatrien-1-one (I: $R^1$ and $R^2$ = H and $R^3$ = 2-(4-chlorophenoxy)ethyl): mp 89—91°C (crystallized from ethyl acetate); ir($CHCl_3$) 1565 $cm^{-1}$; uv max(MeOH) 351 ($\varepsilon$=9575), 255 ($\varepsilon$=14625) and 227 nm ($\varepsilon$=23650); nmr($CDCl_3$) $\delta$ 2.80 (m, 6H), 3.40 (t, 4H), 4.10 (t, 2H) and 7.00 (m, 9H); and *Anal.* Calcd for $C_{19}H_{21}ClN_2O_2$: C, 66.17% H, 6.14% N, 8.12% and Found: C, 65.94% H, 6.13% N, 8.09%; 2-[4-[2-[4-(acetylamino)phenoxy]ethyl]-1-piperazinyl]-2,4,6-cycloheptatrien-1-one (I: $R^1$ and $R^2$ = H and $R^3$ = 2-[4-(acetylamino)phenoxy]ethyl): mp 141—143°C; ir($CHCl_3$) 3430, 3300, 1680 and 1560 $cm^{-1}$; uv max(MeOH) 351 ($\varepsilon$=9990) and 252 nm ($\varepsilon$=30610); nmr($CDCl_3$) $\delta$ 2.10 (s, 3H), 2.80 (m, 6H); 3.40 (t, 4H); 4.10 (t, 4H) and 7.00 (m, 9H); and *Anal.* Calcd for $C_{21}H_{25}N_3O_3$: C, 68.64% H, 6.86% N, 11.44% and Found: C, 68.48% H, 6.76% N, 11.31%; 2-[4-[2-(3-indolyl)ethyl]-1-piperazinyl]-2,4,6-cycloheptatrien-1-one (I: $R^1$ and $R^2$ = H and $R^3$ = 2-(3-indolyl)ethyl): mp 128—129°C (crystallized from ethyl acetate-hexane); ir(mull) 3310 and 1555 $cm^{-1}$; uv max(MeOH) 352 ($\varepsilon$=9700), 290 ($\varepsilon$=8680), 257 ($\varepsilon$=16960) and 222 nm ($\varepsilon$=46990); nmr(DMSO-$d_6$) $\delta$ 2.60 (m, 8H), 3.25 (m, 4H), 7.00 (m, 10H) and 10.2 (s, 1H); and *Anal.* Calcd for $C_{21}H_{23}N_3O$: C, 75.65% H, 6.95% N, 12.60% and Found: C, 75.53% H, 7.11% N, 12.47%; 2-[4-[2-(1H-imidazol-4-yl)ethyl]-1-piperazinyl]-2,4,6-cycloheptatrien-1-one (I: $R^1$ and $R^2$ = H and $R^3$ = 2-(1H-imidazol-4-yl)ethyl): mp 139—141°C (crystallized from acetone-diethyl ether); ir(mull) 2800 and 1560 $cm^{-1}$; uv max(MeOH) 350 ($\varepsilon$=9970) and 255 nm ($\varepsilon$=14915); nmr($CDCl_3$) $\delta$ 2.57 (m, 8H), 3.40 (m, 4H) and 7.00 (m, 7H); and *Anal.* Calcd for $C_{16}H_{20}N_4O$: C, 67.58% H, 7.09% N, 19.71% and Found: C, 67.10% H, 7.09% N, 19.64%; 2-[4-[2-(4-fluorophenyl)ethyl]-1-piperazinyl]-2,4,6-cycloheptatrien-1-one hydrochloride (I: $R^1$ and $R^2$ = H and $R^3$ = 2-(4-fluorophenyl)ethyl): mp 208—210°C; ir(nujol) 2280 $cm^{-1}$; uv max(MeOH) 343 ($\varepsilon$=9700), 251 ($\varepsilon$=14315) and 222 nm ($\varepsilon$=11555); nmr(DMSO-$d_6$) $\delta$ 3.4 (m, 12H) and 7.1 (m, 9H); and *Anal.* Calcd for $C_{19}H_{21}FN_2O$: C, 65.40% H, 6.35% N, 8.06% and Found: C, 65.27% H, 6.37% N, 8.04%; and 2-[4-(3,4-dimethoxyphenyl)methyl-1-piperazinyl]-2,4,6-cycloheptatrien-1-one (I: $R^1$ and $R^2$ = H and $R^3$ = 3,4-dimethoxyphenylmethyl): mp 98—100°C (crystallized from diethyl ether-ethyl acetate); *Anal.* Calcd for $C_{20}H_{24}N_2O_3$: C, 70.57% H, 7.11% N, 8.23% and Found: C, 70.23% H, 7.27% N, 7.90%.

Example 11

2-[4-[2-(3,4-Dimethoxyphenyl)ethyl]-1-piperazinyl]-2,4,6-cycloheptatrien-1-one (I: $R^1$ and $R^2$ = H and $R^3$ = 2-(3,4-dimethoxyphenyl)ethyl)

To a solution of 3,4-dimethoxyphenethanol (4.4 g) in methylene chloride (15 ml) containing triethylamine (2.7 g or 3.7 ml), was added gradually a solution of tosyl chloride (5.07 g) in methylene chloride (15 ml). The mixture was stirred at room temperature overnight. The mixture was diluted with chloroform and washed with water. The organic layer was separated, dried and evaporated to yield a crude product (11.7 g). The product was passed through a column of silica gel (250 g) and the eluates were evaporated to give 3,4-dimethoxyphenethyl tosylate (7.2 g): ir($CHCl_3$) 1600 and 1500 $cm^{-1}$; and nmr($CDCl_3$)$\delta$ 2.4 (s, 3H), 2.85 (m, 2H), 3.72 (s, 3H), 3.75 (s, 3H), 4.15 (t, 2H), 6.6 (m, 2H), 7.23 (m, 3H) and 7.64 (m, 2H).

A mixture of the latter compound (15.4 g), 2-(1-piperazinyl)-2,4,6-cycloheptatrien-1-one acetate (10.4 g, described in Example 3) and potassium carbonate (12.54 g) in acetone (105 ml) was refluxed for 24 hr and evaporated. A solution of the residue in chloroform was washed with water, dried and evaporated. The residue was treated with a solution of hydrogen chloride in diethyl ether and the precipitate was collected and crystallized from methanol to obtain the hydrochloride salt (8.53 g) of the title compound, mp 175—178°C. The latter salt (3.2 g) was dissolved in water and 10% sodium hydroxide was added until the solution was alkaline. After extraction with chloroform, the extract was dried, evaporated and crystallized from diethyl ether-hexane to obtain the title compound (2.0 g): mp 83—85°C; ir($CHCl_3$) 1565, 1200 and 1145 $cm^{-1}$; uv max(MeOH) 350 ($\varepsilon$=9860), 256 ($\varepsilon$=15130) and 227 nm ($\varepsilon$=19500); nmr($CDCl_3$) $\delta$ 2.7 (m, 2H), 3.4 (m, 4H), 3.85 (s, 6H) and 6.8 (m, 8H); and *Anal.* Calcd for $C_{21}H_{26}N_2O_3$: C, 71.16% H, 7.39% N, 7.91% and Found: C, 71.31% H, 7.62% N, 7.78%.

Example 12

2-[4-[2-(3,4-Dihydroxyphenyl)ethyl]-1-piperazinyl]-2,4,6-cycloheptatrien-1-one (I: $R^1$ and $R^2$ = H and $R^3$ = 2-(3,4-dihydroxyphenyl)ethyl)

A solution of 2-[4-[2-(3,4-dimethoxyphenyl)ethyl]-1-piperazinyl]-2,4,6-cycloheptatrien-1-one (1.10 g, described in Example 11) in methylene chloride (5 ml) was cooled to −65°C in dry ice bath. A

solution of boron tribromide (3.36 g) in methylene chloride (5 ml) was added dropwise to the solution of the compound. After the addition, the temperature was allowed to rise to room temperature. After 4 hr, the mixture was cooled in an ice bath and methanol (20 ml) was added dropwise. The resulting mixture was slowly brought to reflux and the solid was allowed to dissolve. The solution was then concentrated to the point at which crystals start to separate. Some diethyl ether was added, the mixture was cooled overnight, and filtered to give crude product (1.4 g). Recrystallization from methanol gave the hydrobromide salt (0.5 g) of the title compound: mp 256—258°C; ir(nujol) 3490, 2540 and 1522 $cm^{-1}$; uv max(MeOH) 342 ($\varepsilon$=7820), 250 ($\varepsilon$=10720) and 222 nm ($\varepsilon$=11535); nmr(DMSO-$d_6$) $\delta$ 3.25 (m, 12H), 6.8 (m, 7H) and 8.7 (broad, 2H); and *Anal.* Calcd for $C_{19}H_{22}N_2O_3 \cdot HBr$: C, 56.02% H, 5.69% N, 6.88% and Found: C, 55.91% H, 5.74% N, 6.86%.

Example 13

4-(2-Oxo-3,5,7-cycloheptatrien-1-yl)-1-piperazine-acetamide (I: $R^1$ and $R^2$ = H and $R^3$ = aminocarbonylmethyl)

A solution of 4-(2-oxo-3,5,7-cycloheptatrien-1-yl)-1-piperazine-acetonitrile (3.0 g, described in Example 10) in conc. sulfuric acid (30 ml) was allowed to stay at room temperature for 3 days. The reaction mixture was poured over ice and conc. ammonium hydroxide slowly while stirring, then extracted with chloroform. Evaporation of the solvent left 2.7 g crude product, which was recrystallized from methanol to yield 2.2 g of the title compound: mp 169—171°C; ir($CHCl_3$) 3500, 3430, 3370, 1685 and 1563 $cm^{-1}$; uv max(MeOH) 351 ($\varepsilon$=9920), 254 ($\varepsilon$=14810) and 221 nm ($\varepsilon$=12090); nmr($CDCl_3$) $\delta$ 2.75 (t, 4H), 3.09 (s, 2H), 3.35 (t, 4H), 6.00 (2H) and 6.8 (m, 5H); and *Anal.* Calcd for $C_{13}H_{17}N_3O_2$: C, 63.14% H, 6.93% N, 16.99% and Found: C, 63.24% H, 7.00% N, 16.99%.

Example 14

2-[4-[2-(Acetyloxy)ethyl]-1-piperazinyl]-2,4,6-cycloheptatrien-1-one (I: $R^1$ and $R^2$ = H and $R^3$ = 2-(acetyloxy)ethyl)

A mixture of 1-(2-hydroxyethyl)piperazine (10.0 g) and 2-methoxy-2,4,6-cycloheptatrien-1-one (10.9 g) in methanol (50 ml) was refluxed for 24 hr and evaporated. The residue was chromatographed on silica gel (100 g) using methanol-ethyl acetate (1:4). The eluates were evaporated and crystallized from ethyl acetate-hexane to give 4-(2-oxo-3,5,7-cycloheptatrien-1-yl)-1-piperazine-ethanol (9.2 g): mp 85—86°C; ir($CHCl_3$) 3440, 1616 and 1555 $cm^{-1}$; nmr ($CDCl_3$) $\delta$ 2.55 (t, 2H), 2.63 (m, 4H), 3.30 (m, 4H), 3.60 (t, 2H) and 6.40—7.65 (m, 5H); and *Anal.* Calcd for $C_{13}H_{18}N_2O_2$: C, 66.64% H, 7.74% N, 11.96% and Found: C, 66.12% H, 7.83% N, 11.99%.

To a solution of the latter compound (0.70 g) in pyridine (5 ml), acetic anhydride (15 ml) was added and the reaction mixture was allowed to stay at room temperature for 16 hr and evaporated. The residue was chromatographed on 50 g of silica gel with methanol-ethyl acetate (20:80) to yield 950 mg of still impure product. It was rechromatographed on 100 g silica gel with methanol-ethyl acetate (5:95) to obtain 780 mg of the pure title compound as an oil: ir($CHCl_3$) 1730 and 1560 $cm^{-1}$; uv max(MeOH) 351 ($\varepsilon$=9445) and 255 nm ($\varepsilon$=14240); and nmr($CDCl_3$) $\delta$ 2.08 (s, 3H), 2.7 (t, 6H), 3.35 (t, 4H), 4.2 (t, 2H) and 6.9 (m, 5H).

Example 15

2,2-Dimethylpropanoic Acid, 2-[4-(2-Oxo-3,5,7-cycloheptatrien-1-yl)-1-piperazinyl]ethyl Ester (I: $R^1$ and $R^2$ = H, and $R^3$ = 2-(2,2-dimethyl-1-oxopropoxy)ethyl

To a solution of 4-(2-oxo-3,5,7-cycloheptatrien-1-yl)-1-piperazine-ethanol (7.04 g, described in Example 14) in methylene chloride (100 ml,) triethylamine (4.3 g) was added followed by the dropwise addition of 2,2-dimethylpropanoyl chloride. The resulting solution was allowed to stay at room temperature for 3 days, then washed with water, dried over sodium sulfate and evaporated. The residue was chromatographed twice on 250 g of silica gel with ethyl acetate to yield 3.5 g of the title compound. The title compound (3.0 g) was dissolved in acetone (15 ml) and a solution of maleic acid (1.16 g) in acetone (15 ml) was added. Diethyl ether (100 ml) was added to give the (Z)-2-butenedioate salt (2.5 g) of the title compound: mp 101—102°C; ir(mull) 2350, 1723, 1700 and 1545 $cm^{-1}$; uv max(MeOH) 343 ($\varepsilon$ = 8040) and 251 nm($\varepsilon$ = 12645); nmr(DMSO-$d_6$) $\delta$ 1.2 (m, 9H), 3.3 (m, 10H), 4.3 (m, 2H), 6.1 (s, 2H) and 6.95 (m, 5H); and *Anal.* Calcd for $C_{18}H_{26}N_2O_3.C_4H_4O_4$: C, 60.82% H, 6.96% N, 6.45% and Found: C, 60.74% H, 7.01% N, 6.16%.

Example 16

2-[4-[2-(Acetylthio)ethyl]-1-piperazinyl]-2,4,6-cycloheptatrien-1-one (I: $R^1$ and $R^2$ = H and $R^3$ = 2-(acetylthio)ethyl)

A mixture of 4-(2-oxo-3,5,7-cycloheptatrien-1-yl)-1-piperazine-ethanol (10.0 g, described in Example 14), potassium carbonate (26 g) and chloroform (150 ml) was stirred and cooled in an ice-bath. Thionyl chloride (11.7 g) was added dropwise over a period of 30 min and allowed to react with stirring at room temperature for 16 hr. Water (50 ml) was added and extracted with chloroform. After drying over magnesium sulfate, the extract was evaporated and the residue (11.0 g) was chromatographed on 500 g silica gel with ethyl acetate to yield 6.2 g of 2-[4-(2-chloroethyl)-1-

piperazinyl]-2,4,6-cycloheptatrien-1-one mp 71—73°C; ir($CHCl_3$) 1560 $cm^{-1}$; uv max(MeOH) 352 ($\varepsilon = 9730$) and 255 nm($\varepsilon = 14300$); nmr($CDCl_3$) $\delta$2.75 (m, 6H), 3.5 (m, 6H) and 7.0 (m, 5H) and *Anal.* Calcd for $C_{13}H_{17}ClN_2O$: C, 61.77% H, 6.78% N, 11.08% and Found: C, 61.60% H, 6.79% N, 10.98%.

A mixture of the latter compound (2.53 g), thiourea (800 mg) and ethanol (25 ml) was refluxed for 2 hr and evaporated. The residue was dissolved in water and washed with chloroform. The aqueous layer was freeze-dried to yield 3.06 g of the hydrochloride salt of 2-[4-[2-[[(aminoiminomethyl)-amino]thio]ethyl]-1-piperazinyl]-2,4,6-cycloheptatrien-1-one. A part of this salt (1.5 g) was stirred at 50°C with 25 ml acetic anhydride for 45 min and cooled to room temperature. Methanol (10 ml) was added and the solution was evaporated to dryness in vacuo, to obtain 2.87 g of crude product. It was dissolved in ethyl acetate, washed first with 10% sodium hydroxide, then with water, dried over sodium sulfate and evaporated to give a residue (1.2 g). This residue was chromatographed on silica gel using ethyl acetate to give the title compound (0.39 g): mp 75—77°C; ir($CHCl_3$) 1680, 1610 and 1560 $cm^{-1}$; uv max(MeOH) 351 ($\varepsilon = 8960$), 254 ($\varepsilon = 13930$) and 225 nm($\varepsilon = 14100$); nmr($CDCl_3$) $\delta$ 2.67 (m, 6H), 3.03 (t, 2H), 3.34 (t, 4H) and 6.60—7.20 (m, 5H); and *Anal.* Calcd for $C_{15}H_{20}N_2O_2S$: C, 61.63% H, 6.90% N, 9.58% and Found: C, 61.68% H, 6.95% N, 9.46%.

Example 17

4-(2-Oxo-3,5,7-cycloheptatrien-1-yl)-1-piperazine-ethanol (I: $R^1$ and $R^2 = H$ and $R^3 = $ 2-hydroxyethyl)

A mixture of 1-(2-hydroxyethyl)piperazine (10.0 g) and 2-methoxy-2,4,6-cycloheptatrien-1-one (10.9 g) in methanol (50 ml) was refluxed for 24 hr and evaporated. The residue was chromatographed on silica gel (100 g) using methanol-ethyl acetate (1.4). The eluates were evaporated and crystallized from ethyl acetate-hexane to give the title compound (9.2 g): mp 85—86°C; ir ($CHCl_3$) 3440, 1616 and 1555 $cm^{-1}$; nmr($CDCl_3$) $\delta$ 2.55 (t, 2H), 2.63 (m, 4H), 3.30 (m, 4H), 3.60 (t, 2H) and 6.40—7.65 (m, 5H); and *Anal.* Calcd for $C_{13}H_{18}N_2O_2$: C, 66.64% H, 7.74% N, 11.96% and Found: C, 66.12% H, 7.83% N, 11.99%.

Similarly, by condensing 1'-(2-hydroxyethyl)piperazine with 5-chloro-2-methoxy-2,4,6-cycloheptatrien-1-one or 7-bromo-2-methoxy-2,4,6-cycloheptatrien-1-one, the following compounds of formula I were obtained respectively: 5-chloro-2-[4-(2-hydroxyethyl)-1-piperazinyl]-2,4,6-cycloheptatrien-1-one (I: $R^1 = $ 5-Cl, $R^2 = H$, and $R^3 = $ 2-hydroxyethyl): ir($CHCl_3$) 3450 and 1563 $cm^{-1}$; uv max (MeOH 259 ($\varepsilon = 12300$) and 253 nm ($\varepsilon = 10340$); and nmr ($CDCl_3$) $\delta$ 2.2 (s, 1H), 2.7 (m, 6H), 3.4 (t, 4H), 3.65 (t, 2H) and 7.0 (m, 4H); and 7-bromo-2-[4-(2-hydroxyethyl)-1-piperazinyl]-2,4,6-cycloheptatrien-1-one hydrobromide (I: $R^1 = $ 7-Br, $R^2 = H$ and $R^3 = $ 2-hydroxyethyl): mp 214—215°C (crystallized from ethyl acetate-methanol); ir(mull) 3350, 2700 and 1577 $cm^{-1}$; uv max(MeOH) 252 ($\varepsilon = 17260$) and 221 nm ($\varepsilon = 18760$); nmr(DMSO-$d_6$) $\delta$ 3.4 (m, 12H) 5.2 (s, 1H) and 7.0—8.15 (m, 5H); and *Anal.* Calcd for $C_{13}H_{17}N_2O_2$ HBr: C, 39.61% H, 4.60% N, 7.10% and Found: C, 39.83% H, 4.65% N, 7.31%.

Example 18

2-[4-(3-Hydroxypropyl)-1-piperazinyl]-2,4,6-cycloheptatrien-1-one (I: $R^1$ and $R^2 = H$ and $R^3 = $ 3-hydroxypropyl)

A mixture of 2-(1-piperazinyl)-2,4,6-cycloheptatrien-1-one acetate (15.5 g, described in Example 3), 3-bromopropanol (10.5 g) and potassium carbonate (13.8 g) in acetonitrile (150 ml) was stirred at room temperature for 16 hr, diluted with water (50 ml) and extracted with chloroform. The chloroform extract was dried and evaporated. The residue (16.0 g) was chromatographed on silica gel (500 g) using methanol-acetone (1:1). After evaporation of the eluate, the residue was crystallized from ethyl acetate-hexane to give 1.38 g of the title compound: mp 98—100°C; ir($CHCl_3$) 3260 and 1565 $cm^{-1}$; uv max (MeOH) 350 ($\varepsilon = 9410$), 255 ($\varepsilon = 14180$) and 227 nm($\varepsilon = 11470$); nmr($CDCl_3$) $\delta$ 1.75 (m, 2H), 2.65 (m, 6H), 3.5 (m, 7H) and 6.81 (m, 5H); and *Anal.* Calcd for $C_{14}H_{20}N_2O_2$: C, 67.71% H, 8.12% N, 11.28% and Found: C, 67.38% H, 8.38% N, 11.08%. In the same manner but replacing 3-bromopropanol with an equivalent amount of 3-[4-(1,1-dimethylethyl)phenoxy]-1,2-epoxypropane, 3-(4-chlorophenoxy)-1,2-epoxypropane, 2-bromopropanol or 3-bromo-1,2-propandiol, the following compounds of formula I were obtained respectively: 2-[4-[3-[4-(1,1-dimethylethyl)phenoxy]-2-hydroxypropyl]-1-piperazinyl]-2,4,6-cycloheptatrien-1-one (I: $R^1$ and $R^2 = H$ and $R^3 = $ 3-[4-(1,1-dimethylethyl)phenoxy]-2-hydroxypropyl); mp 88—90°C (crystallized from ethyl acetate-hexane); ir(mull) 3300, 1560 and 1250 $cm^{-1}$; uv max(MeOH) 352 ($\varepsilon = 7420$), 280 ($\varepsilon = 5520$), 257 ($\varepsilon = 11720$) and 223 nm($\varepsilon = 21680$); nmr($CDCl_3$) $\varepsilon$1.3 (s, 9H), 2.7 (m, 6H), 3.35 (t, 4H), 4.00 (m, 3H) and 7.00 (m, 9H); and *Anal.* Calcd for $C_{24}H_{32}N_2O_3$: C, 72.69% H, 8.14% N, 7.07% and Found: C, 72.98% H, 8.21% N, 7.01%; 2-[4-[3-(4-chlorophenoxy)-2-hydroxypropyl]1-piperazinyl]-2,4,6-cycloheptatrien-1-one (I: $R^1$ and $R^2 = H$ and $R^3 = $ 3-(4-chlorophenoxy)-2-hydroxypropyl) mp 110—112°C (crystallized from ethyl acetate-hexane); ir($CHCl_3$) 3570, 3410 and 1560 $cm^{-1}$; uv max(MeOH) 353 ($\varepsilon = 9950$), 256 ($\varepsilon = 15360$) and 227 nm($\varepsilon = 24710$), nmr($CDCl_2$) $\delta$ 2.70 (m, 6H), 3.35 (t, 4H), 4.00 (m, 3H) and 6.90 (m, 9H); and *Anal.* Calcd for $C_{20}H_{23}ClNO_3$: C, 64.07% H, 6.18% N, 7.47% and Found: C, 63.37% H, 6.19% N, 7,31%; 2-[4-[1-(hydroxymethyl)ethyl]-1-piperazinyl]-2,4,6-cycloheptatrien-1-one (Z)-2-butenedioate (I: $R^1$ and $R^2 = H$ and $R^3 = $ 1-(hydroxymethyl)ethyl): mp 115—116°C (crystallized from methanoldiethyl ether) ir($CHCl_3$) 3670, 3320, 1705 and 1570 $cm^{-1}$; uv

max(MeOH) 342 ($\varepsilon = 9505$) and 250 nm($\varepsilon = 15975$); nmr($CDCl_3$) $\delta$1.35 (d, 3H), 3.6 (m, 11H), 6.2 (s, 2H), 7.0 (m, 5H) and 11.0 (m, 3H); and *Anal.* Calcd for $C_{14}H_{20}N_2O_2$. $C_4H_4O_4$: C, 59.32% H, 6.64% N, 7.69% and Found: C, 59.11% H, 6.72% N, 7.63% and 2-[4-(2,3-dihydroxypropyl)-1-piperazinyl]-2,4,6-cycloheptatrien-1-one (I: $R^1$ and $R^2$ = H and $R^3$ = 2,3-dihydroxypropyl): mp 111—112°C (crystallized from ethyl acetate-hexane); ir ($CHCl_3$) 3580, 3420 and 1565 $cm^{-1}$; uv max (MeOH) 351 ($\varepsilon = 9700$), 255 ($\varepsilon = 14500$) and 221 nm($\varepsilon = 11400$); nmr($CDCl_3$) $\delta$2.6 (m, 6H), 3.05 (s, 1H), 3.35 (t, 4H), 3.65 (m, 3H) and 6.8 (m, 5H); and *Anal.* Calcd for $C_{14}H_{20}N_3O_3$: C, 63.61% H, 7.62% N, 10.60% and Found: C, 63.43% H, 7.87% N, 10.55%.

## Example 19

2-[4-[2-Hydroxy-2-(3,4-dimethoxyphenyl)ethyl]-1-piperazinyl]-2,4,6-cycloheptatrien-1-one (I: $R^1$ and $R^2$ = H and $R^3$ = 2-hydroxy-2-(3,4-dimethoxyphenyl)ethyl)

A solution of bromine (30.6 g, 1.2 eg) in chloroform (150 ml) was added dropwise to a solution of 1-(3,4-dimethoxyphenyl)-1-ethanone (28.8 g) in chloroform (50 ml) over a period of 3—5 hr. The mixture was stirred for 1.5 hr. A stream of nitrogen was passed to remove the hydrobromic acid gas. The mixture was diluted with chloroform, washed with water, aqueous sodium bicarbonate and water, dried and evaporated. The crude product (48.3 g) was passed through a silica gel column (1.5 kg) using ethyl acetate-hexane (1:4) and the eluates were evaporated to give 2-bromo-1-(3,4-dimethoxyphenyl)-1-ethanone (19.7 g), mp 87—90°C.

Similarly, by replacing 1-(3,4-dimethoxyphenyl)-1-ethanone with 1-[4-(methylsulfonylamino)-phenyl)-1-ethanone, 1-phenyl-1-ethanone, 1-(3,4-dimethoxyphenyl)-1-propanone, 1-(3,4,5-trimethoxyphenyl)-1-ethanone, 1-(4-methoxyphenyl)-1-ethanone, 2-butanone, 1-(4-fluorophenyl)-1-ethanone or 3,3-dimethyl-2-butanone, the following compounds are obtained respectively: 2-bromo-1-[4-(methylsulfonylamino)phenyl]-1-ethanone, mp 185—190°C (crystallized from methanol); 1-phenyl-2-bromo-1-ethanone, 2-bromo-1-(3,4-dimethoxyphenyl)-1-propanone, 2-bromo-1-(3,4,5-trimethoxyphenyl)-1-ethanone, 2-bromo-1-(4-methoxyphenyl)-1-ethanone, 2-bromo-2-butanone, 2-bromo-1-(4-fluorophenyl)-1-ethanone and 1-bromo-3,3-dimethyl-2-butanone.

To a suspension of 2-(1-piperazinyl)-2,4,6-cycloheptatrien-1-one methane sulfonate (19.64 g, described in Example 3) in acetonitrile, (150 ml) was added potassium carbonate (20.8 g). The resulting suspension was refluxed for about 20 min. A solution of 2-bromo-1-(3,4-dimethoxyphenyl)-1-ethanone (17.8 g) in acetonitrile (100 ml) was added dropwise. The mixture was stirred at 85°C for 1.25 hr. The mixture was filtered and the filtrate was evaporated. The filtered solid and oily residue was taken up in chloroform-water, and the chloroform layer was dried and evaporated to yield crude product (24.17 g). Chromatographic purification on silica gel (1000 g) using ethyl acetate gave 2-[4-[2-(3,4-dimethoxyphenyl)-2-oxoethyl]-1-piperazinyl]-2,4,6-cycloheptatrien-1-one (8.2 g), mp 132—135°C.

Similarly by replacing 2-bromo-1-(3,4-dimethoxyphenyl)-1-ethanone with 2-bromo-1-[4-(methylsulfonylamino)phenyl]-1-ethanone, 1-phenyl-2-bromo-1-ethanone, 2-bromo-1-(3,4-dimethoxyphenyl)-1-propanone, 2-bromo-1-(3,4,5-trimethoxyphenyl)-1-ethanone, 2-bromo-1-(4-methoxyphenyl)-1-ethanone, 2-bromo-2-butanone, 2-bromo-1-(4-fluorophenyl)-1-ethanone, 2-bromo-1-(3-methoxyphenyl)-1-ethanone, 2-bromo-1-(4-hydroxy-3-methoxyphenyl)-1-ethanone or 1-bromo-3,3-dimethyl-2-butanone, the following compounds of formula V are obtained respectively: 2-[4-[2-oxo-2-[4-(methylsulfonylamino)phenyl]ethyl]-1-piperazinyl]-2,4,6-cycloheptatrien-1-one: mp 187—190°C and nmr(DMSO-$d_6$) $\delta$ 2.6 (m, 4H), 3.10 (s 3H), 3.25 (m, 4H), 3.8 (s, 2H), 6.85 (m, 5H), 7.25 (d, 2H) and 7.95 (d, 2H); 2-[4-(2-phenyl-2-oxoethyl)-1-piperazinyl]-2,4,6-cycloheptatrien-1-one; mp 88—92°C (crystallized from ethyl acetate) and nmr ($CDCl_3$) $\delta$2.75 (m, 4H), 3.35 (m, 4H), 3.75 (s, 2H) and 7.3 (m, 9H); 2-[4-[2-(3,4-dimethoxyphenyl)-1-methyl-2-oxoethyl]-1-piperazinyl]-2,4,6-cycloheptatrien-1-one; ir($CHCl_3$) 1652, 1495, 1445 and 1000 $cm^{-1}$; and nmr($CDCl_3$)$\delta$ 1.3 (d, 3H), 2.75 (m, 5H), 3.35 (m, 4H), 3.9 (s, 6H) and 7.3 (m, 8H); 2-[4-[2-(3,4,5-trimethoxyphenyl)-2-oxoethyl]-1-piperazinyl]-2,4,6-cycloheptatrien-1-one; 2-[4-[2-(4-methoxyphenyl)-2-oxoethyl]-1-piperazinyl]-2,4,6-cycloheptatrien-1-one; 2-[4-(1-methyl-2-oxopropyl)-1-piperazinyl]-2,4,6-cycloheptatrien-1-one; 2-[4-[2-(4-fluorophenyl)-2-oxoethyl]-1-piperazinyl]-2,4,6-cycloheptatrien-1-one; 2-[4-[2-(3-methoxyphenyl)-2-oxoethyl]-1-piperazinyl]-2,4,6-cycloheptatrien-1-one; 2-[4-[2-(4-hydroxy-3-methoxyphenyl-2-oxoethyl]-1-piperazinyl]-2,4,6-cycloheptatrien-1-one; and 2-[4-(3,3-dimethyl-2-oxobutyl)-1-piperazinyl]-2,4,6-cycloheptatrien-1-one.

To a solution of 2-[4-[2-(3,4-dimethoxyphenyl)-2-oxoethyl]-1-piperazinyl]-2,4,6-cycloheptatrien-1-one (1.33 g, described above) in methanol (20 ml) and chloroform (5 ml), sodium borohydride (0.31 g) was added portionwise over a period of one hr. The solvent was then removed under vacuum. The residue was taken up in chloroform, washed with water, ammonium chloride solution followed by water, dried and evaporated to give the title compound (1.33 g): mp 122—124°C; ir$CHCl_3$) 3420 1565, 1260 and 1140 $cm^{-1}$; uv max(MeOH) 352 ($\varepsilon = 10040$), 256 ($\varepsilon = 15660$) and 227 nm($\varepsilon = 20250$); nmr($CDCl_3$)$\delta$ 2.6 (m, 6H), 3.3 (m, 4H), 3.85 (s, 6H), 4.7 (m, 1H) and 6.8 (m, 6H); and *Anal.* Calcd for $C_{21}H_{26}N_2O_4$: C, 68.08% H, 7.08% N, 7.56% and Found: C, 67.78% H, 7.06% N, 7.36%.

The title compound (3.4 g) was dissolved in methanol containing hydrogen chloride (6 ml of 2N methanolic HCl). The mixture was stirred for 5 min and diethyl ether was gradually added. The precipitated oil was allowed to crystallize and more diethyl ether was added to precipitate all the salt.

The mixture was stirred for 20 min and then filtered to yield crude product 3.26 g, mp 165—168°C. Crystallization from methanol diethyl ether gave the hydrochloride salt of the title compound: mp 175—178°C; ir(nujol) 3300, 2550, 1545 and 1265 $cm^{-1}$; uv max(MeOH) 344 ($\varepsilon = 9025$), 252 ($\varepsilon = 27600$) and 227 nm($\varepsilon = 36870$); nmr(DMSO-$d_6$)$\delta$ 3.5 (m, 10H), 3.73 (s, 3H), 3.77 (s, 3H), 5.15 (t, 1H), 6.1 (broad, 1H), 7.0 (m, 8H) and 10.75 (broad, 1H); and *Anal.* Calcd for $C_{21}H_{26}N_2O_4$ HCl: C, 61.84% H, 6.38% N, 6.87% and Found: C, 61.30% H, 6.65% N, 6.78%.

Similarly, by replacing 2-[4-[2-(3,4-dimethoxyphenyl)-2-oxoethyl]-1-piperazinyl]-2,4,6-cycloheptatrien-1-one with an equivalent amount of another compound of formula V, described above, the following compounds of formula I are obtained respectively, 2-[4-[2-hydroxy-2-[4-(methylsulfonylamino)phenyl]-ethyl]-1-piperazinyl]-2,4,6-cycloheptatrien-1-one (I: $R^1$ and $R^2 = H$ and $R^3 = $ 2-hydroxy-2-[4-(methylsulfonylamino)phenyl] ethyl): mp 204—206°C (crystallized from chloroform-methanol); ir(nujol) 3090, 1610 and 1580 $cm^{-1}$; uv max(MeOH) 351 ($\varepsilon = 10020$), 255 ($\varepsilon = 15570$) and 229 nm($\varepsilon = 25100$); nmr(DMSO-$d_6$)$\delta$ 2.6 (4H), 2.95 (s, 3H), 3.25 (m, 6H), 4.7 (m, 1H), 4.9 (s,1H), 7.0 (m, 9H) and 9.5 (s, 1H); and *Anal.* Calcd for $C_{20}H_{25}N_3O_4S$: C, 59.55% H, 6.20% N, 10.42% and Found: C, 59.41% H, 6.12% N, 10.49%; 2-[4-(2-hydroxy-2-phenylethyl)-1-piperazinyl]-2,4,6-cycloheptatrien-1-one (I: $R^1$ and $R^2 = H$ and $R^3 = $ 2-hydroxy-2-phenylethyl): mp 129—131°C (crystallized from methanol-diethyl); ir($CHCl_3$) 3590, 3420 and 1565 $cm^{-1}$; uv max(MeOH) 352 ($\varepsilon = 9550$) and 256 nm($\varepsilon = 15595$); nmr($CDCl_3$)$\delta$ 2.6 (m, 6H), 3.35 (m, 4H), 3.8 (1H, broad), 4.75 (m, 1H) and 7.0 (m, 10H); and *Anal.* Calcd for $C_{19}H_{22}N_2O_2$: C, 73.52% H, 7.14% N, 9.03% and Found: C, 73.36% H, 7.01% N, 8.96%; isomer A of 2-[4-[2-hydroxy-1-methyl-2-(3,4-dimethoxyphenyl)ethyl]-1-piperazinyl]-2,4,6-cycloheptatrien-1-one (I: $R^1$ and $R^2 = H$ and $R^3 = $ 2-hydroxy-1-methyl-2-(3,4-dimethoxyphenyl)ethyl):: mp 133—135°C (crystallized from methanol-diethyl ether); ir(nujol) 3340, 1560, 1260 and 1150 $cm^{-1}$; uv max(MeOH) 352 ($\varepsilon = 9180$), 256 ($\varepsilon = 14480$) and 229 nm($\varepsilon = 20700$); and nmr(DMSO-$d_6$)$\delta$ 0.8(d, 3H), 2.75 (m, 5H), 3.4 (t, 4H), 3.83 (s, 3H), 3.9 (s, 3H), 4.23 (d, 1H), 5.0 (1H, broad) and 6.85 (m, 8H); maleate of isomer B of 2-[4-[2-hydroxy-1-methyl-2-(3,4-dimethoxyphenyl)-ethyl]-1-piperazinyl]-2,4,6-cycloheptatrien-1-one (I: $R^1$ and $R^2 = H$ and $R^3 = $ 2-hydroxy-1-methyl-2-(3,4-dimethoxyphenyl)ethyl): mp 165—168°C (crystallized from methanol-diethyl ether); ir(nujol) 3420, 2700, 1578, 1550 and 1265 $cm^{-1}$; uv max(MeOH) 343 ($\varepsilon = 9800$) and 251 nm($\varepsilon = 15550$); nmr(DMSO-$d_6$) $\delta$1.05 (d 3H), 3.5 (m, 9H), 3.75 (s, 6H), 5.2 (s, 1H), 6.0 (s, 2H) and 6.9 m, 8H); and *Anal.* Calcd for $C_{22}H_{28}N_2O_4.C_4H_4O_4$: C, 62.40% H, 6.40% N, 5.60% and Found: C, 62.49% H, 6.46% N, 5.45%; 2-[4-[2-hydroxy-2-(3,4,5-trimethoxyphenyl)ethyl]-1-piperazinyl]-2,4,6-cycloheptatrien-1-one (I: $R^1$ and $R^2 = H$ and $R^3 = $ 2-hydroxy-2-(3,4,5-trimethoxyphenyl)ethyl: mp 144.5—145.5°C (crystallized from methanol-ethyl acetate); ir($CHCl_3$) 3600, 3420, 1565 and 1135 $cm^{-1}$; uv max(MeOH) 352($\varepsilon = 10150$) and 256 nm($\varepsilon = 15865$); nmr($CDCl_3$) $\delta$ 3.8 and 3.85 (9H, singlets), 4.7 (s, 1H) and 6.8 (m, 7H); and *Anal.* Calcd for $C_{22}H_{28}N_2O_5$: C, 65.97% H, 7.05% N, 7.00% and Found: C, 65.77% H, 7.07% N, 6.87%; 2-[4-[2-hydroxy-2-(4-methoxyphenyl)ethyl]-1-piperazinyl]-2,4,6-cycloheptatrien-1-one (I: $R^1$ and $R^2 = H$ and $R^3 = $ 2-hydroxy-2-(4-methoxyphenyl)ethyl: mp 139—140°C (crystallized from ethyl acetate-diethyl ether); ir($CHCl_3$) 3440, 1665, 1250 and 1175 $cm^{-1}$; uv max(MeOH) 351 ($\varepsilon = 10160$), 255 ($\varepsilon = 15635$) and 225 nm($\varepsilon = 23530$); nmr($CDCl_3$)$\delta$ 2.75 (m, 6H), 3.35 (t, 4H), 3.8 (s, 3H), 4.0 (1H, broad), 4.7 (t, 1H) and 7.0 (m, 9H); and *Anal.* Calcd for $C_{20}H_{23}N_2O_3$: C, 70.77% H, 6.83% N, 8.26% and Found: C, 70.46% H, 7.20% N, 8.12%; 2-[4-(2-hydroxy-1-methylpropyl)-1-piperazinyl]-2,4,6-cycloheptatrien-1-one (I: $R^1$ and $R^2 = H$ and $R^3 = $ 2-hydroxy-1-methylpropyl): mp 126°C (crystallized from hexane-ethyl acetate); ir($CHCl_3$) 3380 and 1565 $cm^{-1}$; uv max(MeOH) 351 ($\varepsilon = 9755$), 255 ($\varepsilon = 14855$) and 221 nm($\varepsilon = 12135$); nmr($CDCl_3$) $\delta$ 0.95 (d, 3H), 1.15 (d, 3H), 4.25 (1H, broad) and 6.9 (m, 5H); and *Anal.* Calcd for $C_{15}H_{22}N_2O_2$: C, 68.66% H, 8.45% N, 10.68% and Found: C, 68.85% H, 8.64% N, 10.55%; 2-[4-[2-hydroxy-2-(4-fluorophenyl)ethyl]-1-piperazinyl]-2,4,6-cycloheptatrien-1-one (I: $R^1$ and $R^2 = H$ and $R^3 = $ 2-hydroxy-2-(4-fluorophenyl)ethyl): mp 130—132°C (crystallized from chloroform-hexane); ir ($CHCl_3$) 3600, 3440 and 1565 $cm^{-1}$; uv max(MeOH) 352 ($\varepsilon = 9900$) and 255 nm($\varepsilon = 16500$) and nmr($CDCl_3$) $\delta$ 2.65 (m, 7H), 3.35 (t, 4H), 4.7 (m, 1H) and 7.0 (m, 9H); 2-[4-[2-hydroxy-2-(3-methoxyphenyl)ethyl]-1-piperazinyl]-2,4,6-cycloheptatrien-1-one (I: $R^1$ and $R^2 = H$ and $R^3 = $ 2-hydroxy-2-(3-methoxyphenyl)ethyl): mp 105—107°C (crystallized from ethyl acetate-diethyl ether); ir ($CHCl_3$) 3600, 3420, 1560, 1260 and 1140 $cm^{-1}$; uv max(MeOH) 351 ($\varepsilon = 9970$) and 256 nm($\varepsilon = 15450$): nmr($CDCl_3$) $\delta$ 2.6 (m, 6H), 3.35 (t, 4H), 3.75 (s, 3H), 4.7 (m, 1H) and 7.0 (m, 9H); and *Anal.* Calcd for $C_{20}H_{24}N_2O_3$: C, 70.57% H, 7.11% N, 8.23% and Found: C, 70.11% H, 7.21% N, 8.10%; 2-[4-[2-hydroxy-2-(4-hydroxy-3-methoxyphenyl)ethyl]-1-piperazinyl]2,4,6-cycloheptatrien-1-one (I: $R^1$ and $R^2 = H$ and $R^3 = $ 2-hydroxy-2-(4-hydroxy-3-methoxyphenyl)ethyl): mp 167—167.5°C (crystallized from methanol); ir (nujol) 3380, 2900 and 1515 $cm^{-1}$; uv max(MeOH) 352 ($\varepsilon = 10100$), 255 ($\varepsilon = 15600$) and 226 nm($\varepsilon = 18600$); nmr(DMSO-$d_6$) $\delta$ 2.6 (m, 6H), 3.3 (m, 4H), 3.75 (s, 3H), 4.65 (m, 1H), 4.8 (s, 1H), 6.9 (m, 8H) and 8.65 (s, 1H); and *Anal.* Calcd for $C_{20}H_{24}N_2O_4$: C, 67.40% H, 6.79% N, 7.86% and Found: C, 67.39% H, 6.94% N, 7.8%; and 2-[4-(2-hydroxy-3,3-dimethyl-1-butyl)-1-piperazinyl]-2,4,6-cycloheptatrien-1-one (I: $R^1$ and $R^2 = H$ and $R^3 = $ 2-hydroxy-3,3-dimethyl-1-butyl): mp 104—105°C (crystallized from diethyl ether-hexane); ir ($CHCl_3$) 3400 and 1560 $cm^{-1}$; uv max (MeOH) 351 ($\varepsilon = 9555$), 255 ($\varepsilon = 14460$) and 221 ($\varepsilon = 11415$); and *Anal.* Calcd for $C_{17}H_{26}N_2O_2$: C, 70.31% H, 9.02% N, 9.65% and Found: C, 70.02% H, 9.20% N, 9.59%.

Example 20

2-[4-(2-Hydroxyethyl)-3-methyl-1-piperazinyl]-2,4,6-cycloheptatrien-1-one (I: $R^1 = H$, $R^2 = 3—CH_3$ and $R^3 = $ 2-hydroxyethyl)

A solution of 2-methoxy-2,4,6-cycloheptatrien-1-one (6.8 g) and 2-methylpiperazine (5 g) in methanol was refluxed for 16 hr and evaporated. The residue (11 g) was chromatographed on silica gel (250 g) using methanol and the eluate was evaporated to give 7.2 g of 2-(3-methyl-1-piperazinyl)-2,4,6-cycloheptatrien-1-one.

A solution of the latter compound (7.2 g) in methanol (20 ml) was cooled in an ice bath, then ethylene oxide gas (3 g) was introduced. The reaction mixture was kept at 0°C for 16 hr and evaporated. The residue (5 g) was chromatographed twice on 150 g silica gel with methanol-ethyl acetate (2:8) to yield 3.1 g of the title compound. It was converted to the methane sulfonic acid salt which was crystallized from methanol-acetone to give 1.8 g of the methane sulfonate salt of the title compound: mp 136—138°C; ir(mull) 3320, 2626 and 1546 $cm^{-1}$; uv max(MeOH) 343 ($\varepsilon = 9460$), 251 ($\varepsilon = 14090$) and 223 nm($\varepsilon = 11340$); nmr ($CDCl_3$) $\delta$ 1.45 (d, 3H), 2.70 (s, 3H), 3.50 (m, 11H), 5.00 (broad, 2H) and 6.9 (m, 5H); and *Anal.* Calcd for $C_{14}H_{20}N_2O_2$. $CH_3SO_3H$: C, 52.30% H, 7.24% N, 8.13% and Found: C, 52.46% H, 7.09% N, 7.91%.

Example 21

2-[4-(2-Hydroxypropyl)-1-piperazinyl]-2,4,6-cycloheptatrien-1-one (I: $R^1$ and $R^2 = H$ and $R^3 = $ 2-hydroxypropyl)

A mixture of 2-(1-piperazinyl)-2,4,6-cycloheptatrien-1-one (12.4 g), described in Example 3), 1-chloro-2-propanone (5.55 g), potassium carbonate (11.04 g) an acetonitrile (120 ml) was refluxed for 4 hr and filtered. Chloroform and water were added to the filtrate. The organic phase was separated, washed with water, dried and evaporated. The residue was chromatographed on silica gel using methanol-ethyl acetate and the eluates were evaporated to give an oil (6.21 g) of 2-[4-(2-oxopropyl)-1-piperazinyl]-2,4,6-cycloheptatrien-1-one (V; $R^1$, $R^2$, $R^3$, $R^4$ and $R^5 = H$ and $Alk^1 = C—CH_2$): ir($CHCl_3$) 3660, 3360, 1720, 1710, 1660 and 1560 $cm^{-1}$; uv max(MeOH) 351 ($\varepsilon = 9310$), 255 ($\varepsilon = 13915$) and 221 nm($\varepsilon = 11750$); nmr($CDCl_3$) $\delta$ 2.12 (s, 3H), 2.62 (t, 4H), 3.21 (s, 2H), 3.35 (s, 4H) and 6.40—7.10 (m, 5H); and *Anal.* Calcd for $C_{14}H_{18}N_2O_2$: C, 68.26% H, 7.37% N, 11.37% and Found: C, 67.83% H, 7.59% N,11.44%.

A solution of the latter compound (12.0 g) in methanol (100 ml) was cooled in an ice-bath, then sodium borohydride was added in portions and the progress of the reduction was monitored by tlc. When the reaction was complete the solvent was evaporated, and the residue was dissolved in water. It was extracted with chloroform, dried over sodium sulfate and evaporated to yield 9.1 g of crude product. Chromatographic purification on 350 g silica gel with methanol-ethyl acetate (1:9) afforded 7.0 g of a residue which after two crystallizations from ethyl acetate yielded 4.0 g of the title compound: mp 92—93°C; ir($CHCl_3$) 3450 and 1565 $cm^{-1}$; uv max(MeOH) 353 ($\varepsilon = 9870$), 225 ($\varepsilon = 7263$) and 221 nm($\varepsilon = 5785$); nmr($CDCl_3$) $\delta$ 1.13 (d, 3H), 2.6 (m, 6H), 3.35 (t, 4H), 3.75 (m, 1H) and 6.8 (m, 5H); and *Anal.* Calcd for $C_{14}H_{20}N_2O_2$: C, 67.71% H, 8.12% N, 11.28% and Found: c, 67.61% H, 8.18% N, 11.60%.

Example 22

2-[4-[2-Hydroxy-2-(3,4-dimethoxyphenyl)ethyl]-1-piperazinyl]-2,4,6-cycloheptatrien-1-one (I: $R^1$ and $R^2 = H$ and $R^3 = $ 2-hydroxy-2-(3,4-dimethoxyphenyl)ethyl)

Sodium carbonate (18.84 g) and water (41.4 ml) was added to a stirring suspension at 0 to 5°C of 2-bromo-1-(3,4-dimethoxyphenyl)-1-ethanone (32.03 g, described in Example 19) and 1-benzylpiperazine (24 ml) in acetonitrile (90 ml). The suspension was stirred at room temperature for 2.5 hr and evaporated. The residue was dissolved in a mixture of water and chloroform, and the chloroform layer was dried and evaporated. Chromatographic purification on silica gel (700 g) using ethyl acetate-hexane (60:1 to 100:1) gave 32 g of 2-(4-benzyl-1-piperazinyl)-1-(3,4-dimethoxyphenyl)-1-ethanone, nmr($CDCl_3$) $\delta$ 2.65, 3.57, 3.74, 3.89, 3.91, 6.81, 7.37 and 7.53.

A suspension of the latter compound (32 g), 10% palladium on carbon (8.22 g), 2N hydrogen chloride in methanol (40.58 ml) and methanol (345 ml) was stirred rapidly under an atmosphere of hydrogen for 24 hr and filtered. The filtrate was washed with chloroform and basified with 20% sodium hydroxide (20 ml). The resulting solution was extracted with chloroform and the extract was dried and evaporated to give 22.41 g of 2-(1-piperazinyl)-1-(3,4-dimethoxyphenyl)-1-ethanone, nmr($CDCl_3$) $\delta$ 2.12, 2.55, 2.90, 3.70, 3.92, 6.82 and 7.60.

To a solution at room temperature of the latter compound (22.4 g) in methanol (120 g), sodium borohydride (4.46 g) was slowly added. The solution was evaporated and the residue was dissolved in a mixture of water and chloroform. The chloroform phase was separated, dried and evaporated to give 16.65 g of 2-(1-piperazinyl)-1-(3,4-dimethoxyphenyl)-1-ethanol, nmr($CDCl_3$) $\delta$ 2.4 (m, 4H), 2.6 (m, 2H), 2.9 (t, 4H), 3.82 and 3.85 (singlets, 6H), 4.65 (m, 1H) and 6.85 (m, 3H).

A solution of the latter compound (0.728 g), 2-methoxy-2,4,6-cycloheptatrien-1-one (0.048 g), methanol (5 ml) and sodium (0.070 g) in methanol (2.9 ml) was stirred at 70°C for one hr, at room temperature for 20 hr and at 80°C for one hr and evaporated. The residue was dissolved in a solution of

water and chloroform. The chloroform phase was separated, dried, evaporated (1.25 g of residue) and crystallized from chloroform-hexane to give the title compound, mp 120—123°C and nmr($CDCl_3$) $\delta$ 2.7 (m, 6H), 3.35 (t, 4H), 3.85 and 3.9 (singlets, 6H), 4.7 (t, 1H) and 6.8 (m, 8H). The title compound of this example is identical to the title compound of Example 19.

Example 23

Resolution of 2-[4-[2-Hydroxy-2-(3,4-dimethoxyphenyl)ethyl]-1-piperazinyl]-2,4,6-cycloheptatrien-1-one

A solution of 2-[4-[2-hydroxy-2-(3,4-dimethoxyphenyl)ethyl]-1-piperazinyl]-2,4,6-cycloheptatrien-1-one (3.7 g, described in Example 19) and L-ditoluyltartaric acid (4.24 g) in methanol was stirred at room temperature overnight and filtered. Diethyl ether (15 ml) was added to the filtrate and the solution was filtered. The above two collected precipitates were combined (7.3 g, mp 178—180°C), methanol (150 ml) was added and the mixture was refluxed for 5 to 10 min, cooled to room temperature and filtered to give solid A (3.5 g, mp 195—197°C) and filtrate B. Solid A was washed with boiling methanol (30 ml) to give a solid (3.45 g) of the salt of (+)-2-[4-[2-hydroxy-2-(3,4-dimethoxyphenyl)ethyl]-1-piperazinyl]-2,4,6-cycloheptatrien-1-one with L-ditoluyltartic acid; mp 197—199°C and $[\alpha]_D^{25}$ + 45.8° (C = 1, dimethylformamide). A suspension of the latter salt in water was basified with aqueous 10% sodium hydroxide and extracted with ethyl acetate. The extract was evaporated and crystallized from hexane-chloroform to obtain the optical (+)-isomer (1.1 g) of the title compound; mp 124—125°C, and $[\alpha]_D^{25}$ + 11.7° (C = 1, dimethylformamide).

The above filtrate B was evaporated. A suspension of the residue in water was basified with aqueous 10% sodium hydroxide and extracted with ethyl acetate. the extract was evaporated to give a residue (1.6 g) which was treated with a solution of D-ditoluyltartaric acid (1.67 g) in methanol (7 ml) at room temperature overnight. The precipitate (2.92 g) was collected to give the salt of (—)-2-[4-[2-hydroxy-2-(3,4-dimethoxyphenyl)ethyl]-1-piperazinyl]-2,4,6-cycloheptatrien-1-one with D-ditoluyltartaric acid: mp 193—195°C, and $[\alpha]_D^{25}$ —45.0° (c = 1, dimethylformamide). A suspension of the latter salt in water was basified with aqueous 10% sodium hydroxide and extracted with ethyl acetate. The extract was evaporated and crystallized from hexane-chloroform to obtain the optical (—)-isomer of the title compound; mp 122—124°C, and $[\alpha]_D^{25}$ —12.0° (c = 1, dimethylformamide).

Similarly, by replacing 2-[4-[2-hydroxy-2-(3,4-dimethoxyphenyl)ethyl]-1-piperazinyl]-2,4,6-cycloheptatrien-1-one with isomer B of 2-[4-[2-hydroxy-1-methyl-2-(3,4-dimethoxyphenyl)-ethyl]-1-piperazinyl]-2,4,6-cycloheptatrien-1-one (described in Example 19), the following optical isomers of this compound were obtained: optical (+) isomer, mp 96—98°C (crystallized from ethyl acetatediethyl ether) and $[\alpha]_D^{25}$ +84.2° (c = 1, dimethylformamide); maleate salt of optical (+) isomer, mp 161—163°C (crystallized from methanol-diethyl ether) and $[\alpha]_D^{25}$ +37.4° (c = 1, dimethylformamide); optical (—) isomer, mp 95—98°C (crystallized from ethyl acetate-diethyl ether) and $[\alpha]_D^{25}$ —82.5° (c = 1, dimethylformamide); and maleate salt of optical (—) isomer, mp 160—162°C (crystallized from methanol-diethyl ether) and $[\alpha]_D^{25}$ —36.5° (c = 1, dimethylformamide).

**Claims for the Contracting States: BE CH DE FR GB IT LI LU NL SE**

1. A compound of formula I

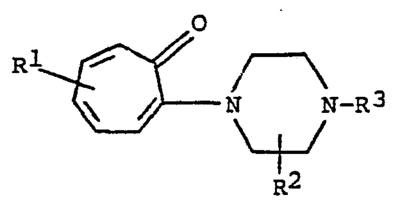

(I)

in which $R^1$ represents a substituent at positions 3, 4, 5, 6 or 7 of the 2,4,6-cycloheptatrien-1-one ring and is selected from hydrogen, halo, lower alkyl, lower alkoxy, trifluoromethyl or 1-oxo(lower)-alkylamino; $R^2$ is hydrogen or lower alkyl having one to three carbon atoms; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alkyl; lower alkenyl substituted with phenyl; phenyl mono-, di- or trisubstituted with lower alkyl, halo, lower alkoxy, hydroxy or trifluoromethyl; 2-(4,5-dihydro-2-oxazolyl)benzoyl, 2,3-dihydro-8-oxo-cyclohepta[b]furan-2-ylmethyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to six carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di or trisubstituted with lower

alkyl, halo, lower alkoxy, hydroxy or trifluoromethyl, or phenoxy mono-, di- or trisubstituted with lower alkyl, halo, lower alkoxy, acetylamino, hydroxy or trifluoromethyl, or a group of formula

$$\underset{\displaystyle OH}{\underset{|}{CR^5R^6—Alk^1—R^7}}$$

wherein $Alk^1$ is a trivalent alkylene having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkyl, halo, lower alkoxy, hydroxy or trifluoromethyl, or phenoxy mono-, di- or trisubstituted with lower alkyl, halo, lower alkoxy, hydroxy or trifluoromethyl; with the proviso that when $R^7$ is hydroxy, the hydroxy groups are joined to different carbon atoms; or a therapeutically acceptable acid addition salt thereof; [and wherein the term "lower alkyl", unless stated otherwise, means a straight chain alkyl radical containing from one to six carbon atoms or a branched chain alkyl radical containing three or four carbon atoms; the term "lower alkoxy" means a straight chain alkoxy radical containing from one to six carbon atoms or a branched chain alkoxy radical containing three or four carbon atoms; the term "1-oxo(lower)alkyl" means a straight chain 1-oxoalkyl radical containing from two to six carbon atoms or a branched chain 1-oxoalkyl radical containing four to six carbon atoms; the term "lower alkenyl" means a straight chain alkenyl radical containing two to six carbon atoms or a branched chain alkenyl radical containing three or four carbon atoms; the term "lower alkynyl" means a straight chain alkynyl radical containing from two to 6 carbon atoms or a branched chain alkynyl radical containing four carbon atoms; the term "1-oxo(lower)alkoxy" means a straight chain 1-oxoalkoxy radical containing from two to six carbon atoms or a branched chain 1-oxoalkoxy radical containing from four to six carbon atoms; and the term "cyclo(lower)alkyl" means a saturated cyclic hydrocarbon radical containing from three to six carbon atoms].

2. A compound of Claim 1 in which $R^1$ represents a substituent at position 7 of the 2,4,6-cycloheptatrien-1-one ring and is selected from hydrogen, halo, lower alkyl, lower alkoxy, trifluoromethyl, or 1-oxo(lower)alkylamino; $R^2$ is hydrogen or lower alkyl having one to three carbon atoms; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alkyl; lower alkenyl substituted with a phenyl; phenyl mono-, di- or trisubstituted with lower alkyl, halo, lower alkoxy, hydroxy or trifluoromethyl; 2-(4,5-dihydro-2-oxazolyl)benzoyl, 2,3-dihydro-8-oxocyclohepta[b]furan-2-ylmethyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to six carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di- or trisubstituted with lower alkoxy or hydroxy, or phenoxy mono-, di- or trisubstituted with lower alkyl, halo or acetylamino, or a group of formula

$$\underset{\displaystyle OH}{\underset{|}{CR^5R^6—Alk^1—R^7}}$$

wherein $Alk^1$ is a trivalent alkylene having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkoxy or hydroxy, or phenoxy mono-, di- or trisubstituted with lower alkyl or halo; with the proviso that when $R^7$ is hydroxy, the hydroxy groups are joined to different carbon atoms; or a therapeutically acceptable acid addition salt thereof.

3. A compound of Claim 1 in which $R^1$ represents a substituent at position 7 of the 2,4,6-cycloheptatrien-1-one ring and is selected from hydrogen or bromo; $R^2$ is hydrogen or methyl; and $R^3$ is hydrogen, phenyl, lower alkyl, lower alkenyl, lower alkynyl, cyclo(lower)alkyl, lowr alkoxycarbonyl, 1-oxo((lower)alkyl, cyclo(lower)alkyl substituted with a lower alkyl, phenyl monosubstituted with lower alkyl, halo or trifluoromethyl, 2-(4,5-dihydro-2-oxazolyl)benzoyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to three carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono- or disubstituted with lower alkoxy or hydroxy, or phenoxy monosubstituted with lower alkyl, halo or acetylamino; or a group of formula

$$\underset{\displaystyle OH}{\underset{|}{CR^5R^6—Alk^1—R^7}}$$

wherein $Alk^1$ is a trivalent alkylene having one to three carbon atoms; $R^5$ and $R^6$ each is hydrogen or lower alkyl having one to three carbon atoms; $R^7$ is hydrogen, phenyl, hydroxy, phenoxy, phenyl mono-, di- or trisubstituted with lower alkoxy or hydroxy or phenoxy monosubstituted with lower alkyl or halo, with the proviso that when $R^7$ is hydroxy, the hydroxy groups are joined to different carbon atoms; or a therapeutically acceptable acid addition salt thereof.

4. A compound of claim 1 in which $R^1$ and $R^2$ are hydrogen; and $R^3$ is hydrogen, lower alkyl, lower alkenyl, lower alkoxycarbonyl, a group of formula Alk-$R^4$ wherein Alk is a divalent alkyl having one or two carbon atoms and $R^4$ is lower alkoxy, cyano, 1-oxo(lower)alkoxy, or phenyl mono- or disubstituted with lower alkoxy or hydroxy, or a group of formula

$$\underset{\displaystyle OH}{CR^5R^6—\underset{|}{Alk^1}—R^7}$$

wherein $Alk^1$ is CH; $R^5$ is hydrogen, $R^6$ is hydrogen or lower alkyl having one to three carbon atoms; and $R^7$ is hydrogen, phenyl or phenyl mono-, di- or tri-substituted at positions 3, 4 or 5 with lower alkoxy or hydroxy; or a therapeutically acceptable acid addition salt thereof.

5. A compound as claimed in claim 1, which is selected from the group of (—)-2-[4-[2-hydroxy-2-(3,4-dimethoxyphenyl)ethyl]-1-piperazinyl]-2,4,6-cycloheptatrien-1-one [having $[\alpha]_D^{25}$—12.0° (c=1, dimethylformamide)], (—)-2-[4-[2-hydroxy-1-methyl-2-(3,4-dimethoxyphenyl)ethyl]-1-piperazinyl]-2,4,6-cycloheptatrien-1-one, [having $[\alpha]_D^{25}$—82.5° (c=1, dimethylformamide)], 2-[4-[2-hydroxy-2-(3,4-dimethoxyphenyl)ethyl-1-piperazinyl]-2,4,6-cycloheptatrien-1-one, 2-[4-[2-hydroxy-1-methyl-2-(3,4-dimethoxyphenyl)ethyl-1-piperazinyl]-2,4,6-cycloheptatrien-1-one, 2-[4-[2-hydroxy-1-methyl-2-(3,4-dimethoxyphenyl)ethyl]-1-piperazinyl]-2,4,6-cycloheptatrien-1-one, [the maleate salt thereof having m.p. 165—168°C], 4-(2-oxo-3,5,7-cycloheptatrien-1-yl)-1-piperazine-ethanol, (+)-2-[4-[2-hydroxy-2-(3,4-dimethoxyphenyl)ethyl-1-piperazinyl]-2,4,6-cycloheptatrien-1-one [having $[\alpha]_D^{25}$+ 11.7° (c=1, dimethylformamide)], 2-[4-(2-hydroxypropyl)-1-piperazinyl]-2,4,6-cycloheptatrien-1-one, (+)-2-[4-[2-hydroxy-1-methyl-2-(3,4-dimethoxyphenyl)ethyl]-1-piperazinyl]-2,4,6-cycloheptatrien-1-one, [having $[\alpha]_D^{25}$ +84.2° (c=1, dimethylformamide)] and 2-[4-[2-hydroxy-2-(4-methoxyphenyl)ethyl]-1-piperazinyl-2,4,6-cycloheptatrien-1-one, or a therapeutically acceptable acid addition salt thereof.

6. A compound as claimed in Claim 1 which is 7-bromo-2-[4-(2-hydroxyethyl)-1-piperazinyl]-2,4,6-cycloheptatrien-1-one, or a therapeutically acceptable acid addition salt thereof.

7. A compound as claimed in Claim 1, which is selected from the group of 4-(2-oxo-3,5,7-cycloheptatrien-1-yl)-1-piperazine-acetonitrile, 2-[4-[2-(3,4-dimethoxyphenyl)ethyl]-1-piperazinyl]-2,4,6-cycloheptatrien-1-one, 2-[4-[2-(acetyloxy)ethyl-1-piperazinyl]-2,4,6-cycloheptatrien-1-one, 2,2-dimethylpropanoic acid, 2-[4-(2-oxo-3,5,7-cycloheptatrien-1-yl)-1-piperazinyl]ethyl ester, or a therapeutically acceptable acid addition salt thereof.

8. A compound as claimed in Claim 1, which is selected from the group of 2-(4-methyl-1-piperazinyl)-2,4,6-cycloheptatrien-1-one, 2-[4-(2-propenyl)-1-piperazinyl]-2,4,6-cycloheptatrien-1-one, 2-[4-(1-methylethyl)-1-piperazinyl]-2,4,6-cycloheptatrien-1-one, 2-(4-propyl-1-piperazinyl)-2,4,6-cycloheptatrien-1-one and 2-[4-(1-methylpropyl)-1-piperazinyl]-2,4,6-cycloheptatrien-1-one or a therapeutically acceptable acid addition salt thereof.

9. A compound of formula

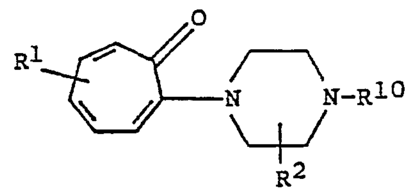

in which $R^1$ represents a substituent at positions 3, 4, 5, 6 or 7 of the 2,4,6-cycloheptatrien-1-one ring and is selected from hydrogen, halo, lower alkyl, lower alkoxy, trifluoromethyl or 1-oxo(lower)alkylamino; $R^2$ is hydrogen or lower alkyl having one to three carbon atoms; $R^{10}$ is 2-amino-ethyl, -Alk-cl (where Alk is a divalent alkyl radical having one to six carbon atoms) or a group of the formula

$$—CR^5R^6—\underset{\displaystyle O}{\underset{\|}{Alk^1}}—R^7 \quad \text{where} \quad \underset{\displaystyle O}{\underset{\|}{Alk^1}}$$

is a divalent alkanone having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkyl, halo, lower alkoxy, hydroxy or trifluoromethyl, or phenoxy mono-, di- or trisubstituted with lower alkyl, halo, lower alkoxy, hydroxy or trifluoromethyl;

with the proviso that when $R^7$ is hydroxy, the hydroxy and ketone groups are joined to different carbon atoms; [and wherein the term "lower alkyl", unless stated otherwise, means a straight chain alkyl radical containing from one to six carbon atoms or a branched chain alkyl radical containing three or four carbon atoms; the term "lower alkoxy" means a straight chain alkoxy radical containing from one to six carbon atoms or a branched chain alkoxy radical containing three or four carbon atoms; and the term "1-oxo(lower)alkyl" means a straight chain 1-oxoalkyl radical containing from two to six carbon atoms or a branched chain 1-oxoalkyl radical containing four to six carbon atoms].

10. A compound of Claim 9 wherein $R^1$ and $R^2$ are hydrogen and $R^{10}$ is

$$—CR^5R^6—\underset{\underset{\displaystyle O}{\|}}{Alk^1}—R^7$$

where $R^5$ and $R^6$ each is hydrogen or lower alkyl having one to three carbon atoms; $R^7$ is hydrogen, phenyl or phenyl mono-, di- or trisubstituted with halo, lower alkoxy or hydroxy and

$$\underset{\underset{\displaystyle O}{\|}}{Alk^1}$$

is a divalent alkanone having one to three carbon atoms.

11. A pharmaceutical composition comprising a compound of formula I or a therapeutically acceptable acid addition salt thereof, as claimed in any one of Claims 1 to 8, and a pharmaceutically acceptable carrier.

12. A compound of formula I, or a therapeutically acceptable acid addition salt thereof, as claimed in any one of Claims 1 to 8 for use as a dopamine receptor stimulating agent.

## Claims for the Contracting State: AT

1. A process for preparing a compound of formula I

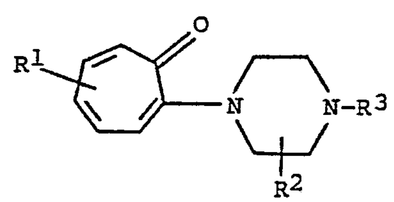

(I)

in which $R^1$ represents a substituent at positions 3, 4, 5, 6 or 7 of the 2,4,6-cycloheptatrien-1-one ring and is selected from hydrogen, halo, lower alkyl, lower alkoxy, trifluoromethyl or 1-oxo(lower)-alkylamino; $R^2$ is hydrogen or lower alkyl having one to three carbon atoms; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alkyl; lower alkenyl substituted with phenyl; phenyl mono-, di or trisubstituted with lower alkyl, halo, lower alkoxy, hydroxy or trifluoromethyl; 2-(4,5-dihydro-2-oxazolyl)benzoyl, 2,3-dihydro-8-oxo-cyclohepta[b]furan-2-ylmethyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to six carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di or trisubstituted with lower alkyl, halo, lower alkoxy, hydroxy or trifluoromethyl, or phenoxy mono-, di- or trisubstituted with lower alkyl, halo, lower alkoxy, acetylamino, hydroxy or trifluoromethyl, or a group of formula

$$CR^5R^6—\underset{\underset{\displaystyle OH}{|}}{Alk^1}—R^7$$

wherein $Alk^1$ is a trivalent alkylene having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkyl, halo, lower alkoxy, hydroxy or trifluoromethyl, or phenoxy mono-, di-or trisubstituted with lower alkyl, halo, lower alkoxy, hydroxy or trifluoromethyl; with the proviso that when $R^7$ is hydroxy, the hydroxy groups are joined to different carbon

atoms; or a therapeutically acceptable acid addition salt thereof; [and wherein the term "lower alkyl", unless stated otherwise, means a straight chain alkyl radical containing from one to six carbon atoms or a branched chain alkyl radical containing three or four carbon atoms; the term "lower alkoxy" means a straight chain alkoxy radical containing from one to six carbon atoms or a branched chain alkoxy radical containing three or four carbon atoms; the term "1-oxo(lower)alkyl" means a straight chain 1-oxoalkyl radical containing from two to six carbon atoms or a branched chain 1-oxoalkyl radical containing four to six carbon atoms; the term "lower alkenyl" means a straight chain alkenyl radical containing two to six carbon atoms or a branched chain alkenyl radical containing three or four carbon atoms; the term "lower alkynyl" means a straight chain alkynyl radical containing from two to 6 carbon atoms or a branched chain alkynyl radical containing four carbon atoms; the term "1-oxo(lower)alkoxy" means a straight chain 1-oxoalkoxy radical containing from two to six carbon atoms or a branched chain 1-oxoalkoxy radical containing from four to six carbon atoms; and the term "cyclo(lower)alkyl" means a saturated cyclic hydrocarbon radical containing from three to six carbon atoms] which comprises selecting a process from the group of:

a) when a compound of formula I in which $R^1$, $R^2$ and $R^3$ are as defined herein is required, condensing a compound of formula II

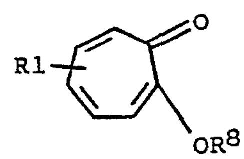

(II)

in which $R^1$ is as defined herein and $R^8$ is lower alkyl with a compound of formula III

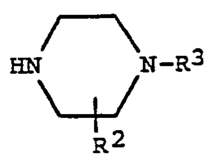

(III)

in which $R^2$ is as defined herein and $R^3$ is as defined above or is amino(lower)alkyl;

b) when a compound of formula I in which $R^1$ and $R^2$ are as defined herein and $R^3$ is lower alkyl, lower alkenyl, lower alkynyl, cyclo(lower)alkyl, lower alkoxycarbonyl, 1-oxo(lower)alkyl, cyclo(lower)-alkyl substituted with a lower alkyl, lower alkenyl substituted with phenyl, 2-(4,5-dihydro-2-oxazolyl)-benzoyl, 2,3-dihydro-8-oxo-cyclohepta[b]furan-2-ylmethyl, 2,3-diacetyloxypropyl, 2-(1,3-dihydro-1,3-dioxo-2H-isoindol-2-yl)ethyl, a group of formula Alk-$R^4$ wherein Alk and $R^4$ are as defined herein, or a group of formula

$$CR^5R^6\text{—}Alk^1\text{—}R^7 \quad (\text{with OH on } Alk^1)$$

wherein $R^5$, $R^6$, $R^7$ and $Alk^1$ are as defined herein is required, reacting the compound of formula I in which $R^1$ and $R^2$ are as defined herein and $R^3$ is hydrogen with a halide of formula X—$R^3$ wherein X is bromo, chloro or iodo and $R^3$ is lower alkyl, lower alkenyl, lower alkynyl, cyclo(lower)alkyl, lower alkoxycarbonyl, 1-oxo(lower)alkyl, cyclo(lower)alkyl substituted with a lower alkyl, lower alkenyl substituted with phenyl, 2,3-dihydro-8-oxocyclohepta[b]furan-2-ylmethyl, 2,3-diacetyloxypropyl, 2-(1,3-dihydro-1,3-dioxo-2H-isoindol-2-yl)ethyl; a halide of formula X-Alk-$R^4$ wherein $R^4$, X and Alk are as defined herein; or a halide of formula

$$X\text{—}CR^5R^6\text{—}Alk^1\text{—}R^7 \quad (\text{with OH on } Alk^1)$$

wherein $R^5$, $R^6$, $R^7$, X and $Alk^1$ are as defined herein in the presence of a proton acceptor;

(c) acetylating a compound of formula I in which $R^1$ and $R^2$ are as defined therein and $R^3$ is 2,3-dihydroxypropyl to give a compound of formula I in which $R^1$ and $R^2$ are as defined herein and $R^3$ is 2,3-diacetyloxypropyl;

(d) acylating the compound of formula I in which $R^1$ and $R^2$ are as defined therein and $R^3$ is hydrogen, to give the corresponding compound of formula I in which $R^1$ and $R^2$ are as defined therein and $R^3$ is 1-oxo(lower)alkyl;

(e) reacting a compound of formula I in which $R^1$ and $R^2$ are as defined therein and $R^3$ is a 2-oxopropyl group with potassium cyanide and ammonium carbonate to obtain a compound of formula I in which $R^1$ and $R^2$ are as defined therein and $R^3$ is 5-methyl-2,4-imidazolidinedione-5-yl-methyl;

(f) acetylating a compound of formula I in which $R^1$ and $R^2$ are as defined therein and $R^3$ is a 2-

aminoethyl group to give a compound of formula I in which $R^1$ and $R^2$ are as defined therein and $R^3$ is 2-(acetylamino)ethyl;

(g) reacting the compound of formula I in which $R^1$ and $R^2$ are as defined herein and $R^3$ is hydrogen in the presence of a proton acceptor with a tosylate of the formula TsO-Alk-$R^4$ wherein Alk is as defined herein and $R^4$ is phenyl, phenoxy, phenyl mono-, di- or trisubstituted with lower alkyl, halo, lower alkoxy or trifluoromethyl, or phenoxy mono-, di- or trisubstituted with lower alkyl, halo, lower alkoxy, acetylamino or trifluoromethyl to give the corresponding compound of formula I in which $R^1$ and $R^2$ are as defined herein and $R^3$ is a group of formula Alk-$R^4$ wherein Alk is as defined herein and $R^4$ is as defined immediately above;

(h) reacting the compound of formula I in which $R^1$ and $R^2$ are as defined herein and $R^3$ is a group of formula Alk-$R^4$ wherein Alk is as defined herein and $R^4$ is phenyl mono-, di- or trisubstituted with lower alkoxy with boron tribromide to obtain the corresponding compound of formula I in which $R^1$ and $R^2$ are as defined herein and $R^3$ is a group of formula Alk-$R^4$ wherein Alk is as defined herein and $R^4$ is phenyl mono-, di or trisubstituted with hydroxy;

(i) reacting the compound of formula I in which $R^1$ and $R^2$ are as defined herein and $R^3$ is a group of formula Alk-$R^4$ wherein Alk is as defined herein and $R^4$ is cyano with concentrated sulfuric acid, followed by concentrated ammonium hydroxide, to give the corresponding compound of formula I in which $R^1$ and $R^2$ are as defined herein and $R^3$ is a group of formula Alk-$R^4$ wherein Alk is as defined herein and $R^4$ is aminocarbonyl;

(j) acylating a compound of formula IV in which

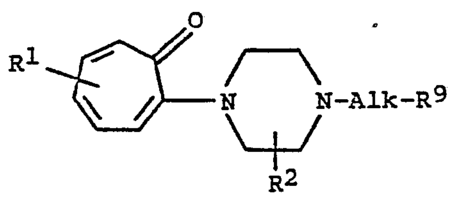

(IV)

Alk, $R^1$ and $R^2$ are as defined herein and $R^9$ is hydroxy to obtain the corresponding compound of formula I in which $R^1$ and $R^2$ are as defined herein and $R^3$ is a group of formula Alk-$R^4$ wherein Alk is as defined herein and $R^4$ is 1-oxo(lower)alkoxy;

(k) reacting the compound of formula IV in which Alk, $R^1$ and $R^2$ are as defined herein and $R^9$ is chloro with thiourea to give the corresponding intermediate having an [(aminoiminomethyl)amino] thio group and acetylating said latter intermediate to give the corresponding compound of formula I in which $R^1$ and $R^2$ are as defined herein and $R^3$ is a group of formula Alk-$R^4$ wherein Alk is as defined herein and $R^4$ is acetylthio;

(1) reacting the compound of formula I in which $R^1$ and $R^2$ are as defined herein and $R^3$ is hydrogen with an epoxide of formula

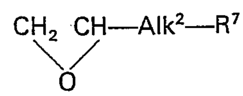

wherein $Alk^2$ is a divalent lower alkyl having one to five carbon atoms and $R^7$ is as defined herein to give the corresponding compound of formula I in which $R^1$ and $R^2$ are as defined herein; and $R^3$ is a group of formula

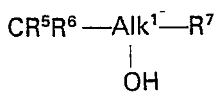 wherein $R^5$ and $R^6$ are hydrogen; 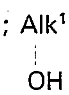

is CH(OH)-$Alk^2$ wherein $Alk^2$ is as defined herein; and $R^7$ is as defined herein;

(m) reacting the compound of formula I in which $R^1$ and $R^2$ are as defined herein and $R^3$ is hydrogen in the presence of a proton acceptor with a chloroepoxide of the formula

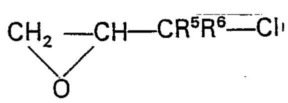 or 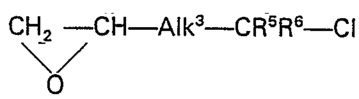

wherein $Alk^3$ is a divalent lower alkyl having one to four carbon atoms and $R^5$ and $R^6$ are as defined herein to give the corresponding compound of formula I in which $R^1$ and $R^2$ are as defined herein and $R^3$ is a group of formula

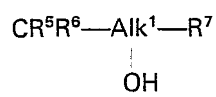

wherein $R^5$ and $R^6$ are as defined herein; $R^7$ is a hydroxy; and

$$Alk^1 \text{ is } \underset{\displaystyle OH}{\underset{|}{CH}}—\underset{\displaystyle OH}{\underset{|}{CH_2}}— \text{ or } Alk^3—\underset{\displaystyle OH}{\underset{|}{CH}}—CH_2—$$

wherein $Alk^3$ is as defined herein;

(n) reducing a ketone of formula (V)

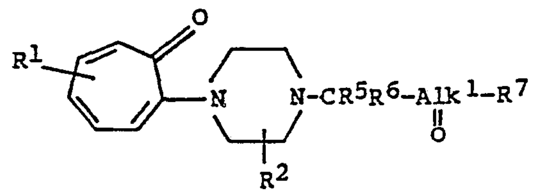

in which $R^1$, $R^2$ $R^5$, $R^6$ and $R^7$ are as defined herein and

$$—\underset{\displaystyle O}{\underset{\|}{Alk^1}}—$$

is a divalent alkanone having one to six carbon atoms with the proviso that when $R^7$ is hydroxy, the hydroxy and ketone groups are joined to different carbon atoms, to obtain the corresponding compound of formula I in which $R^1$ and $R^2$ are as defined herein and $R^3$ is a group of formula

$$CR^5R^6—\underset{\displaystyle OH}{\underset{|}{Alk^1}}—R^7$$

wherein $R^5$, $R^6$, $R^7$ and $Alk^1$ are as defined herein;

(o) reacting a compound of formula (I) in which $R^1$ and $R^2$ are as defined herein and $R^3$ is hydrogen with ethylene oxide to give a compound of formula (I) in which $R^1$ and $R^2$ are as defined herein and $R^3$ is $—CH_2CH_2OH$; and

(p) reacting the compound of formula I in which $R^1$, $R^2$ and $R^3$ are as defined herein with a therapeutically acceptable acid to obtain the corresponding compound of formula I in which $R^1$, $R^2$ and $R^3$ are as defined herein as a therapeutically acceptable acid addition salt.

2. A process as claimed in claim 1 for preparing a compound of formula I in which $R^1$ represents a substituent at position 7 of the 2,4,6-cycloheptatrien-1-one ring and is selected from hydrogen, halo, lower alkyl, lower alkoxy, trifluoromethyl, or 1-oxo(lower)alkylamino; $R^2$ is hydrogen or lower alkyl having one to three carbon atoms; and $R^3$ is hydrogen, phenyl, lower alkyl, lower alkenyl, lower alkynyl, cyclo(lower)alkyl, lower alkoxycarbonyl, 1-oxo(lower)alkyl, cyclo(lower)alkyl substituted with a lower alkyl; lower alkenyl substituted with phenyl; phenyl mono-, di- or trisubstituted with lower alkyl, halo, lower alkoxy, hydroxy or trifluoromethyl; 2-(4,5-dihydro-2-oxazolyl)benzoyl, 2,3-dihydro-8-oxocyclohepta[b]furan-2-ylmethyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to six carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono-, di- or trisubstituted with lower alkoxy or hydroxy, or phenoxy mono-, di- or trisubstituted with lower alkyl, halo or acetylamino, or a group of formula

$$CR^5R^6—\underset{\displaystyle OH}{\underset{|}{Alk^1}}—R^7$$

wherein $Alk^1$ is a trivalent alkylene having one to six carbon atoms; $R^5$ and $R^6$ each is hydrogen or lower alkyl having one to three carbon atoms; and $R^7$ is hydrogen, phenyl, hydroxy, phenoxy, phenyl mono-, di- or trisubstituted with methylsulfonylamino, lower alkoxy or hydroxy, or phenoxy mono-, di- or trisubstituted with lower alkyl or halo; with the proviso that when $R^7$ is hydroxy, the hydroxy groups are joined to different carbon atoms; or a therapeutically acceptable acid addition salt thereof.

3. A process as claimed in claim 1 for preparing a compound of formula I in which $R^1$ represents a substituent at position 7 of the 2,4,6-cycloheptatrien-1-one ring and is selected from hydrogen or bromo; $R^2$ is hydrogen or methyl; and $R^3$ is hydrogen, phenyl, lower alkyl, lower alkenyl, lower alkynyl,

cyclo(lower)alkyl, lower alkoxycarbonyl, 1-oxo((lower)alkyl, cyclo(lower)alkyl substituted with a lower alkyl, phenyl monosubstituted with lower alkyl, halo or trifluoromethyl, 2-(4,5-dihydro-2-oxazolyl)-benzoyl, 1-oxo-2,4,6-cycloheptatrien-2-yl, 2-(acetylamino)ethyl, 2,3-diacetyloxypropyl, 2-(1,3-dihydro-1,3-dioxo-2H-isoindol-2-yl)ethyl, 5-methyl-2,4-imidazolidinedione-5-ylmethyl, a group of formula Alk-$R^4$ wherein Alk is a divalent alkyl having one to three carbon atoms and $R^4$ is lower alkoxy, cyano, aminocarbonyl, lower alkoxycarbonyl, cyclo(lower)alkyl, phenyl, phenoxy, hydroxy(lower)alkoxy, 3-indolyl, 1-oxo-2,4,6-cycloheptatrien-2-yl-amino, 1H-imidazol-4-yl, 1-oxo(lower)alkoxy, acetylthio, phenyl mono- or disubstituted with lower alkoxy or hydroxy, or phenoxy monosubstituted with lower alkyl, halo or acetylamino; or a group of formula

$$CR^5R^6—Alk^1—R^7$$
$$\quad\quad\quad\quad |$$
$$\quad\quad\quad\quad OH$$

wherein $Alk^1$ is a trivalent alkylene having one to three carbon atoms; $R^5$ and $R^6$ each is hydrogen or lower alkyl having one to three carbon atoms; $R^7$ is hydrogen, phenyl, hydroxy, phenoxy, phenyl mono-, di- or trisubstituted with lower alkoxy or hydroxy or phenoxy monosubstituted with lower alkyl or halo, with the proviso that when $R^7$ is hydroxy, the hydroxy groups are joined to different carbon atoms; or a therapeutically acceptable acid addition salt thereof.

4. A process as claimed in Claim 1 for preparing a compound of formula I in which $R^1$ and $R^2$ are hydrogen; and $R^3$ is hydrogen, lower alkyl, lower alkenyl, lower alkoxycarbonyl, a group of formula Alk-$R^4$ wherein Alk is a divalent alkyl having one or two carbon atoms and $R^4$ is lower alkoxy, cyano, 1-oxo(lower)alkoxy, or phenyl mono- or disubstituted with lower alkoxy or hydroxy, or a group of formula

$$CR^5R^6—Alk^1—R^7$$
$$\quad\quad\quad\quad |$$
$$\quad\quad\quad\quad OH$$

wherein $Alk^1$ is CH; $R^5$ is hydrogen, $R^6$ is hydrogen or lower alkyl having one to three carbon atoms; and $R^7$ is hydrogen, phenyl or phenyl mono-, di- or trisubstituted at positions 3, 4 or 5 with lower alkoxy or hydroxy; or a therapeutically acceptable acid addition salt thereof.

5. A process as claimed in Claim 1, wherein the compound of formula I, which is prepared as selected from the group of (—)-2-[4-[2-hydroxy-2-(3,4-dimethoxyphenyl)ethyl]-1-piperazinyl]-2,4,6-cycloheptatrien-1-one [having $[\alpha]_D^{25}$—12.0° (c=1, dimethylformamide)], (—)-2-[4-[2-hydroxy-1-methyl-2-(3,4-dimethoxyphenyl)ethyl]-1-piperazinyl]-2,4,6-cycloheptatrien-1-one, [having $[\alpha]_D^{25}$—82.5° (c=1, dimethylformamide)], 2-[4-[2-hydroxy-2-(3,4-dimethoxyphenyl)ethyl-1-piperazinyl]-2,4,6-cycloheptatrien-2-one, 2-[4-[2-hydroxy-1-methyl-2-(3,4-dimethoxyphenyl)ethyl-1-piperazinyl]-2,4,6-cycloheptatrien-1-one, 2-[4-[2-hydroxy-1-methyl-2-(3,4-dimethoxyphenyl)ethyl]-1-piperazinyl]-2,4,6-cycloheptatrien-1-one, [the maleate salt thereof having m.p. 165—168°C], 4-(2-oxo-3,5,7-cycloheptatrien-1-yl)-1-piperazine-ethanol, (+)-2-[4-[2-hydroxy-2-(3,4-dimethoxyphenyl)ethyl-1-piperazinyl]-2,4,6-cycloheptatrien-1-one [having $[\alpha]_D^{25}$+ 11.7° (c=1, dimethylformamide)], 2-[4-(2-hydroxypropyl)-1-piperazinyl]-2,4,6-cycloheptatrien-1-one, (+)-2-[4-[2-hydroxy-1-methyl-2-(3,4-dimethoxyphenyl)ethyl]-1-piperazinyl]-2,4,6-cycloheptatrien-1-one, [having $[\alpha]_D^{25}$ +84.2° (c=1, dimethylformamide)] and 2-[4-[2-hydroxy-2-(4-methoxyphenyl)ethyl]-1-piperazinyl-2,4,6-cycloheptatrien-1-one, or a therapeutically acceptable acid addition salt thereof.

6. A process as claimed in Claim 1 wherein the compound of formula I which is prepared is 7-bromo-2-[4-(2-hydroxyethyl)-1-piperazinyl]-2,4,6-cycloheptatrien-1-one, or a therapeutically acceptable acid addition salt thereof.

7. A process as claimed in Claim 1, wherein the compound of formula I which is prepared is selected from the group of 4-(2-oxo-3,5,7-cycloheptatrien-1-yl)-1-piperazine-acetonitrile, 2-[4-[2-(3,4-dimethoxyphenyl)ethyl]-1-piperazinyl]-2,4,6-cycloheptatrien-1-one, 2-[4-[2-(acetyloxy)ethyl-1-piperazinyl]-2,4,6-cycloheptatrien-1-one, 2,2-dimethylpropanoic acid, 2-[4-(2-oxo-3,5,7-cycloheptatrien-1-yl)-1-piperazinyl]ethyl ester, or a therapeutically acceptable acid addition salt thereof.

8. A process as claimed in Claim 1, wherein the compound of formula I which is prepared is selected from the group of 2-(4-methyl-1-piperazinyl)-2,4,6-cycloheptatrien-1-one, 2-[4-(2-propenyl)-1-piperazinyl]-2,4,6-cycloheptatrien-1-one, 2-[4-(1-methylethyl)-1-piperazinyl]-2,4,6-cycloheptatrien-1-one, 2-(4-propyl-1-piperazinyl)-2,4,6-cycloheptatrien-1-one and 2-[4-(1-methylpropyl)-1-piperazinyl]-2,4,6-cycloheptatrien-1-one or a therapeutically acceptable acid addition salt thereof.

9. A process of Claim 1 for preparing a compound of formula I in which $R^1$ and $R^2$ are as defined herein and $R^3$ is a group of formula

$$CR^5R^6—Alk^1—R^7$$
$$\quad\quad\quad\quad |$$
$$\quad\quad\quad\quad OH$$

wherein $R^5$, $R^6$, $R^7$ and $Alk^1$ are as defined therein, which comprises reducing the corresponding ketone of formula V in which $R^1$, $R^2$, $R^5$, $R^6$, $R^7$ and

$$\underset{\displaystyle O}{\overset{\displaystyle Alk^1}{\|}}$$

as defined therein with sodium borohydride.

10. A process of Claim 9 for preparing a compound of formula I in which $R^1$ and $R^2$ are hydrogen; and $R^3$ is a group of formula

$$CR^5R^6—\underset{\displaystyle OH}{\underset{|}{Alk^1}}—R^7$$

wherein $Alk^1$ is CH; $R^5$ is hydrogen; $R^6$ is hydrogen or lower alkyl having one to three carbon atoms; and $R^7$ is phenyl or phenyl mono-, di- or trisubstituted at positions 3, 4 or 5 with lower alkoxy or hydroxy.

11. A process of Claim 1 for preparing a compound of formula I in which $R^1$ and $R^2$ are hydrogen and $R^3$ is a group of formula

$$CR^5R^6—\underset{\displaystyle OH}{\underset{|}{Alk^1}}—R^7$$

wherein $Alk^1$ is CH, $R^5$ is hydrogen, $R^6$ is hydrogen or lower alkyl having one to three carbon atoms, and $R^7$ is phenyl or phenyl mono-, di- or trisubstituted at positions 3, 4 or 5 with lower alkoxy or hydroxy, which comprises condensing a compound of formula II in which $R^1$ is as defined herein and $R^8$ is methyl or ethyl with a compound of formula III in which $R^2$ and $R^3$ are as defined herein.

12. A process wherein a compound of formula I, as defined in claim 9, 10 or 11, is resolved to obtain the separate optical isomers.

13. A process for preparing a compound of formula V, as defined in claim 1, which comprises reacting a corresponding compound of formula I in which $R^1$ and $R^2$ are as defined in claim 1 and $R^3$ is hydrogen with an alkanone of the formula

$$X—CR^5R^6—\underset{\displaystyle O}{\underset{\|}{Alk^1}}—R^7 \quad \text{wherein } R^5, R^6, R^7 \text{ and } \underset{\displaystyle O}{\underset{\|}{Alk^1}}$$

are as defined in claim 1 and X is bromo, chloro or iodo in the presence of a proton acceptor.

14. A process for preparing a pharmaceutical composition which comprises mixing a compound of formula I as defined in claim 1 or a therapeutically acceptable acid addition salt thereof with a pharmaceutically acceptable carrier.

**Patentansprüche für die Vertragsstaaten: BE CH DE FR GB IT LI LU NL SE**

1. Eine Verbindung der Formel (I)

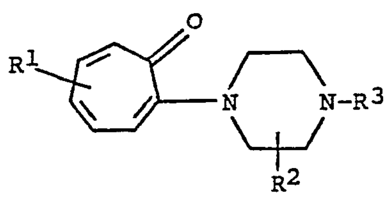

(I)

worin $R^1$ ein Substituent in den Stellungen 3, 4, 5, 6 oder 7 des 2, 4, 6-Cycloheptatrien-1-on-Ringes ist und unter Wasserstoff, Halogen, nied.Alkyl, nied.Alkoxy, Trifluormethyl oder 1-Oxo(nied.)alkylamino gewählt ist; $R^2$ Wasserstoff oder nied.Alkyl mit 1 bis 3 C-Atomen bedeutet; und $R^3$ Wasserstoff, Phenyl, nied.Alkyl, nied.Alkenyl, nied.Alkinyl, Cyclo(nied.)alkyl, nied.Alkoxycarbonyl, 1-Oxo(nied.)alkyl, Cyclo(nied.)alkyl substituiert mit nied.Alkyl; nied.Alkenyl substituiert mit Phenyl; Phenyl mono-, di- oder trisubstituiert mit nied.Alkyl, Halogen, nied.Alkoxy, Hydroxy oder Trifluormethyl; 2-(4,5-Dihydro-2-oxazolyl)-benzoyl, 2,3-Dihydro-8-oxo-cyclohepta[b]furan-2-ylmethyl, 1-Oxo-2,4,6-cycloheptatrien-2-yl, 2-(Acetylamino)-äthyl, 2,3-Diacetyloxypropyl, 2-(1,3-Dihydro-1,3-dioxo-2H-isoindol-2-yl)-äthyl, 5-Methyl-2,4-imidazolidindion-5-ylmethl, eine Gruppe der Formel Alk-$R^4$, wobei Alk eine zweiwertige Alkylgruppe mit 1 bis 6 C-Atomen ist, bedeutet; und $R^4$ nied.Alkoxy, Cyano, Aminocarbonyl, nied.Alkoxycarbonyl, Cyclo(nied.)alkyl, Phenyl, Phenoxy, Hydroxy(nied.)alkoxy, 3-Indolyl, 1-Oxo-2,4,6-cycloheptatrien-2-yl-amino, 1H-Imidazol-4-yl, 1-Oxo(nied.)alkoxy, Acetylthio, Phenyl mono-, di- oder

trisubstituiert mit nied.Alkyl, Halogen, nied.Alkoxy, Hydroxy oder Trifluormethyl, oder Phenoxy mono-, di- oder trisubstituiert mit nied.Alkyl, Halogen, nied.Alkoxy, Acetylamino, Hydroxy oder Trifluormethyl oder eine Gruppe der Formel

$$\underset{\displaystyle OH}{CR^5R^6—\overset{}{Alk^1}}—R^7$$

bedeutet, wobei $Alk^1$ driewertiges Alkylen mit 1 bis 6 C-Atomen ist; $R^5$ und $R^6$ jeweils Wasserstoff oder nied.Alkyl mit 1 bis 3 C-Atomen sind; und $R^7$ Wasserstoff, Phenyl, Hydroxy, Phenoxy, Phenyl mono-, di- oder trisubstituiert mit Methylsulfonylamino, nied.Alkyl, Halogen, nied.Alkoxy, Hydroxy oder Trifluormethyl, oder Phenoxy mono-, di- oder trisubstituiert mit nied.Alkyl, Halogen, nied.Alkoxy, Hydroxy oder Trifluormethyl darstellt; mit der Maßgabe, daß, wenn $R^7$ Hydroxy ist, die Hydroxygruppen an verschiedene C-Atome gebunden sind; oder ein therapeutisch annehmbares Säureadditionssalz hievon (wobei der Ausdruck "nied.Alkyl", wenn nichts anderesangegeben, geradkettiges Alkyl mit 1 bis 6 C-Atomen oder verzweigtkettiges Alkyl mit 3 oder 4 C-Atomen bedeutet; der Ausdruck "nied.Alkoxy" einen geradkettigen Alkoxyrest mit 1 bis 6 C-Atomen oder einen verzweigtkettigen Alkoxyrest mit 3 oder 4 C-Atomen darstellt; der Ausdruck "1-Oxo(nied.)alkyl" einen geradkettigen 1-Oxoalkylrest mit 2 bis 6 C-Atomen oder einen verzweigtket igen 1-Oxoalkylrest mit 4 bis 6 C-Atomen bedeutet; der Ausdruck "nied.Alkenyl" einen geradkettigen Alkenylrest mit 2 bis 6 C-Atomen oder einen Verzweigtkettigen Alkenylrest mit 3 oder 4 C-Atomen bezeichnet, der Ausdruck "nied.Alkinyl" einen geradkettigen Alkinylrest mit 2 bis 6 C-Atomen oder einen verzweigtkettigen Alkinylrest mit 4 C-Atomen darstellt; der Ausdruck "1-Oxo(nied.)alkoxy" einen geradkettigen 1-Oxoalkoxyrest mit 2 bis 6 C-Atomen oder einen verzweigtkettigen 1-Oxoalkylrest mit 4 bis 6 C-Atomen bedeutet; und der Ausdruck "Cyclo-(nied.)alkyl" einen gesättigten cyclischen Kohlenwasserstoffrest mit 3 bis 6 C-Atomen bezeichnet).

2. Eine Verbindung gemäß Anspruch 1, worin $R^1$ einen Substituenten in Stellung 7 des 2,4,6-Cycloheptatrien-1-on-Ringes bedeutet und ausgewählt ist unter Wasserstoff, Halogen, nied.Alkyl, nied.-Alkoxy, Trifluormethyl oder 1-Oxo(nied.)alkylamino; $R^2$ Wasserstoff oder nied.Alkyl mit 1 bis 3 C-Atomen darstellt, und $R^3$ Wasserstoff, Phenyl, nied.Alkyl, nied.Alkenyl, nied.Alkinyl, Cyclo(nied.)alkyl substituiert mit nied.Alkyl; nied.Alkenyl substituiert mit Phenyl; Phenyl mono-, di- oder trisubstituiert mit nied.Alkyl, Halogen, nied.Alkoxy, Hydroxy oder Trifluormethyl; 2-(4,5-Dihydro-2-oxazolyl)benzoyl, 2,3-Dihydro-8-oxo-cyclohepta[b]furan-2-ylmethyl, 1-Oxo-2,4,6-cycloheptatrien-2-yl, 2-(Acetylamino)-äthyl, 2,3-Di-acetyloxypropyl, 2-(1,3-Dihydro-1,3-dioxo-2H-isoindol-2-yl)-äthyl, 5-Methyl-2,4-imidazolidindion-5-ylmethyl, eine Gruppe der Formel Alk-$R^4$, wobei Alk zweiwertiges Alkyl mit 1 bis 6 C-Atomen bedeutet und $R^4$ nied.Alkoxy, Cyano, Aminocarbonyl, nied.Alkoxycarbonyl, Cyclo(nied.)alkyl, Phenyl, Phenoxy, Hydroxy(nied.)alkoxy, 3-Indolyl, 1-Oxo-2,4,6-cycloheptatrien-2-yl-amino, 1H-Imidazol-4-yl, 1-Oxo(nied.)alkoxy, Acetylthio, Phenyl mono-, di- oder trisubstituiert mit nied.Alkoxy oder Hydroxy, oder Phenoxy mono-, di- oder trisubstituiert mit nied.Alkyl, Halogen oder Acetylamino, oder eine Gruppe der Formel

$$\underset{\displaystyle OH}{CR^5R^6—\overset{}{Alk^1}}—R^7,$$

wobei $Alk^1$ dreiwertiges $C_1—C_6$-Alkylen ist, darstellt; $R^5$ und $R^6$ jeweils Wasserstoff oder nied.Alkyl mit bis 3 C-Atomen sind; und $R^7$ Wasserstoff, Phenyl, Hydroxy, Phenoxy, Phenyl mono-, di- oder trisubstituiert mit Methylsulfonylamino, nied.Alkoxy oder Hydroxy, oder Phenoxy mono-, di- oder trisubstituiert mit nied.Alkyl oder Halogen, ist; mit der Maßgabe, daß, wenn $R^7$ Hydroxy ist, die Hydroxygruppen an verschiedene C-Atome gebunden sind; oder ein therapeutisch annehmbares Säureadditionssalz hievon.

3. Eine Verbindung gemäß Anspruch 1, worin $R^1$ einen Substituenten in Stellung 7 des 2,4,6-Cycloheptatrien-1-on-Ringes bedeutet und ausgewählt ist unter Wasserstoff oder Brom; $R^2$ Wasserstoff oder methyl darstellt; und $R^3$ Wasserstoff, Phenyl, nied.Alkyl, nied.Alkenyl, nied.Alkinyl, Cyclo(nied.)alkyl, nied.Alkoxycarbonyl, 1-Oxo(nied.)alkyl, Cyclo(nied.)alkyl substituiert mit nied.Alkyl, Phenyl monosubstituiert mit nied.Alkyl, Halogen oder Trifluormethyl, 2-(4,5-Dihydro-2-oxazolyl)-benzoyl, 1-Oxo-2,4,6-cycloheptatrien-2-yl, 2-(Acetylamino)-äthyl, 2,3-Diacetyloxypropyl, 2-(1,3-Dihydro-1,3-dioxo-2H-isoindol-2-yl)-äthyl, 5-Methyl-2,4-imidazolidindion-5-ylmethyl, eine Gruppe der Formel Alk-$R^4$, wobei Alk zweiwertiges Alkyl mit 1 bis 3 C-Atomen ist und $R^4$ nied.Alkoxy, Cyano, Aminocarbonyl, nied.Alkoxycarbonyl, Cyclo(nied.)alkyl, Phenyl, Phenoxy, Hydroxy(nied.)alkoxy, 3-Indolyl, 1-Oxo-2,4,6-cycloheptatrien-2-yl-amino, 1H-Imidazol-4-yl, 1-Oxo(nied.)alkoxy, Acetylthio, Phenyl mono- oder disubstituiert mit nied.Alkoxy oder Hydroxy, oder Phenoxy monosubstituiert mit nied.Alkyl, Halogen oder Acetylamino bedeutet; oder eine Gruppe der Formel

$$\underset{\displaystyle OH}{CR^5R^6—\overset{}{Alk^1}}—R^7$$

ist, wobei Alk¹ dreiwertiges Alkyl mit 1 bis 3 C-Atomen bedeutet, $R^5$ und $R^6$ jeweils Wasserstoff oder nied.Alkyl mit 1 bis 3 C-Atomen darstellen; $R^7$ Wasserstoff, Phenyl, Hydroxy, Phenoxy, Phenyl mono-, di- oder trisubstituiert mit nied.Alkoxy oder Hydroxy, oder Phenoxy monosubstituiert mit nied.Alkyl oder Halogen bedeutet; mit der Maßgabe, daß, wenn $R^7$ Hydroxy ist, die Hydroxygruppen an verschiedene C-Atome gebunden sind; oder ein therapeutisch annehmbares Säurreadditionssalz hievon.

4. Eine Verbindung gemäß Anspruch 1, worin $R^1$ und $R^2$ Wasserstoff bedeuten; und $R^3$ Wasserstoff, nied.Alkyl, nied.Alkenyl, nied.Alkoxycarbonyl, eine Gruppe der Formel Alk-$R^4$ ist, wobei Alk zweiwertiges Alkyl mit 1 oder 2 C-Atomen und $R^4$ nied.Alkoxy, Cyano, 1-Oxo(nied.)alkoxy, oder Phenyl mono- oder disubstituiert mit nied.Alkoxy oder Hydroxy, oder eine Gruppe der Formel

$$CR^5R^6—Alk^1—R^7$$
$$|$$
$$OH$$

ist, wobei Alk¹ CH bedeutet; $R^5$ Wasserstoff darstellt; $R^6$ Wasserstoff oder nied.Alkyl mit 1 bis 3 C-Atomen ist und $R^7$ Wasserstoff Phenyl oder Phenyl mono-, di- oder trisubstituiert in Stellung 3, 4 oder 5 mit nied.Alkoxy oder Hydroxy bedeutet; oder ein therapeutisch annehmbares Säureadditionssalz hievon.

5. Eine Verbindung wie in Anspruch 1 beansprucht, die ausgewählt ist aus der Gruppe (—)-2-[4-[2-Hydroxy-2-(3,4-dimethoxyphenyl)äthyl]-1-piperazinyl]-2,4,6-cycloheptatrien-1-on (mit $[\alpha]_D^{25}$ —12,0° (c = 1, Dimethylformamid)], (—)-2-[4-[2-Hydroxy-1-methyl-2-(3,4-dimethyoxyphenyl)-äthyl]-1-piperazinyl]-2,4,6-cycloheptatrien-1-on (mit $[\alpha]_D^{25}$ —82,5° (c = 1, Dimethylformamid)], 2-[4-[2-Hydroxy-2-(3,4-dimethoxyphenyl)-äthyl-1-piperazinyl]-2,4,6-cycloheptatrien-1-on, 2-[4-[2-Hydroxy-1-methyl-2-(3,4-dimethoxyphenyl)-äthyl-1-piperazinyl]-2,4,6-cycloheptatrien-1-on, 2-[4-[2-Hydroxy-1-methyl-2-(3,4-dimethoxyphenyl)-äthyl]-1-piperazinyl]-2,4,6-cycloheptatrien-1-on (dessen Maleatsalz einen Fp. von 165—168°C aufweist), 4-(2-Oxo-3,5,7-cycloheptatrien-1-yl)-1-piperazin-äthanol, (+)-2-[4-[2-Hydroxy-2-(3,4-dimethoxyphenyl)-äthyl-1-piperazinyl]-2,4,6-cycloheptatrien-1-on (mit $[\alpha]_D^{25}$ +11,7° (c = 1, Dimethylformamid)), 2-[4-(2-Hydroxypropyl)-1-piperazinyl]-2,4,6-cycloheptatrien-1-on, (+)-2-[4 - [2 - Hydroxy - 1 - methyl - 2 - (3,4 - dimethoxyphenyl) - äthyl] - 1 - piperazinyl] - 2,4,6 - cycloheptatrien-1-on [mit $[\alpha]_D^{25}$ +84,2° (c = 1, Dimethylformamid)] und 2-[4-[2-Hydroxy-2-(4-methoxyphenyl)-äthyl]-1-piperazinyl-2,4,6-cycloheptatrien-1-on oder ein therapeutisch annehmbares Säureadditionssalz hievon.

6. Eine Verbindung wie in Anspruch 1 beansprucht, nämlich 7-Brom-2-[4-(2-hydroxyäthyl)-1-piperazinyl]-2,4,6-cycloheptatrien-1-on oder ein therapeutisch annehmbares Säureadditionssalz hievon.

7. Eine Verbindung wie in Anspruch 1 beansprucht, die ausgewählt ist aus der Gruppe 4-(2-Oxo-3,5,7-cycloheptatrien-1-yl)-1-piperazin-acetonitril, 2-[4-[2-(3,4-Dimethoxyphenyl)-äthyl]-1-piperazinyl]-2,4,6-cycloheptatrien-1-on, 2-[4-[2-(Acetyloxy)-äthyl-1-piperazinyl]-2,4,6-cycloheptatrien-1-on, 2,2-Dimethylpropansäure, 2-[4-(2-Oxo-3,5,7-Cycloheptatrien-1-yl)-1-piperazinyl]-äthylester oder ein therapeutisch annehmbares Säureadditionssalz hievon.

8. Eine Verbindung wie in Anspruch 1 beansprucht, die ausgewählt ist aus der Gruppe 2-(4-Methyl-1-piperazinyl)-2,4,6-cycloheptatrien-1-on, 2-[4-(2-Propenyl)-1-piperazinyl]-2,4,6-cycloheptatrien-1-on, 2-[4-(1-Methyläthyl)-1-piperazinyl]-2,4,6-cycloheptatrien-1-on, 2-(4-Propyl-1-piperazinyl)-2,4,6-cycloheptatrien-1-on und 2-[4-(1-Methylpropyl)-1-piperazinyl]-2,4,6-cycloheptatrien-1-on oder ein therapeutisch annehmbares Säureadditionssalz hievon.

9. Eine Verbindung der Formel

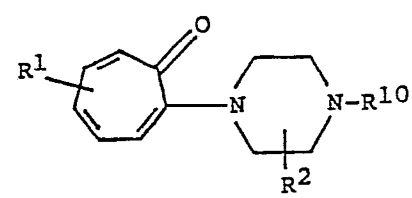

worin $R^1$ ein Substituent in den Stellungen 3, 4, 5, 6 oder 7 des 2,4,6-Cycloheptatrien-1-on-Ringes ist und ausgewählt ist unter Wasserstoff, Halogen, nied.Alkyl, nied.Alkoxy, Trifluormethyl oder 1-Oxo(nied.)alkylamino; $R^2$ Wasserstoff oder nied.Alkyl mit 1 bis 3 C-Atomen bedeutet; $R^{10}$ 2-Aminoäthyl, -Alk-Cl (wobei Alk ein zweiwertiger Alkylrest mit 1 bis 6 C-Atomen ist) oder eine Gruppe der Formel

$CR^5R^6—Alk^1—R^7$ (with OH on Alk¹) bedeutet, wobei $Alk^1$ (=O)

ein zweiwertiges Alkanon mit 1 bis 6 C-Atomen darstellt; $R^5$ und $R^6$ jeweils Wasserstoff oder nied.Alkyl mit 1 bis 3 C-Atomen bedeuten; und $R^7$ Wasserstoff, Phenyl, Hydroxy, Phenoxy, Phenyl mono-, di- oder

trisubstituiert mit Methylsulfonylamino, nied.Alkyl, Halogen, nied.Alkoxy, Hydroxy oder Trifluormethyl, oder Phenoxy mono-, di- oder trisubstituiert mit nied.Alkyl, Halogen, nied.Alkoxy, Hydroxy oder Trifluormethyl ist; mit der Maßgabe, daß, wen $R^7$ Hydroxy ist, die Hydroxy- und Ketongruppen an verschiedene C-Atome gebunden sind [und wobei der Ausdruck "nied.Alkyl", wenn nichtes anderes angegeben, einen geradkettigen Alkylrest mit 1 bis 6 C-Atomen oder einen verzweigtkettigen Alkylrest mit 3 oder 4 C-Atomen bedeutet; der Ausdruck "nied.Alkoxy" einen geradkettigen Alkoxyrest mit 1 bis 6 C-Atomen oder einen verzweigtkettigen Alkoxyrest mit 3 oder 4 C-Atomen darstellt; und der Ausdruck "1-Oxo-(nied.)alkyl" ein geradkettiger 1-oxoalkylrest mit 2 bis 6 C-Atomen oder ein verzweigtkettiger 1-Oxoalkylrest mit 4 bis 6 C-Atomen ist.

10. Eine Verbindung gemäß Anspruch 9, worin $R^1$ und $R^2$ Wasserstoff sind und $R^{10}$

$$CR^5R^6—\underset{\underset{O}{\|}}{Alk^1}—R^7$$

bedeutet, wobei $R^5$ und $R^6$ jeweils Wasserstoff oder nied.Alkyl mit 1 bis 3 C-Atomen darstellen; $R^7$ Wasserstoff, Phenyl oder Phenyl mono-, di- oder tri-substituiert mit Halogen, nied.Alkoxy oder Hydroxy ist und

$$\underset{\underset{O}{\|}}{Alk^1}$$

zweiwertiges Alkanon mit 1 bis 3 C-Atomen bedeutet.

11. Pharmazeutische Zusammensetzung, die eine Verbindung der Formel (I) oder ein therapeutisch annehmbares Säureadditionssalz hievon, wie in einem der Ansprüche 1 bis 8 beansprucht, und einen pharmazeutisch annehmbaren Träger umfaßt.

12. Eine Verbindung der Formel (I) oder ein therapeutisch annehmbares Säureadditionssalz hievon, wie in einem der Ansprüche 1 bis 8 beansprucht, zur Verwendung als ein dopaminrezeptorstimulierendes Mittel.

**Patentansprüche für den Vertragsstaat: AT**

1. Verfahren zum Herstellen einer Verbindung der Formel (I)

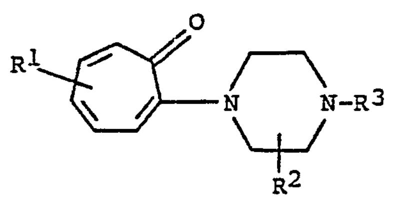

(I)

worin $R^1$ ein Substituent in den Stellungen 3, 4, 5, 6 oder 7 des 2, 4, 6-Cycloheptatrien-1-on-Ringes ist und unter Wasserstoff, Halogen, nied.Alkyl, nied.Alkoxy, Trifluormethyl oder 1-Oxo(nied.)alkylamino gewählt ist; $R^2$ Wasserstoff oder nied.Alkyl mit 1 bis 3 C-Atomen bedeutet; und $R^3$ Wasserstoff, Phenyl, nied.Alkyl, nied.Alkenyl, nied.Alkinyl, Cyclo(nied.)alkyl, nied.Alkoxycarbonyl, 1-Oxo(nied.)alkyl, Cyclo(nied.)alkyl substituiert mit nied.Alkyl; nied.Alkenyl substituiert mit Phenyl; Phenyl mono-, di- oder trisubstituiert mit nied.Alkyl, Halogen, nied.Alkoxy, Hydroxy oder Trifluormethyl; 2-(4,5-Dihydro-2-oxazolyl)-benzoyl, 2,3-Dihydro-8-oxo-cyclohepta[b]furan-2-ylmethyl, 1-Oxo-2,4,6-cycloheptatrien-2-yl, 2-(Acetylamino)-äthyl, 2,3-Diacetyloxypropyl, 2-(1,3-Dihydro-1,3-dioxo-2H-isoindol-2-yl)-äthyl, 5-Methyl-2,4-imidazolidindion-5-ylmethl, eine Gruppe der Formel Alk-$R^4$, wobei Alk eine zweiwertige Alkylgruppe mit 1 bis 6 C-Atomen ist, bedeutet; und $R^4$ nied.Alkoxy, Cyano, Aminocarbonyl, nied.Alkoxycarbonyl, Cyclo(nied.)alkyl, Phenyl, Phenoxy, Hydroxy(nied.)alkoxy, 3-Indolyl, 1-Oxo-2,4,6-cycloheptatrien-2-yl-amino, 1H-Imidazol-4-yl, 1-Oxo(nied.)alkoxy, Acetylthio, Phenyl mono-, di- oder trisubstituiert mit nied.Alkyl, Halogen, nied.Alkoxy, Hydroxy oder Trifluormethyl, oder Phenoxy mono-, di- oder trisubstituiert mit nied.Alkyl, Halogen, nied.Alkoxy, Acetylamino, Hydroxy oder Trifluormethyl oder eine Gruppe der Formel

$$CR^5R^6—\underset{\underset{OH}{|}}{Alk^1}—R^7$$

bedeutet, wobei $Alk^1$ driewertiges Alkylen mit 1 bis 6 C-Atomen ist; $R^5$ und $R^6$ jeweils Wasserstoff oder nied.Alkyl mit 1 bis 3 C-Atomen sind; und $R^7$ Wasserstoff, Phenyl, Hydroxy, Phenoxy, Phenyl mono-, di- oder trisubstituiert mit Methylsulfonylamino, nied.Alkyl, Halogen, nied.Alkoxy, Hydroxy oder Trifluormethyl, oder Phenoxy mono-, di- oder trisubstituiert mit nied.Alkyl, Halogen, nied.-Alkoxy, Hydroxy oder Trifluormethyl darstellt; mit der Maßgabe, daß, wenn $R^7$ Hydroxy ist, die Hydroxygruppen an verschiedene C-Atome gebunden sind, oder eines therapeutisch annehmbaren Säureadditionsadditionssalzes hievon (wobei der Ausdruck "nied.Alkyl", wenn nichts anderesangegeben; geradkettiges Alkyl mit 1 bis 6 C-Atomen oder verzweigtkettiges Alkyl mit 3 oder 4 C-Atomen bedeutet; der Ausdruck "nied.Alkoxy" einen geradkettigen Alkoxyrest mit 1 bis 6 C-Atomen oder einen verzweigtkettigen Alkoxyrest mit 3 oder 4 C-Atomen darstellt; der Ausdruck "1-Oxo(nied.)alkyl" einen geradkettigen 1-Oxoalkylrest mit 2 bis 6 C-Atomen oder einen verzweigtkettigen 1-Oxoalkylrest mit 4 bis 6 C-Atomen bedeutet; der Ausdruck "nied.Alkenyl" einen geradkettigen Alkenylrest mit 2 bis 6 C-Atomen oder einen verzweigtkettigen Alkenylrest mit 3 oder 4 C-Atomen bezeichnet; der Ausdruck "nied.Alkinyl" einen geradkettigen Alkinylrest mit 2 bis 6 C-Atomen oder einen verzweigtkettigen Alinylrest mit 4 C-Atomen darstellt; der Ausdruck "1-Oxo(nied.)alkoxy" eine geradkettigen 1-Oxoalkoxyrest mit 2 bis 6 C-Atomen oder einen verzweigtkettigen 1-Oxoalkoxyrest mit 4 bis 6 C-Atomen bedeutet; und der Ausdruck "Cyclo(nied.)alkyl" einen gesättigten cyclischen Kohlenwasserstoffrest mit 3 bis 6 C-Atomen bezeichnet), welches das Auswählen eines Verfahrens aus folgender Gruppe umfaßt:

a) wenn eine Verbindung der Formel (I), worin $R^1$, $R^2$ und $R^3$ wie hier definiert sind, gewünscht wird, Kondensieren einer Verbindung der Formel (II).

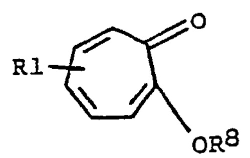

(II)

worin $R^1$ wie hier definiert ist und $R^8$ nied.Alkyl bedeutet, mit einer Verbindung der Formel (III)

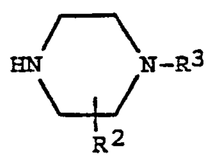

(III)

worin $R^2$ wie hier definiert ist und $R^3$ wie oben definiert ist oder Amino(nied.)alkyl bedeutet;

b) wenn eine Verbindung der Formel (I), worin $R^1$ und $R^2$ wie hier definiert sind und $R^3$ nied.Alkyl, nied.Alkenyl, nied.Alkinyl, Cyclo(nied.)alkyl, nied.Alkoxycarbonyl, 1-Oxo(nied.)alkyl, Cycyl(nied.)alkyl substituiert mit nied.Alkyl, nied.Alkenyl substituiert mit Phenyl, 2-(4,5-Dihydro-2-oxazolyl)-benzoyl, 2,3-Dihydro-8-oxo-cyclohepta[b]furan-2-ylmethyl, 2,3-Diacetyloxypropyl, 2-(1,3-Dihydro-1,3-dioxo-2H-isoindol-2-yl)-äthyl, eine Gruppe der Formel Alk-$R^4$, wobei Alk und $R^4$ wie hier definiert sind, oder eine Gruppe der Formel

$$\underset{\displaystyle OH}{\underset{|}{CR^5R^6}}—Alk^1—R^7$$

wobei $R^5$, $R^6$, $R^7$ und $Alk^1$ wie hier definiert sind, bedeutet, gewünscht wird, Umsetzen der Verbindung der Formel (I), worin $R^1$ und $R^2$ wir hier definiert sind und $R^3$ Wasserstoff ist, mit einem Halogenid der Formel X—$R^3$, wobei X Brom, Chlor oder Jod ist und $R^3$ nied.Alkyl, nied.Alkenyl, nied.Alkinyl, Cyclo(nied.)alkyl, nied.Alkoxycarbonyl, 1-Oxo(nied.)alkyl, Cyclo(nied.)alkyl substituiert mit nied.Alkyl, nied.Alkenyl substituiert mit Phenyl, 2,3-Dihydro-8-oxo-cyclohepta[b]furan-2-ylmethyl, 2,3-Diacetyloxypropyl, 2-(1,3-Dihydro-1,3-dioxo-2H-isoindol-2-yl)-äthyl darstellt; einem Halogenid der Formel X-Alk-$R^4$, wobei $R^4$, X und Alk wie hier definiert sind; oder einem Halogenid der Formel

$$X—\underset{\displaystyle OH}{\underset{|}{CR^5R^6}}—Alk^1—R^7$$

wobei $R^5$, $R^6$, $R^7$, X und $Alk^1$ wir hier definiert sind, in Anwesenheit eines Protonenakzeptors;

c) Acetylieren einer Verbindung der Formel (I), worin $R^1$ und $R^2$ wie hier definiert sind und $R^3$ 2,3-Dihydroxypropyl bedeutet, wobei eine Verbindung der Formel (I) erhalten wird, worin $R^1$ und $R^2$ wie hier definiert sind und $R^3$ 2,3-Diacetyloxypropyl ist;

d) Acylieren der Verbindung der Formel (I), worin $R^1$ und $R^2$ wir hier definiert sind und $R^3$ Wasserstoff ist, zur entsprechenden Verbindung der Formel (I), worin $R^1$ und $R^2$ wie hier definiert sind und $R^3$ 1-Oxo(nied.)alkyl ist;

e) Umsetzen einer Verbindung der Formel (I), worin $R^1$ und $R^2$ wie hier definiert sind und $R^3$ 2-Oxopropyl ist, mit Kaliumcyanid und Ammoniumcarbonat zur Erzielung einer Verbindung der Formel (I), worin $R^1$ und $R^2$ wie hier definiert sind und $R^3$ 5-Methyl-2,4-imidazolidindion-5-yl-methyl darstellt;

f) Acetylieren einer Verbindung der Formel (I), worin $R^1$ und $R^2$ wie hier definiert sind und $R^3$ eine 2-Aminoäthylgruppe ist, zu einer Verbindung der Formel (I), worin $R^1$ und $R^2$ wie hier definiert sind und $R^3$ 2-(Acetylamino)äthyl bedeutet;

g) Umsetzen der Verbindung der Formel (I), worin $R^1$ und $R^2$ wie hier definiert sind und $R^3$ Wasserstoff bedeutet, in Anwesenheit eines Protonenakzeptors mit einem Tosylat der Formel TsO-Alk-$R^4$, wobei Alk wie hier definiert ist und $R^4$ Phenyl, Phenoxy, Phenyl mono-, di- oder trisubstituiert mit nied.Alkyl, Halogen, nied.Alkoxy oder Trifluormethyl, oder Phenoxy mono-, di- oder trisubstituiert mit nied.Alkyl, Halogen, nied.Alkoxy, Acetylamino oder Trifluormethyl bedeutet, zur entsprechenden Verbindung der Formel (I), worin $R^1$ und $R^2$ wie hier definiert sind und $R^3$ eine Gruppe der Formel Alk-$R^4$ bedeutet, wobei Alk wie hier definiert ist und $R^4$ wie unmittelbar oben definiert ist;

h) Umsetzen der Verbindung der Formel (I), worin $R^1$ und $R^2$ wie hier definiert sind und $R^3$ eine Gruppe der Formel Alk-$R^4$ ist, wobei Alk wie hier definiert ist und $R^4$ Phenyl mono-, di- oder trisubstituiert mit nied.Alkoxy bedeutet, mit Bortribromid zur Erzielung der entsprechenden Verbindung der Formel (I), worin $R^1$ und $R^2$ wie hier definiert sind und $R^3$ eine Gruppe der Formel Alk-$R^4$ ist, wobei Alk wie hier definiert ist und $R^4$ Phenyl mono-, di- oder trisubstituiert mit Hydroxy bedeutet;

i) Umsetzen der Verbindung der Formel (I), worin $R^1$ und $R^2$ wie hier definiert sind und $R^3$ eine Gruppe der Formel Alk-$R^4$ bedeutet, wobei Alk wie hier definiert ist und $R^4$ Cyano darstellt, mit konz.Schwefelsäure gefolgt von konz.Ammoniumhydroxid zur Erzielung der entsprechenden Verbindung der Formel (I), worin $R^1$ und $R^2$ wie hier definiert sind und $R^3$ eine Gruppe der Formel Alk-$R^4$ bedeutet, wobei Alk wie hier definiert ist und $R^4$ Aminocarbonyl darstellt;

j) Acylieren einer Verbindung der Formel (IV)

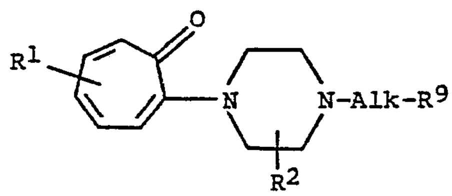

(IV)

worin Alk, $R^1$ und $R^2$ wie hier definiert sind und $R^9$ Hydroxy ist, zur Erzielung der entsprechenden Verbindung der Formel (I), worin $R^1$ und $R^2$ wie hier definiert sind und $R^3$ eine Gruppe der Formel Alk-$R^4$ ist, wobei Alk wie hier definiert ist und $R^4$ 1-Oxo(nied.)alkoxy bedeutet;

k) Umsetzen der Verbindung der Formel (IV), worin Alk, $R^1$ und $R^2$ wie hier definiert sind und $R^9$ Chlor bedeutet, mit Thioharnstoff zur Erzielung des entsprechenden Zwischenproduktes mit einer [(Aminoiminomethyl)-amino]thiogruppe und Acetylieren dieses letzteren Zwischenproduktes zur Erzielung der entsprechenden Verbindung der Formel (I), worin $R^1$ und $R^2$ wie hier definiert sind und $R^3$ eine Gruppe der Formel Alk-$R^4$ ist, wobei Alk wie hier definiert ist und $R^4$ Acetylthio bedeutet;

(l) Umsetzen der Verbindung der Formel (I), worin $R^1$ und $R^2$ wie hier definiert sind und $R^3$ Wasserstoff ist, mit einem Epoxid der Formel

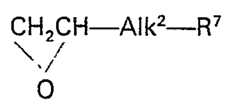

worin $Alk^2$ zweiwertiges nied.Alkyl mit 1 bis 5 C-Atomen ist und $R^7$ wie hier definiert ist, zur Erzielung der entsprechenden Verbindung der Formel (I), worin $R^1$ und $R^2$ wie hier definiert sind, und $R^3$ eine Gruppe der Formel

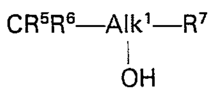

bedeutet, wobei $R^5$ und $R^6$ Wasserstoff darstellen;

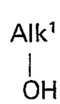

die Bedeutung $CH(OH)Alk^2$ hat, wobei $Alk^2$ wie hier definiert ist und $R^7$ wie hier definiert ist;

m) Umsetzen der Verbindung der Formel (I), worin $R^1$ und $R^2$ wie hier definiert sind und $R^3$ Wasserstoff ist, in Anwesenheit eines Protonenakzeptors mit einem Chlorepoxid der Formel

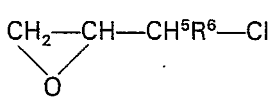 oder 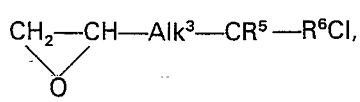

wobei $Alk^3$ zweiwertiges nied.Alkyl mit 1 bis 4 C-Atomen ist und $R^5$ und $R^6$ wie hier definiert sind, zur Erzielung der entsprechenden Verbindung der Formel (I), worin $R^1$ und $R^2$ wie hier definiert sind und $R^3$ eine Gruppe der Formel

$$\begin{array}{c} CR^5R^6—Alk^1—R^7 \\ \quad | \\ \quad OH \end{array}$$

bedeutet, wobei $R^5$ und $R^6$ wie hier definiert sind; $R^7$ Hydroxy darstellt; und

$$\begin{array}{c} Alk^1 \\ | \\ OH \end{array} \text{ die Bedeutung } \begin{array}{c} CH—CH_2— \\ | \\ OH \end{array} \text{ oder } \begin{array}{c} AlK^3—CH—CH_2— \\ | \\ OH \end{array}$$

hat, wobei $Alk^3$ wie hier definiert ist,

n) Reduzieren eines Ketons der Formel

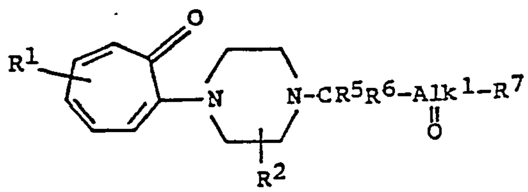

(V)

worin $R^1$, $R^2$, $R^5$, $R^6$ und $R^7$ wie hier definiert sind und

$$\begin{array}{c} —Alk^1— \\ \| \\ O \end{array}$$

ein zweiwertiges Alkanon mit 1 bis 6 C-Atomen ist, mit der Maßgabe, daß, wenn $R^7$ Hydroxy ist, die Hydroxy- und Ketongruppen an verschiedene C-Atome gebunden sind, zur Erzielung der entsprechenden Verbindung der Formel (I), worin $R^1$ und $R^2$ wie hier definiert sind und $R^3$ eine Gruppe der Formel

$$\begin{array}{c} CR^5R^6—Alk^1—R^7 \\ \quad | \\ \quad OH \end{array}$$

bedeutet, wobei $R^5$, $R^6$, $R^7$ und $Alk^1$ wie hier definiert sind;

o) Umsetzen einer Verbindung der Formel (I), worin $R^1$ und $R^2$ wie hier definiert sind und $R^3$ Wasserstoff bedeutet, mit Äthylenoxid zur Erzielung einer Verbindung der Formel (I), worin $R^1$ und $R^2$ wie hier definiert sind und $R^3$ $—CH_2CH_2OH$ bedeutet; und

p) Umsetzen der Verbindung der Formel (I), worin $R^1$, $R^2$ und $R^3$ wie hier definiert sind, mit einer therapeutisch annehmbaren Säure zur Erzielung der entsprechenden Verbindung der Formel (I), worin $R^1$, $R^2$ und $R^3$ wie hier definiert sind, als therapeutisch annehmbares Säureadditionssalz.

2. Verfahren wie in Anspruch 1 beansprucht zur Herstellung einer Verbindung der Formel (I), worin $R^1$ einen Substituenten in Stellung 7 des 2,4,6-Cycloheptatrien-1-on-Ringes bedeutet und ausgewählt ist unter Wasserstoff, Halogen, nied.Alkyl, nied.Alkoxy, Trifluormethyl oder 1-Oxo(nied.)alkylamino; $R^2$ Wasserstoff oder nied.Alkyl mit 1 bis 3 C-Atomen darstellt, und $R^3$ Wasserstoff, Phenyl, nied.Alkyl, nied.Alkenyl, nied.Alkinyl, Cyclo(nied.)alkyl, nied.Alkoxycarbonyl, 1-Oxo(nied.)alkyl, Cyclo(nied.)alkyl substituiert mit nied.Alkyl; nied.Alkenyl substituiert mit Phenyl; Phenyl mono-, di- oder trisubstituiert mit nied.Alkyl, Halogen, nied.Alkoxy, Hydroxy oder Trifluormethyl; 2-(4,5-Dihydro-2-oxazolyl)benzoyl, 2,3-Dihydro-8-oxo-cyclohepta[b]furan-2-ylmethyl, 1-Oxo-2,4,6-cycloheptatrien-2-yl, 2-(Acetylamino)-äthyl, 2,3-Diacetyloxypropyl, 2-(1,3-Dihydro-1,3-dioxo-2H-isoindol-2-yl)-äthyl, 5-Methyl-2,4-imidazolidindion-5-ylmethyl, eine Gruppe der Formel Alk-$R^4$, wobei Alk zweiwertiges Alkyl mit 1 bis 6 C-Atomen bedeutet und $R^4$ nied.Alkoxy, Cyano, Aminocarbonyl, nied.Alkoxycarbonyl, Cyclo(nied.)alkyl, Phenyl, Phenoxy, Hydroxy(nied.)alkoxy, 3-Indolyl, 1-Oxo-2,4,6-cycloheptatrien-2-yl-amino, 1H-Imidazol-4-yl, 1-Oxo(nied.)alkoxy, Acetylthio, Phenyl mono-, di- oder trisubstituiert mit nied.Alkoxy oder Hydroxy, oder Phenoxy mono-, di- oder trisubstituiert mit nied.Alkyl, Halogen oder Acetylamino, oder eine Gruppe der Formel

$$\begin{array}{c} CR^5R^6—Alk^1—R^7 \\ \quad | \\ \quad OH \end{array}$$

wobei $Alk^1$ dreiwertiges $C_1$—$C_6$-Alkylen ist, darstellt; $R^5$ und $R^6$ jeweils Wasserstoff oder nied.Alkyl mit 1 bis 3 C-Atomen sind; und $R^7$ Wasserstoff, Phenyl, Hydroxy, Phenoxy, Phenyl mono-, di- oder trisubstituiert mit Methylsulfonylamino, nied.Alkoxy oder Hydroxy oder Phenoxy mono-, di- oder trisubstituiert mit nied.Alkyl oder Halogen, ist; mit der Maßgabe, daß, wenn $R^7$ Hydroxy ist, die Hydroxygruppen an verschiedene C-Atome gebunden sind, oder eines therapeutisch annehmbaren Säureadditionssalzes hievon.

3. Verfahren wie in Anspruch 1 beansprucht zur Herstellung einer Vebindung der Formel (I), worin $R^1$ einen Substituenten in Stellung 7 des 2,4,6-Cycloheptatrien-1-on-Ringes bedeutet und ausgewählt ist unter Wasserstoff oder Brom; $R^2$ Wasserstoff oder Methyl darstellt; und $R^3$ Wasserstoff, Phenyl, nied.Alkyl, nied.Alkenyl, nied.Alkinyl, Cyclo(nied.)alkyl, nied.Alkoxycarbonyl, 1-Oxo(nied.)alkyl, Cyclo(nied.)alkyl substituiert mit nied.Alkyl, Phenyl monosubstituiert mit nied.Alkyl, Halogen oder Trifluormethyl, 2-(4,5-Dihydro-2-oxazolyl)-benzoyl, 1-Oxo-2,4,6-cycloheptatrien-2-yl, 2-(Acetylamino)-äthyl, 2,3-Diacetyloxypropyl, 2-(1,3-Dihydro-1,3-dioxo-2H-isoindol-2-yl)-äthyl, 5-Methyl-2,4-imidazolidindion-5-ylmethyl, eine Gruppe der Formel Alk-$R^4$, wobei Alk zweiwertiges Alkyl mit 1 bis 3 C-Atomen ist und $R^4$ nied.Alkoxy, Cyano, Aminocarbonyl, nied.Alkoxycarbonyl, Cyclo(nied.)alkyl, Phenyl, Phenoxy, Hydroxy(nied.)alkoxy, 3-Indolyl, 1-Oxo-2,4,6-Cycloheptatrien-2-yl-amino, 1H-Imidazol-4-yl, 1-Oxo(nied.)alkoxy, Acetylthio, Phenyl mono- oder disubstituiert mit nied.Alkoxy oder Hydroxy, oder Phenoxy monosubstituiert mit nied.Alkyl, Halogen oder Acetylamino bedeutet; oder eine Gruppe der Formel

$$\begin{array}{c} CR^5R^6—\underset{\displaystyle |\atop\displaystyle OH}{Alk^1}—R^7 \end{array}$$

ist, wobei $Alk^1$ dreiwertiges Alkylen mit 1 bis 3 C-Atomen bedeutet, $R^5$ und $R^6$ jeweils Wasserstoff oder nied.Alkyl mit 1 bis 3 C-Atomen darstellen; $R^7$ Wasserstoff, Phenyl, Hydroxy, Phenoxy, Phenyl mono-, di- oder trisubstituiert mit nied.Alkoxy oder Hydroxy, oder Phenoxy monosubstituiert mit nied.Alkyl oder Halogen bedeutet; mit der Maßgabe, daß, wenn $R^7$ Hydroxy ist, die Hydroxygruppen an verschiedene C-Atome gebunden sind; oder eines therapeutisch annehmbaren Säureadditionssalz hievon.

4. Verfahren wie in Anspruch 1 beansprucht zur Herstellung einer Verbindung der Formel (I), worin $R^1$ und $R^2$ Wasserstoff bedeuten; und $R^3$ Wasserstoff, nied.Alkyl, nied.Alkenyl, nied.Alkoxycarbonyl, eine Gruppe der Formel Alk-$R^4$ ist, wobei Alk zweiwertiges Alkyl mit 1 oder 2 C-Atomen und $R^4$ nied.Alkoxy, Cyano, 1-Oxo-(nied.)alkoxy, oder Phenyl mono- oder disubstituiert mit nied.Alkoxy oder Hydroxy, oder eine Gruppe der Formel

$$\begin{array}{c} CR^5R^6—\underset{\displaystyle |\atop\displaystyle OH}{Alk^1}—R^7 \end{array}$$

ist, wobei $Alk^1$ CH bedeutet; $R^5$ Wasserstoff darstellt; $R^6$ Wasserstoff oder nied.Alkyl mit 1 bis 3 C-Atomen ist und $R^7$ Wasserstoff Phenyl oder Phenyl mono-, di- oder trisubstituiert in Stellung 3, 4 oder 5 mit nied.Alkoxy oder Hydroxy bedeutet; oder eines therapeutisch annehmbaren Säureadditionssalzes hievon.

5. Verfahren wie in Anspruch 1 beansprucht, worin die Verbindung der Formel (I), die hergestellt wird, ausgewählt ist aus der Gruppe (—)-2-[4-[2-Hydroxy-2-(3,4-dimethoxyphenyl)äthyl]-1-piperazinyl]-2,4,6-cycloheptatrien-1-on (mit $[\alpha]_D^{25}$ —12,0° (c = 1, Dimethylformamid)], (—)-2-[4-[2-Hydroxy-1-methyl-2-(3,4-dimethoxyphenyl)-äthyl]-1-piperazinyl]-2,4,6-cycloheptatrien-1-on mit $[\alpha]_D^{25}$ —82,5° (c = 1, Dimethylformamid)], 2-[4-[2-Hydroxy-2-(3,4-dimethoxyphenyl)-äthyl-1-piperazinyl]-2,4,6-cycloheptatrien-1-on, 2-[4-[2-Hydroxy-1-methyl-2-(3,4-dimethoxyphenyl)-äthyl-1-piperazinyl]-2,4,6-cycloheptatrien-1-on, 2-[4-[2-Hydroxy-1-methyl-2-(3,4-dimethoxyphenyl)-äthyl]-1-piperazinyl]-2,4,6-cycloheptatrien-1-on (dessen Maleatsalz einen Fp. von 165—168°C aufweist), 4-(2-Oxo-3,5,7-cycloheptatrien-1-yl)-1-piperazin-äthanol, (+)-2-[4-[2-Hydroxy-2-(3,4-dimethoxyphenyl)-äthyl-1-piperazinyl]-2,4,6-cycloheptatrien-1-on (mit $[\alpha]_D^{25}$ +11,7° (c = 1, Dimethylformamid)), 2-[4-(2-Hydroxypropyl)-1-piperazinyl]-2,4,6-cycloheptatrien-1-on, (+)-2-[4-[2-Hydroxy-1-methyl-2-(3,4-dimethoxyphenyl)-äthyl]-1-piperazinyl]-2,4,6-cycloheptatrien-1-on [mit $[\alpha]_D^{25}$ +84,2° (c = 1, Dimethylformamid)] und 2-[4-[2-Hydroxy-2-(4-methoxyphenyl)-äthyl]-1-piperazinyl-2,4,6-cycloheptatrien-1-on oder eines therapeutisch annehmbaren Säureadditionssalzes hievon.

6. Verfahren wie in Anspruch 1 beansprucht, worin die Verbindung der Formel (I), die hergestellt wird, 7-Brom-2-[4-(2-hydroxyäthyl)-1-piperazinyl]-2,4,6-cycloheptatrien-1-on oder ein therapeutisch annehmbares Säureadditionssalz hievon ist.

7. Verfahren wie in Anspruch 1 beansprucht, worin die Verbindung der Formel (I), die hergestellt wird, ausgewählt ist aus der Gruppe 4-(2-Oxo-3,5,7-cycloheptatrien-1-yl)-1-piperazin-acetonitril, 2-[4-[2-(3,4-Dimethoxyphenyl)-äthyl]-1-piperazinyl]-2,4,6-cycloheptatrien-1-on, 2-[4-[2-(Acetyloxy)-äthyl-1-piperazinyl]-2,4,6-cycloheptatrien-1-on, 2,2-Dimethylpropansäure, 2-[4-(2-Oxo-3,5,7-Cycloheptatrien-1-yl)-1-piperazinyl]-äthylester oder ein therapeutisch annehmbares Säureadditionssalz hievon.

8. Verfahren wie in Anspruch 1 beansprucht, worin die Verbindung der Formel (I), die hergestellt wird, ausgewählt ist aus der Gruppe 2-(4-Methyl-1-piperazinyl)-2,4,6-cycloheptatrien-1-on, 2-[4-(2-Propenyl)-1-piperazinyl]-2,4,6-cycloheptatrien-1-on, 2-[4-(1-Methyläthyl)-1-piperazinyl]-2,4,6-cycloheptatrien-1-on, 2-(4-Propyl-1-piperazinyl)-2,4,6-cycloheptatrien-1-on und 2-[4-(1-Methylpropyl)-1-piperazinyl]-2,4,6-cycloheptatrien-1-on oder ein therapeutisch annehmbares Säureadditionssalz hievon.

9. Verfahren wie in Anspruch 1 beansprucht zur Herstellung einer Verbindung der Formel (I), worin $R^1$ und $R^2$ wie hier definiert sind und $R^3$ eine Gruppe der Formel

$$CR^5R^6—\underset{\displaystyle OH}{\underset{|}{Alk^1}}—R^7$$

ist, wobei $R^5$, $R^6$, $R^7$ und $Alk^1$ wie hier definiert sind, welches das Reduzieren des entsprechenden Ketons der Formel (V), worin $R^1$, $R^2$, $R^5$, $R^6$, $R^7$ und

$$\underset{\displaystyle O}{\underset{\|}{Alk^1}}$$

wie hier definiert sind, mit Natriumborhydrid umfaßt.

10. Verfahren gemäß Anspruch 9 zur Herstellung einer Verbindung der Formel (I), worin $R^1$ und $R^2$ Wasserstoff sind und $R^3$ eine Gruppe der Formel

$$CR^5R^6—\underset{\displaystyle OH}{\underset{|}{Alk^1}}—R^7$$

bedeutet, wobei $Alk^1$ CH ist, $R^5$ Wasserstoff bedeutet, $R^6$ Wasserstoff oder nied.Alkyl mit 1 bis 3 C-Atomen darstellt und $R^7$ Phenyl oder Phenyl mono-, di- oder trisubstituiert in den Stellungen 3, 4 oder 5 mit nied.Alkoxy oder Hydroxy ist.

11. Verfahren gemäß Anspruch 1 zur Herstellung einer Verbindung der Formel (I), worin $R^1$ und $R^2$ Wasserstoff bedeuten und $R^3$ eine Gruppe der Formel

$$CR^5R^6—\underset{\displaystyle OH}{\underset{|}{AlK^1}}—R^7$$

ist, wobei $Alk^1$ CH darstellt, $R^5$ Wasserstoff ist, $R^6$ Wasserstoff oder nied.Alkyl mit 1 bis 3 C-Atomen bedeutet und $R^7$ Phenyl oder Phenyl mono-, di- oder trisubstituiert in den Stellungen 3, 4 oder 5 mit nied.Alkoxy oder Hydroxy ist, welches das Kondensieren einer Verbindung der Formel (II), worin $R^1$ wie hier definiert ist und $R^8$ Methyl oder Äthyl bedeutet, mit einer Verbindung der Formel (III), worin $R^2$ und $R^3$ wie hier definiert sind, umfaßt.

12. Verfahren, worin eine Verbindung der Formel (I), wie in Anspruch 9, 10 oder 11 definiert, getrennt wird, um die getrennten optischen Isomeren zu erhalten.

13. Verfahren zur Herstellung einer Verbindung der Formel (V), wie in Anspruch 1 definiert, welches das Umsetzen einer entsprechenden Verbindung der Formel (I), worin $R^1$ und $R^2$ wie in Anspruch 1 definiert sind und $R^3$ Wasserstoff bedeutet, mit einem Alkanon der Formel

$$X—CR^5R^6—\underset{\displaystyle O}{\underset{\|}{Alk^1}}—R^7,\quad \text{wobei } R^5,\ R^6,\ R^7 \text{ und } \underset{\displaystyle O}{\underset{\|}{Alk^1}}$$

wie in Anspruch 1 definiert sind und X Brom, Chlor oder Jod darstellt, in Anwesenheit eines Protonenakzeptors umfaßt.

14. Verfahren zur Herstellung einer pharmazeutischen Zusammensetzung, welches das Mischen einer Verbindung der Formel (I), wie in Anspruch 1 definiert, oder eines therapeutisch annehmbaren Säureadditionssalzes hievon mit einem pharmazeutisch annehmbaren Träger umfaßt.

**Revendications pour les Etats contractants: BE CH DE FR GB IT LI LU NL SE**

1. Composé de formule I:

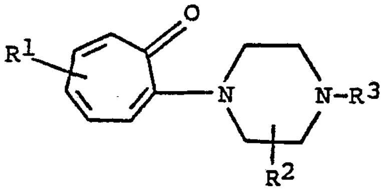

(I)

où $R^1$ représente un substituant aux positions 3, 4, 5, 6 ou 7 du cycle de 2,4,6-cycloheptatrién-1-one et est choisi parmi hydrogène, halo, alcoyle inférieur, alcoxy inférieur, trifluorométhyle et 1-oxoalcoylamino inférieur; $R^2$ représente hydrogène ou alcoyle inférieur comptant 1 à 3 atomes de carbone et $R^3$ représente hydrogène, phényle, alcoyle inférieur, alcényle inférieur, alcynyle inférieur, cycloalcoyle inférieur, alcoxycarbonyl inférieur, 1-oxoalcoyle inférieur, cycloalcoyle inférieur substitué par un alcoyle inférieur, alcényle inférieur substitué par phényle, phényle mono- di- ou trisubstitué par alcoyle inférieur, halo, alcoxy inférieur, hydroxyle ou trifluorométhyle; 2-(4,5-dihydro-2-oxazolyl)benzoyle, 2,3-dihydro-8-oxocyclohepta[b]furan-2-ylméthyl, 1-oxo-2,4,6-cycloheptatrién-2-yle, 2-(acétylamino)-éthyle, 2,3-diacétyloxypropyle, 2-(1,3-dihydro-1,3-dioxo-2H-isoindol-2-yl)-éthyle, 5-méthyl-2,4-imidazolidinedione-5-ylméthyle, un radical de formule Alk-$R^4$, où Alk est un alcoyle divalent comptant 1 à 6 atomes de carbone et $R^4$ est alcoxy inférieur, cyano, aminocarbonyle, alcoxycarbonyle inférieur, cycloalcoyle inférieur, phényle, phénoxy, hydroxyalcoxy inférieur, 3-indolyle, 1-oxo-2,4,6-cycloheptatrién-2-ylamino, 1H-imidazol-4-yle, 1-oxoalcoxy inférieur, acétylthio, phényle mono-, di- ou trisubstitué par alcoyle inférieur, halo, alcoxy inférieur, hydroxyle ou trifluorométhyle, ou phénoxy mono-, di- ou trisubstitué par alcoyle inférieur, halo, alcoxy inférieur, acétylamino, hydroxyle ou trifluorométhyle, ou un radical de formule $CR^5R^6$—$Alk^1(OH)R^7$, où $Alk^1$ représente un alcoylène trivalent comptant 1 à 6 atomes de carbone; $R^5$ et $R^6$ représentent chacun hydrogène ou alcoyle inférieur comptant 1 à 3 atomes de carbone; et $R^7$ représente hydrogène, phényle, hydroxyle, phénoxy, phényle mono-, di- ou trisubstitué par méthylsulfonylamino, alcoyle inférieur, halo, alcoxy inférieur, hydroxyle ou trifluorométhyle, ou phénoxy mono-, di- ou trisubstitué par alcoyle inférieur, halo, alcoxy inférieur, hydroxyle ou trifluorométhyle; avec la restriction que lorsque $R^7$ représente hydroxyle, les radicaux hydroxyle sont portés par des atomes de carbone différents, ou un sel d'addition d'acide thérapeutiquement acceptable de ce composé; étant entendu que le terme "alcoyle inférieur", sauf indication contraire, désigne un radical alcoyle en chaîne droite comptant 1 à 6 atomes de carbone ou un radical alcoyle en chaîne ramifiée comptant 3 ou 4 atomes de carbone; que le terme "alcoxy inférieur" désigne un radical alcoxy en chaîne droite comptant 1 à 6 atomes de carbone ou un radical alcoxy en chaîne ramifiée comptant 3 ou 4 atomes de carbone; que le terme "1-oxoalcoyle inférieur" désigne un radical 1-oxoalcoyle en chaîne droite comptant 2 à 6 atomes de carbone ou un radical 1-oxoalcoyle en chaîne ramifiée comptant 4 à 6 atomes de carbone; que le terme "alcényle inférieur" désigne un radical alcényle en chaîne droite comptant 2 à 6 atomes de carbone ou un radical alcényle en chaîne ramifiée comptant 3 ou 4 atomes de carbone; que le terme "alcynyle inférieur" désigne un radical alcynyle en chaîne droite comptant 2 à 6 atomes de carbone ou un radical alcynyle en chaîne ramifiée comptant 4 atomes de carbone; que le terme "1-oxoalcoxy inférieur" désigne un radical 1-oxoalcoxy en chaîne droite comptant 2 à 6 atomes de carbone ou un radical 1-oxoalcoxy en chaîne ramifiée comptant 4 à 6 atomes de carbone et que le terme "cycloalcoyle inférieur" désigne un radical hydrocarboné cyclique saturé comptant 3 à 6 atomes de carbone.

2. Composé suivant la revendication 1, où $R^1$ représente un substituant en position 7 du cycle de 2,4,6-cycloheptatrién-1-one et est choisi entre hydrogène, halo, alcoyle inférieur, alcoxy inférieur, trifluorométhyle et 1-oxoalcoylamino inférieur; $R^2$ représente hydrogène ou alcoyle inférieur comptant 1 à 3 atomes de carbone; et $R^3$ représente hydrogène, phényle, alcoyle inférieur, alcényle inférieur, alcynyle inférieur, cycloalcoyle inférieur, alcoxycarbonyle inférieur, 1-oxoalcoyle inférieur, cycloalcoyle inférieur substitué par un alcoyle inférieur; alcényle inférieur substitué par un phényle; phényle mono-, di- ou trisubstitué par alcoyle inférieur, halo, alcoxy inférieur, hydroxyle ou trifluorométhyle; 2-(4,5-dihydro-2-oxazolyl)benzoyle, 2,3-dihydro-8-oxocyclohepta[b]furan-2-ylméthyle, 1-oxo-2,4,6-cycloheptatrién-2-yle, 2-(acétylamino)éthyle, 2,3-diacétyloxypropyle, 2-(1,3-dihydro-1,3-dioxo-2H-isoindol-2-yl)éthyle, 5-méthyl-2,4-imidazolidinedione-5-ylméthyle, un radical de formule Alk-$R^4$, où Alk représente un alcoyle divalent comptant 1 à 6 atomes de carbone et $R^4$ représente alcoxy inférieur, cyano, aminocarbonyle, alcoxycarbonyle inférieur, cycloalcoyle inférieur, phényle, phénoxy, hydroxyalcoxy inférieur, 3-indolyle, 1-oxo-2,4,6-cycloheptatrién-2-ylamino, 1H-imidazol-4-yle, 1-oxoalcoxy inférieur, acétylthio, phényle mono-, di- ou trisubstitué par alcoxy inférieur ou hydroxyle ou phénoxy mono-, di- ou trisubstitué par alcoyle inférieur, halo ou acétylamino, ou un radical de formule $CR^5R^6Alk^1(OH)R^7$, où $Alk^1$ représente un alcoylène trivalent de 1 à 6 atomes de carbone, $R^5$ et $R^6$ représentent chacun hydrogène ou alcoyle inférieur comptant 1 à 3 atomes de carbone; et $R^7$ représente hydrogène, phényle, hydroxyle, phénoxy, phényle mono-, di- ou trisubstitué par

méthylsulfonylamino, alcoxy inférieur ou hydroxyle, ou phénoxy mono-, di ou trisubstitué par alcoyle inférieur ou halo; avec la restriction que lorsque $R^7$ représente hydroxyle, les deux radicaux hydroxyle sont portes par des atomes de carbone différents; ou un sel d'addition d'acide thérapeutiquement acceptable de ce composé.

3. Composé suivant la revendication 1, où $R^1$ représente un substituant en position 7 du cycle de 2,4,6-cycloheptatrién-1-one et est choisi entre hydrogène et bromo; $R^2$ représente hydrogène ou méthyle, et $R^3$ représente hydrogène, phényle, alcoyle inférieur, alcényle inférieur, alcynyle inférieur, cycloalcoyle inférieur, alcoxycarbonyle inférieur, 1-oxoalcoyle inférieur, cycloalcoyle inférieur substitué par un alcoyle inférieur, phényle monosubstitué par alcoyle inférieur, halo ou trifluorométhyle, 2-(4,5-dihydro-2-oxazolyl)benzoyle, 1-oxo-2,4,6-cycloheptatrién-2-yle, 2-(acétylamino)éthyl, 2,3-diacétyloxypropyle, 2-(1,3-dihydro-1,3-dioxo-2H-isoindol-2-yl)éthyle, 5-méthyl-2,4-imidazolidinedione-5-yl-méthyle, un radical de formule Alk-$R^4$, où Alk représente un alcoyle divalent comptant 1 à 3 atomes de carbone et $R^4$ représente alcoxy inférieur, cyano, aminocarbonyle, alcoxycarbonyle inférieur, cycloalcoyle inférieur, phényle, phénoxy, hydroxyalcoxy inférieur, 3-indolyle, 1-oxo-2,4,6-cycloheptatrién-2-ylamino, 1H-imidazol-4-yle, 1-oxoalcoxy inférieur, acétylthio, phényle mono- ou disubstitué par alcoxy inférieur ou hydroxyle, ou phénoxy monosubstitué par alcoyle inférieur, halo ou acétylamino; ou un radical de formule $CR^5R^6$-$Alk^1(OH)R^7$, où $Alk^1$ représente un alcoylène trivalent comptant 1 à 3 atomes de carbone; $R^5$ et $R^6$ représentent chacun hydrogène ou alcoyle inférieur comptant 1 à 3 atomes de carbone; $R^7$ représente hydrogène, phényle, hydroxyle, phénoxy, phényle mono- di- ou trisubstitué par alcoxy inférieur ou hydroxyle ou phénoxy monosubstitué par alcoyle inférieur ou halo; avec la restriction que lorsque $R^7$ représente hydroxyle, les radicaux hydroxyle sont portés par des atomes de carbone différents, ou un sel d'addition d'acide thérapeutiquement acceptable de ce composé.

4. Composé suivant la revendication 1, où $R^1$ et $R^2$ représentent hydrogène; et $R^3$ représente hydrogène, alcoyle inférieur, alcényle inférieur, alcoxycarbonyl inférieur, un radical de formule Alk-$R^4$, où Alk représente un alcoyle divalent comptant 1 ou 2 atomes de carbone et $R^4$ représente alcoxy inférieur, cyano, 1-oxoalcoxy inférieur ou phényle mono- ou disubstitué par alcoxy inférieur ou hydroxyle, ou un radical de formule $CR^5R^6Alk^1(OH)R^7$, où $Alk^1$ représente CH; $R^5$ représente hydrogène, $R^6$ représente hydrogène ou alcoyle inférieur comptant 1 à 3 atomes de carbone; et $R^7$ représente hydrogène, phényle ou phényle mono-, di- ou trisubstitué aux positions 3, 4 ou 5 par alcoxy inférieur ou hydroxyle; ou un sel d'addition d'acide thérapeutiquement acceptable de ce composé.

5. Composé suivant la revendication 1, choisi parmi la (—)-2-[4-[2-hydroxy-2-(3,4-diméthoxy-phényl)éthyl]-1-pipérazinyl]-2,4,6-cycloheptatrién-1-one [à$[\alpha]_D^{25}$ —12,0° (c=1, diméthylformamide)], la (—) - 2 -[4-[2-hydroxy-1-méthyl-2-(3,4-diméthoxyphényl)éthyl]-1-pipérazinyl]-2,4,6-cycloheptatrién-1-one, [à$[\alpha]_D^{25}$ —82,5° (c=1, diméthylformamide)], la 2-[4-[2-hydroxy-2-(3,4-diméthoxyphényl)éthyl-1-pipérazinyl]-2,4,6-cycloheptatrién-1-one, la 2-[4-[2-hydroxy-1-méthyl-2-(3,4-diméthoxyphényl)éthyl-1-pipérazinyl]-2,4,6-cycloheptatrién-1-one, la 2-[4-[2-hydroxy-1-méthyl-2-(3,4-diméthoxyphényl)-éthyl]-1-pipérazinyl]-2,4,6-cycloheptatrién-1-one, [dont le maléate a un P.F. de 165—168°C], le 4-(2-oxo-3,5,7-cycloheptatrién-1-yl)-1-pipérazine-éthanol, la (+)-2-[4-[2-hydroxy-2-(3,4-diméthoxy-phényl)éthyl-1-pipérazinyl]-2,4,6-cycloheptatrién-1-one [à $[\alpha]_D^{25}$ +11,7° (c=1, diméthylformamide)]; la 2-[4-(2-hydroxypropyl)-1-pipérazinyl]-2,4,6-cycloheptatrién-1-one, la (+)-2-[4-[2-hydroxy-1-méthyl-2-(3,4-diméthoxyphényl)éthyl]-1-pipérazinyl]-2,4,6-cycloheptatrién-1-one, [à$[\alpha]_D^{25}$ +84,2° (c=1, diméthylformamide)] et la 2-[4-[2-hydroxy-2-(4-méthoxyphényl)éthyl]-1-pipérazinyl-2,4,6-cyclo-heptatrién-1-one, ou un sel d'addition d'acide thérapeutiquement acceptable de ce composé.

6. Composé suivant la revendication 1, qui est la 7-bromo-2-[4-(2-hydroxyéthyl)-1-pipérazinyl]-2,4,6-cycloheptatrién-1-one, ou un sel d'addition d'acide thérapeutiquement acceptable de ce composé.

7. Composé suivant la revendication 1, qui est choisi parmi le 4-(2-oxo-3,5,7-cycloheptatrién-1-yl)-1-pipérazine-acétonitrile, la 2-[4-[2-(3,4-diméthoxyphényl)éthyl]-1-pipérazinyl]-2,4,6-cyclohepta-trién-1-one, la 2-[4-[2-(acétoxy)éthyl-1-pipérazinyl]-2,4,6-cycloheptatrién-1-one, et l'ester 2-[4-(2-oxo-3,5,7-cycloheptatrién-1-yl)-1-pipérazinyl]éthylique d'acide 2,2-diméthylpropanoïque, ou un sel d'addition d'acide pharmaceutiquement acceptable de ce composé.

8. Composé suivant la revendication 1, qui est choisi parmi la 2-(4-méthyl-1-pipérazinyl)-2,4,6-cycloheptatrién-1-one, la 2-[4-(2-propényl)-1-pipérazinyl]-2,4,6-cycloheptatrién-1-one, la 2-[4(1-méthyléthyl)-1-pipérazinyl]-2,4,6-cycloheptatrién-1-one, la 2-(4-propyl-1-pipérazinyl)-2,4,6-cyclo-heptatrièn-1-one et la 2-[4-(1-méthylpropyl)-1-pipérazinyl]-2,4,6-cycloheptatrién-1-one ou un sel d'addition d'acide thérapeutiquement acceptable de ce composé.

9. Composé de formule:

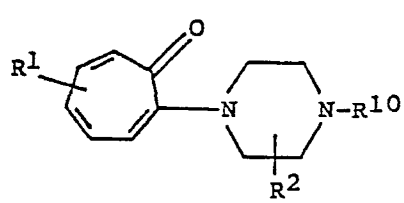

où $R^1$ représente un substituant aux positions 3, 4, 5, 6 ou 7 du cycle de 2,4,6-cycloheptatrién-1-one et est choisi parmi hydrogène, halo, alcoyle inférieur, alcoxy inférieur, trifluorométhyle et 1-oxoalcoylamino inférieur; $R^2$ représente hydrogène ou alcoyle inférieur comptant 1 à 3 atomes de carbone; $R^{10}$ représente 2-aminoéthyle, -Alk-Cl (où Alk représente un radical alcoyle divalent comptant 1 à 6 atomes de carbone) ou un radical de formule $CR^6R^6$-$Alk^1(O)R^7$, où $Alk^1$=O représente une alcanone divalente comptant 1 à 6 atomes de carbone; $R^5$ et $R^6$ représentent chacun hydrogène ou alcoyle inférieur comptant 1 à 3 atomes de carbone et $R^7$ représente hydrogène, phényle, hydroxyle, phénoxy, phényle mono-, di- ou trisubstitué par méthylsulfonylamino, alcoyle inférieur, halo, alcoxy inférieur, hydroxyle ou trifluorométhyle, ou phénoxy mono-, di- ou trisubstitué par alcoyle inférieur, halo, alcoxy inférieur, hydroxyle ou trifluorométhyle; avec la restriction que lorsque $R^7$ représente hydroxyle, les radicaux hydroxyle et cétone sont portés par des atomes de carbone différents, étant entendu que le terme "alcoyle inférieur", sauf indication contraire, désigne un radical alcoyle en chaîne droite comptant 1 à 6 atomes de carbone ou un radical alcoyle en chaîne ramifiée comptant 3 ou 4 atomes de carbone; que le terme "alcoxy inférieur" désigne un radical alcoxy en chaîne droite comptant 1 à 6 atomes de carbone ou un radical alcoxy en chaîne ramifiée comptant 3 ou 4 atomes de carbone; et que le terme "1-oxoalcoyle inférieur" désigne un radical 1-oxoalcoyle en chaîne droite comptant 2 à 6 atomes de carbone ou un radical 1-oxoalcoyle en chaîne ramifiée comptant 4 à 6 atomes de carbone.

10. Composé suivant la revendication 9, où $R^1$ et $R^2$ représentent hydrogène et $R^{10}$ représente $CR^5R^6$—$Alk^1(O)R^7$, où $R^5$ et $R^6$ représentent chacun hydrogène ou alcoyle inférieur comptant 1 à 3 atomes de carbone; $R^7$ représente hydrogène, phényle ou phényle mono-, di- ou trisubstitué par halo, alcoxy inférieur ou hydroxyle et $Alk^1$=O représente une alcanone divalente comptant 1 à 3 atomes de carbone.

11. Composition pharmaceutique qui comprend un composé de formule 1, ou un sel d'addition d'acide thérapeutiquement acceptable de ce composé, suivant l'une quelconque des revendications 1 à 8, outre un excipient pharmaceutiquement acceptable.

12. Composé de formule I, ou un sel thérapeutiquement acceptable de ce composé, suivant l'une quelconque des revendications 1 à 8 à utiliser comme agent de stimulation des récepteurs de la dopamine.

**Revendications pour l'Etat contractant: AT**

1. Procédé de préparation d'un composé de formule:

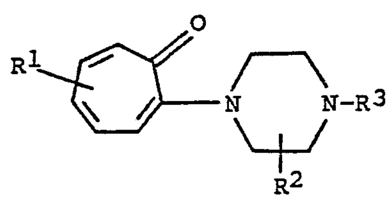

(I)

où $R^1$ représente un substituant aux positions 3, 4, 5, 6 ou 7 du cycle de 2,4,6-cycloheptatrién-1-one et est choisi parmi hydrogène, halo, alcoyle inférieur, alcoxy inférieur, trifluorométhyle et 1-oxoalcoylamino inférieur; $R^2$ représente hydrogène ou alcoyle inférieur comptant 1 à 3 atomes de carbone; et $R^3$ représente hydrogène, phényle, alcoyle inférieur, alcényle inférieur, alcynyle inférieur, cycloalcoyle inférieur, alcoxycarbonyl inférieur, 1-oxoalcoyle inférieur, cycloalcoyle inférieur substitué par un alcoyle inférieur, alcényle inférieur substitué par phényle, phényle mono- di- ou trisubstitué par alcoyle inférieur, halo, alcoxy inférieur, hydroxyle ou trifluorométhyle; 2-(4,5-dihydro-2-oxazolyl)benzoyle, 2,3-dihydro-8-oxocyclohepta[b]furan-2-ylméthyle, 1-oxo-2,4,6-cycloheptatrién-2-yle, 2-(acétylamino)-éthyle, 2,3-diacétyloxypropyle, 2-(1,3-dihydro-1,3-dioxo-2H-isoindol-2-yl)-éthyle, 5-méthyl-2,4-imidazolidinedione-5-ylméthyle, un radical Alk-$R^4$, où Alk est un alcoyle divalent comptant 1 à 6 atomes de carbone et $R^4$ est alcoxy inférieur, cyano, aminocarbonyle, alcoxycarbonyle inférieur, cycloalcoyle inférieur, phényle, phénoxy, hydroxyalcoxy inférieur, 3-indolyle, 1-oxo-2,4,6-cycloheptatrién-2-ylamino, 1H-imidazol-4-yle, 1-oxoalcoxy inférieur, acétylthio, phényle mono-, di- ou trisubstitué par alcoyle inférieur, halo, alcoxy inférieur, hydroxyle ou trifluorométhyle; ou phénoxy mono-, di- ou trisubstitué par alcoyle inférieur, halo, alcoxy inférieur, acétylamino, hydroxyle ou trifluorométhyle, ou un radical de formule $CR^5R^6$-$Alk^1(OH)R^7$, où $Alk^1$ représente un alcoylène trivalent comptant 1 à 6 atomes de carbone; $R^5$ et $R^6$ représentent chacun hydrogène ou alcoyle inférieur comptant 1 à 3 atomes de carbone; et $R^7$ représente hydrogène, phényle, hydroxyle, phénoxy, phényle mono-, di- ou trisubstitué par méthylsulfonylamino, alcoyle inférieur, halo, alcoxy inférieur, hydroxyle ou trifluorométhyle, ou phénoxy mono-, di- ou trisubstitué par alcoyle inférieur, halo, alcoxy inférieur, hydroxyle ou trifluorométhyle; avec la restriction que lorsque $R^7$ représente hydroxyle, les radicaux hydroxyle sont portes par des atomes de carbone différents, ou d'un sel d'addition d'acide thérapeutiquement acceptable de ce composé; étant entendu que le terme "alcoyle inférieur", sauf indication contraire,

désigne un radical alcoyle en chaîne droite comptant 1 à 6 atomes de carbone ou un radical alcoyle en chaîne ramifiée comptant 3 ou 4 atomes de carbone; que le terme "alcoxy inférieur" désigne un radical alcoxy en chaîne droite comptant 1 à 6 atomes de carbone ou un radical alcoxy en chaîne ramifiée comptant 3 ou 4 atomes de carbone; que le terme "1-oxoalcoyle inférieur" désigne un radical 1-oxoalcoyle en chaîne droite comptant 2 à 6 atomes de carbone ou un radical 1-oxoalcoyle en chaîne ramifiée comptant 4 à 6 atomes de carbone; que le terme "alcényle inférieur" désigne un radical alcényle en chaîne droite comptant 2 à 6 atomes de carbone ou un radical alcényle en chaîne ramifiée comptant 3 ou 4 atomes de carbone; que le terme "alcynyle inférieur" désigne un radical alcynyle en chaîne droite comptant 2 à 6 atomes de carbone ou un radical alcynyle en chaîne ramifiée comptant 4 atomes de carbone; que le terme "1-oxoalcoxy inférieur" désigne un radical 1-oxoalcoxy en chaîne droite comptant 2 à 6 atomes de carbone ou un radical 1-oxoalcoxy en chaîne ramifiée comptant 4 à 6 atomes de carbone et que le terme "cycloalcoyle inférieur" désigne un radical hydrocarboné cyclique saturé comptant 3 à 6 atomes de carbone; qui comprend le choix d'un mode opératoire entre

a) lorsqu'il faut un composé de formule I, où $R^1$, $R^2$ et $R^3$ sont tels que définis ici, on condense un composé de formule II:

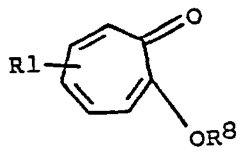

(II)

où $R^1$ est tel que défini ici et $R^8$ représente alcoyle inférieur, avec un composé de formule III:

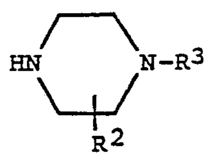

(III)

où $R^2$ est tel que défini ici et $R^3$ est tel que défini ci-dessus ou représente aminoalcoyle inférieur;

b) lorsqu'il faut un composé de formule I, où $R^1$ et $R^2$ sont tels que définis ici et $R^3$ représente alcoyle inférieur, alcényle inférieur, alcynyle inférieur, cycloalcoyle inférieur, alcoxycarbonyl inférieur, 1-oxoalcoyle inférieur, cycloalcoyle inférieur substitué par un alcoyle inférieur, alcényle inférieur substitué par phényle, 2-(4,5-dihydro-2-oxazolyl)benzoyle, 2,3-dihydro-8-oxocyclohepta[b]-furan-2-ylméthyle, 2,3-diacétyloxypropyle, 2-(1,3-dihydro-1,3-dioxo-2H-isoindol-2-yl)éthyle, un radical de formule Alk-$R^4$, où Alk et $R^4$ sont tels que définis ici, ou un radical de formule $CR^5R^6$—$Alk^1(OH)R^7$, où $R^5$, $R^6$, $R^7$ et $Alk^1$ sont tels que définis ici, on fait réagir le composé de formule I, où $R^1$ et $R^2$ sont tels que définis ici et $R^3$ représente hydrogène, avec un halogénure de formule X—$R^3$, où X représente bromo, chloro ou iodo et $R^{2'}$ représente alcoyle inférieur, alcényle inférieur, alcynyle inférieur, cycloalcoyle inférieur, alcoxycarbonyle inférieur, 1-oxoalcoyle inférieur, cycloalcoyle inférieur substitue par un alcoyle inférieur, alcényle inférieur substitué par phényle, 2,3-dihydro-8-oxocyclohepta[b]furan-2-ylméthyle, 2,3-diacétyloxypropyle, 2-(1,3-dihydro-1,3-dioxo-2H-isoindol-2-yl)éthyle; un halogénure de formule X—Alk—$R^4$ où $R^4$, X et Alk sont tels que définis ici; ou un halogénure de formule X—$CR^5R^6$—$Alk^1(OH)R^7$, où $R^5$, $R^6$, $R^7$, X et $Alk^1$ sont tels que définis ici en présence d'un accepteur de protons;

c) on acétyle un composé de formule I, où $R^1$ et $R^2$ sont tels que définis ici et $R^3$ représente 2,3-dihydroxypropyle, pour obtenir un composé de formule I, où $R^1$ et $R^2$ sont tels que définis ici et $R^3$ représente 2,3-diacétyloxypropyle;

d) on acyle le composé de formule I, où $R^1$ et $R^2$ sont tels que définis ici et $R^3$ représente hydrogène, pour obtenir le composé correspondant de formule I, où $R^1$ et $R^2$ sont tels que définis ici et $R^3$ représente 1-oxoalcoyle inférieur;

e) on fait réagir un composé de formule I, où $R^1$ et $R^2$ sont tels que définis ici et $R^2$ représente un radical 2-oxopropyle, avec du cyanure de potassium et du carbonate d'ammonium, pour obtenir un composé de formule I, où $R^1$ et $R^2$ sont tels que définis ici et $R^3$ représente 5-méthyl-2,4-imidazolidinedione-5-ylméthyle;

f) on acétyle un composé de formule I, où $R^1$ et $R^2$ sont tels que définis ici et $R^3$ représente un radical 2-aminoéthyle, pour obtenir un composé de formule I, où $R^1$ et $R^2$ sont tels que définis ici et $R^3$ représente 2-(acétylamino)éthyle;

g) on fait réagir le composé de formule I, où $R^1$ et $R^2$ sont tels que définis ici et $R^3$ représente hydrogène, en présence d'un accepteur de protons, avec un tosylate de formule TsO—Alk—$R^4$, où Alk est tel que défini ici et $R^4$ représente phényle, phénoxy, phényle mono-, di- ou trisubstitué par alcoyle inférieur, halo, alcoxy inférieur ou trifluorométhyle, ou phénoxy mono-, di- ou trisubstitué par alcoyle inférieur, halo, alcoxy inférieur, acétylamino ou trifluorométhyle, pour obtenir le composé correspondant

de formule I, où $R^1$ et $R^2$ sont tels que définis ici et $R^3$ représente un radical de formule Alk—$R^4$, ou Alk est tel que défini ici et $R^4$ est tel que défini immédiatement ci-dessus.

h) on fait réagir le composé de formule I, où $R^1$ et $R^2$ sont tels que définis ici et $R^3$ représente un radical de formule Alk—$R^4$, où Alk est tel que défini ici et $R^4$ représente phényle mono-, di- ou trisubstitué par alcoxy inférieur, avec le tribromure de bore, pour obtenir le composé correspondant de formule I, où $R^1$ et $R^2$ sont tels que définis ici et $R^3$ représente un radical de formule Alk—$R^4$, où Alk est tel que défini ici et $R^4$ représente phényle mono-, di- ou trisubstitué par hydroxyle;

i) on fait réagir le composé de formule I, où $R^1$ et $R^2$ sont tels que définis ici et $R^3$ représente un radical de formule Alk—$R^4$, où Alk est tel que défini ici et $R^4$ représente cyano, avec l'acide sulfurique concentré, puis avec l'hydroxyde d'ammonium concentré, pour obtenir le composé correspondant de formule I, où $R^1$ et $R^2$ sont tels que définis ici et $R^3$ représente un radical de formule Alk—$R^4$, où Alk est tel que défini ici et $R^4$ représente aminocarbonyle;

j) on acyle un composé de formule IV:

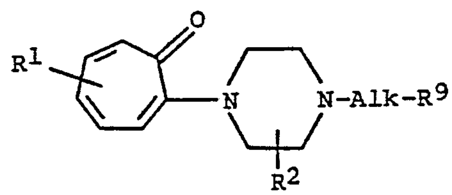

(IV)

où Alk, $R^1$ et $R^2$ sont tels que définis ici et $R^9$ représente hydroxyle, pour obtenir le composé correspondant de formule I, où $R^1$ et $R^2$ sont tels que définis ici et $R^3$ représente un radical de formule Alk—$R^4$, où Alk est tel que défini ici et $R^4$ représente 1-oxoalcoxy inférieur;

k) on fait réagir le composé de formule IV, où Alk, $R^1$ et $R^2$ sont tels que définis ici et $R^9$ représente chloro, avec la thiourée, pour obtenir l'intermédiaire correspondant portant un radical [(aminoiminométhyl)amino]thio et on acétyle ce dernier intermédiaire, pour obtenir le composé correspondant de formule I, où $R^1$ et $R^2$ sont tels que définis ici et $R^3$ représente un radical de formule Alk—$R^4$, où Alk est tel que défini ici et $R^4$ représente acétylthio;

l) on fait réagir le composé de formule I, où $R^1$ et $R^2$ sont tels que définis ici et $R^3$ représente hydrogène, avec un époxyde de formule:

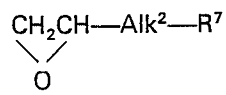

où $Alk^2$ représente un alcoyle inférieur divalent comptant 1 à 5 atomes de carbone et $R^7$ est tel que défini ici, pour obtenir le composé correspondant de formule I, où $R^1$ et $R^2$ sont tels que définis ici; et $R^3$ représente un radical de formule $CR^5R^6$—$Alk^1(OH)R^7$, où $R^5$ et $R^6$ représentent hydrogène, $Alk^1(OH)$ représente CH(OH)—$Alk^2$, où $Alk^2$ est tel que défini ici; et $R^7$ est tel que défini ici;

m) on fait réagir le composé de formule I, où $R^1$ et $R^2$ sont tels que définis ici et $R^3$ représente hydrogène, en présence d'un accepteur de protons, avec un chloroépoxyde de formule:

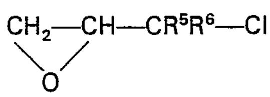 ou 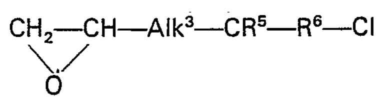

où $Alk^3$ représente un alcoyle inférieur divalent comptant 1 à 4 atomes de carbone et $R^5$ et $R^6$ sont tels que définis ici, pour obtenir le composé correspondant de formule I, où $R^1$ et $R^2$ sont tels que définis ici et $R^3$ représente un radical de formule $CR^5R^6$—$Alk^1(OH)R^7$, où $R^5$ et $R^6$ sont tels que définis ici; $R^7$ représente hydroxyle; et $Alk^1(OH)$ représente CH(OH)—$CH_2$— ou $Alk^3$—CH(OH)—$CH_2$—, où $Alk^3$ est tel que défini ici;

n) on réduit une cétone de formule V:

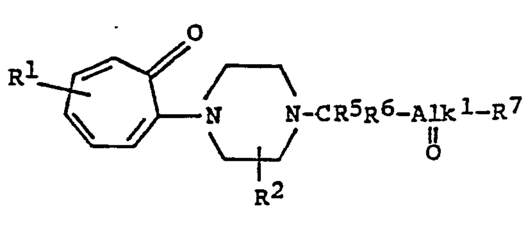

(V)

où $R^1$, $R^2$, $R^5$, $R^6$ et $R^7$ sont tels que définis ici et — $Alk^1(O)$— représente une alcanone divalente comptant de 1 à 6 atomes de carbone, avec la restriction que lorsque $R^7$ représente hydroxyle, les

radicaux hydroxyle et cétone sont portés par des atomes de carbone différents, pour obtenir le composé correspondant de formule I, où $R^1$ et $R^2$ sont tels que définis ici et $R^3$ représente un radical de formule $CR^5R^6$—$Alk^1(OH)R^7$, où $R^5$, $R^6$, $R^7$ et $Alk^1$ sont tels que définis ici;

o) on fait réagir une composé de formule I, où $R^1$ et $R^2$ sont tels que définis ici et $R^3$ représente hydrogène, avec l'oxyde d'éthylène, pour obtenir un composé de formule I, où $R^1$ et $R^2$ sont tels que définis ici et $R^3$ représente —$CH_2CH_2OH$; et

p) on fait réagir le composé de formule I, où $R^1$, $R^2$ et $R^3$ sont tels que définis ici, avec un acide thérapeutiquement acceptable, pour obtenir le composé correspondant de formule I, où $R^1$, $R^2$ et $R^3$ sont tels que définis ici sous forme de sel d'addition d'acide thérapeutiquement acceptable.

2. Procédé suivant la revendication 1, de préparation d'un composé de formule I, où $R^1$ représente un substituant en position 7 du cycle de 2,4,6-cycloheptatrién-1-one et est choisi entre hydrogène, halo, alcoyle inférieur, alcoxy inférieur, trifluorométhyle et 1-oxoalcoylamino inférieur; $R^2$ représente hydrogène ou alcoyle inférieur comptant 1 à 3 atomes de carbone; et $R^3$ représente hydrogène, phényle, alcoyle inférieur, alcényle inférieur, alcynyle inférieur, cycloalcoyle inférieur, alcoxycarbonyle inférieur, 1-oxoalcoyle inférieur, cycloalcoyle inférieur substitué par un alcoyle inférieur; alcényle inférieur substitué par phényle; phényle mono-, di- ou trisubstitué par alcoyle inférieur, halo, alcoxy inférieur, hydroxyle ou trifluorométhyle; 2-(4,5-dihydro-2-oxazolyl)benzoyle, 2,3-dihydro-8-oxocyclohepta[b]furan-2-ylméthyle, 1-oxo-2,4,6-cycloheptatrién-2-yle, 2-(acétylamino)éthyle, 2,3-diacétyloxypropyle, 2-(1,3-dihydro-1,3-dioxo-2H-isoindol-2-yl)éthyle, 5-méthyl-2,4-imidazolidinedione-5-ylméthyle, un radical de formule Alk—$R^4$, où Alk représente un alcoyle divalent comptant 1 à 6 atomes de carbone et $R^4$ représente alcoxy inférieur, cyano, aminocarbonyle, alcoxycarbonyle inférieur, cycloalcoyle inférieur, phényle, phénoxy, hydroxyalcoxy inférieur, 3-indolyle, 1-oxo-2,4,6-cycloheptatrién-2-ylamino, 1H-imidazol-4-yle, 1-oxoalcoxy inférieur, acétylthio, phényle mono-, di- ou trisubstitué par alcoxy inférieur ou hydroxyle, ou phénoxy mono-, di- ou trisubstitué par alcoyle inférieur, halo ou acétylamino, ou un radical de formule $CR^5R^6Alk^1(OH)R^7$, où $Alk^1$ représente un alcoylène trivalent de 1 à 6 atomes de carbone; $R^5$ et $R^6$ représentent chacun hydrogène ou alcoyle inférieur comptant 1 à 3 atomes de carbone; et $R^7$ représente hydrogène, phényle, hydroxyle, phénoxy, phényle mono-, di ou trisubstitué par méthylsulfonylamino, alcoxy inférieur ou hydroxyle, ou phénoxy mono-, di- ou trisubstitué par alcoyle inférieur ou halo; avec la restriction que lorsque $R^7$ représente hydroxyle, les radicaux hydroxyle sont portés par des atomes de carbone différents; ou d'un sel d'addition d'acide thérapeutiquement acceptable de ce composé.

3. Procédé suivant la revendication 1, de préparation d'un composé de formule I, où $R^1$ représente un substituant en position 7 du cycle de 2,4,6-cycloheptatrién-1-one et est choisi entre hydrogène et bromo; $R^2$ représente hydrogène ou méthyle; et $R^3$ représente hydrogène, phényle, alcoyle inférieur, alcényle inférieur, alcynyle inférieur, cycloalcoyle inférieur, alcoxycarbonyle inférieur, 1-oxoalcoyle inférieur, cycloalkyle inférieur substitué par un alcoyle inférieur, phényle monosubstitué par alcoyle inférieur, halo ou trifluorométhyle, 2-(4,5-dihydro-2-oxazolyl)benzoyle, 1-oxo-2,4,6-cycloheptatrién-2-yle, 2-(acétylamino)éthyle, 2,3-diacétyloxypropyle, 2-(1,3-dihydro-1,3-dioxo-2H-isoindol-2-yl)-éthyle, 5-méthyl-2,4-imidazolidinedione-5-ylméthyle, un radical de formule Alk—$R^4$, où Alk représente un alcoyle divalent comptant 1 à 3 atomes de carbone et $R^4$ représente alcoxy inférieur, cyano, aminocarbonyle, alcoxycarbonyle inférieur, cycloalcoyle inférieur, phényle, phénoxy, hydroalcoxy inférieur, 3-indolyle, 1-oxo-2,4,6-cycloheptatrién-2-ylamino, 1H-imidazol-4-yle, 1-oxoalcoxy inférieur, acétylthio, phényle mono- ou disubstitué par alcoxy inférieur ou hydroxyle, ou phénoxy monosubstitué par alcoyle inférieur, halo ou acétylamino; ou un radical de formule $CR^5R^6$—$Alk^1(OH)R^7$, où Alk représente un alcoylène trivalent comptant 1 à 3 atomes de carbone; $R^5$ et $R^6$ représentent chacun hydrogène ou alcoyle inférieur comptant 1 à 3 atomes de carbone; $R^7$ représente hydrogène, phényle, hydroxyle, phénoxy, phényle mono- di- ou trisubstitué par alcoxy inférieur ou hydroxyle ou phénoxy monosubstitué par alcoyle inférieur ou halo; avec la restriction que lorsque $R^7$ représente hydroxyle, les radicaux hydroxyle sont portés par des atomes de carbone différents; ou d'un sel d'addition d'acide thérapeutiquement acceptable de ce composé.

4. Procédé suivant la revendication 1, de préparation d'un composé de formule I, où $R^1$ et $R^2$ représentent hydrogène; et $R^3$ représente hydrogène, alcoyle inférieur, alcényle inférieur, alcoxycarbonyle inférieur, un radical de formule Alk—$R^4$, où Alk représente un alcoyle divalent comptant 1 ou 2 atomes de carbone et $R^4$ représente alcoxy inférieur, cyano, 1-oxoalcoxy inférieur, ou phényle mono- ou disubstitué par alcoxy inférieur ou hydroxyle, ou un radical de formule $CR^5R^6Alk^1(OH)R^7$, où $Alk^1$ représente CH; $R^5$ représente hydrogène, $R^6$ représente hydrogène ou alcoyle inférieur comptant 1 à 3 atomes de carbone; et $R^7$ représente hydrogène, phényle ou phényle mono-, di- ou trisubstitué aux positions 3, 4 ou 5 par alcoxy inférieur ou hydroxyle; ou d'un sel d'addition d'acide thérapeutiquement acceptable de ce composé.

5. Procédé suivant la revendication 1, où le composé de formule I qui est préparé est choisi parmi la (—)-2-[4-[2-hydroxy-2-(3,4-diméthoxyphényl)éthyl]-1-pipérazinyl]-2,4,6-cycloheptatrién-1-one [à $[\alpha]_D^{25}$ —12,0° (c=1, diméthylformamide), ], la (—)-2-[4-[2-hydroxy-1-méthyl-2-(3,4-diméthoxyphényl)-éthyl]-1-pipérazinyl]-2,4,6-cycloheptatrién-1-one, [à $[\alpha]_D^{25}$ —82,5° (c=1, diméthylformamide)], la 2-[4-[2-hydroxy-2-(3,4-diméthoxyphényl)éthyl-1-pipérazinyl]-2,4,6-cycloheptatrién-1-one, la 2-[4-[2-hydroxy-1-méthyl-2-(3,4-diméthoxyphényl)éthyl-1-pipérazinyl]-2,4,6-cycloheptatrién-1-one, la 2-[4-[2-hydroxy-1-méthyl-2-(3,4-diméthoxyphényl)éthyl]-1-pipérazinyl]-2,4,6-cycloheptatrién-1-one, [dont le maléate a un P.F. de 165—168°C], le 4-(2-oxo-3,5,7-cycloheptatrién-1-yl)-1-pipérazine-éthanol, la (+)-2-[4-[2-hydroxy-2-(3,4-diméthoxyphényl)éthyl-1-pipérazinyl]-2,4,6-cycloheptatrién-1-one [à $[\alpha]_D^{25}$ +11,7° (c=1, diméthylformamide)]; la 2-[4-(2-hydroxypropyl)-1-pipérazinyl]-2,4,6-cycloheptatrién-1-one, la (+)-2-[4-[2-hydroxy-1-méthyl-2-(3,4-diméthoxyphényl)éthyl]-1-pipérazinyl]-2,4,6-cycloheptatrién-1-one, [à $[\alpha]_D^{25}$ +84,2° (c=1, diméthylformamide)] et la 2-[4-[2-hydroxy-2-(4-méthoxyphényl)-éthyl]-1-pipérazinyl-2,4,6-cycloheptatrién-1-one, ou un sel d'addition d'acide thérapeutiquement acceptable de ce composé.

6. Procédé suivant la revendication 1, où le composé de formule I qui est préparé est la 7-bromo-2-[4-(2-hydroxyéthyl)-1-pipérazinyl]-2,4,6-cycloheptatrién-1-one, ou un sel d'addition d'acide thérapeutiquement acceptable de ce composé.

7. Procédé suivant la revendication 1, où le composé de formule I qui est préparé est choisi parmi le 4-(2-oxo-3,5,7-cycloheptatrién-1-yl)-1-pipérazineacétonitrile, la 2-[4-[2-(3,4-diméthoxyphényl)-éthyl]-1-pipérazinyl]-2,4,6-cycloheptatrién-1-one, la 2-[4-[2-(acétyloxy)éthyl-1-pipérazinyl]-2,4,6-cycloheptatrién-1-one, et l'ester 2-[4-(2-oxo-3,5,7-cycloheptatrién-1-yl)-1-pipérazinyl]éthylique d'acide 2,2-diméthylpropanoïque, ou un sel d'addition d'acide pharmaceutiquement acceptable de ce composé.

8. Procédé suivant la revendication 1, où le composé de formule I qui est préparé est choisi parmi la 2-(4-méthyl-1-pipérazinyl)-2,4,6-cycloheptatrién-1-one, la 2-[4-(2-propényl)-1-pipérazinyl]-2,4,6-cycloheptatrién-1-one, la 2-[4-(1-méthyléthyl)-1-pipérazinyl]-2,4,6-cycloheptatrién-1-one, la 2-(4-propyl-1-pipérazinyl)-2,4,6-cycloheptatrién-1-one et la 2-[4-(1-méthylpropyl)-1-pipérazinyl]-2,4,6-cycloheptatrién-1-one ou un sel d'addition d'acide thérapeutiquement acceptable de ce composé.

9. Procédé suivant la revendication 1, de préparation d'un composé de formule 1, oú $R^1$ et $R^2$ sont tels que définis ici et $R^3$ est un radical de formule $CR^5R^6$—$Alk^1(OH)R^7$, où $R^5$, $R^6$, $R^7$ et $Alk^1$ sont tels que définis ici, suivant lequel on réduit la cétone correspondante de formule V, où $R^1$, $R^2$, $R^5$, $R^6$, $R^7$ et $Alk^1$=O sont tels que définis ici, au moyen de borohydrure de sodium.

10. Procédé suivant la revendication 9, de préparation d'un composé de formule I, ou $R^1$ et $R^2$ représentent hydrogène; et $R^3$ représente un radical de formule $CR^5R^6$—$Alk^1(OH)R^7$, où $Alk^1$ représente CH, $R^5$ représente hydrogène; $R^6$ représente hydrogène ou alcoyle inférieur comptant 1 à 3 atomes de carbone; et $R^7$ représente phényle ou phényle mono-, di- ou trisubstitué aux positions 3, 4 ou 5 par alcoxy inférieur ou hydroxyle.

11. Procédé suivant la revendication 1, de préparation d'un composé de formule I, où $R^1$ et $R^2$ représentent hydrogène et $R^3$ représente un radical de formule $CR^5R^6$—$Alk^1(OH)R^7$, où $Alk^1$ représente CH, $R^5$ représente hydrogène, $R^6$ représente hydrogène ou alcoyle inférieur comptant 1 à 3 atomes de carbone; et $R^7$ représente phényle ou phényle mono-, di ou trisubstitué aux positions 3, 4 ou 5 par alcoxy inférieur ou hydroxyle, suivant lequel on condense un composé de formule II, où $R^1$ est tel que défini ici et $R^8$ représente méthyle ou éthyle, avec un composé de formule III, où $R^2$ et $R^3$ sont tels que définis ici.

12. Procédé, suivant lequel un composé de formule I, tel que défini dans la revendication 9, 10 ou 11, est dédoublé, pour l'isolement de ses isomères optiques séparés.

13. Procédé de préparation d'un composé de formule V, tel que défini dans la revendication 1, suivant lequel on fait réagir un composé correspondant de formule I, où $R^1$ et $R^2$ sont tels que définis dans la revendication 1 et $R^3$ représente hydrogène, avec une alcanone de formule:

$$X—CR^5R^6—\underset{\underset{O}{\|}}{Alk^1}—R^7$$

où $R^5$ et $R^6$, $R^7$ et $Alk^1$=O sont tels que définis dans la revendication 1 et X représente bromo, chloro ou iodo, en présence d'un accepteur de protons.

14. Procédé de préparation d'une composition pharmaceutique, suivant lequel on mélange un composé de formule I, tel que défini dans la revendication 1, ou un sel d'acide thérapeutiquement acceptable de ce composé avec un excipient pharmaceutiquement acceptable.